# EUROPEAN PATENT APPLICATION

(11) **EP 2 202 224 A1**
(43) Date of publication of application: **30.06.2010**
(21) Application number: 09156568.9
(22) Date of filing: 30.03.2009
(51) Int. Cl.: C07D 221/10, C09K 11/06, H05B 33/14, H01L 51/50, H01L 51/00

(54) **Novel Organometallic Compounds for Electroluminescence and Organic Electroluminescent Device Using the Same**

(30) Priority: 13.11.2008 KR 20080112855
(71) Applicant: Gracel Display Inc., Seoul 133-833 (KR)
(72) Inventor: Kim, Hyun, Marlborough, MA 01752 (US); Kwon, Hyuck Joo, 130-100, Seoul (KR); Kim, Bong Ok, 135-090, Seoul (KR); Kim, Sung Min, 157-886, Seoul (KR); Yoon, Seung Soo, 135-884, Seoul (KR)
(74) Representative: Buckley, Guy Julian

(57) **Abstract**

Provided are an electroluminescent compound represented by Chemical Formula 1 and an electroluminescent device comprising the same as a host material:

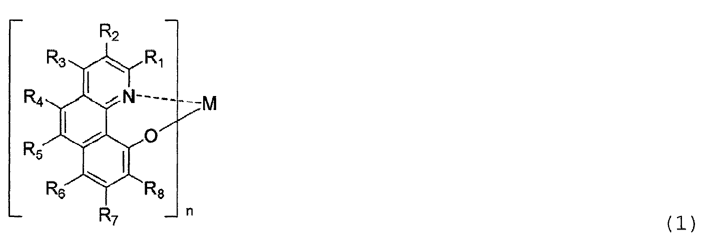

When used as a host material of an electroluminescent material in an OLED device, the disclosed electroluminescent compound exhibits superior red luminous efficiency and excellent life property of the material, as compared to the existing host material. Therefore, it can be used to manufacture OLEDs having very superior operation life.

## Description

### CROSS-REFERENCE TO RELATED APPLICATION

This application claims priority to Korean Patent Application No. 10-2008-0112855, filed on November 13, 2008, and all the benefits accruing therefrom under 35 U.S.C. §119.

### BACKGROUND

### 1. Field

The present invention relates to an electroluminescent compound comprising a metal complex exhibiting excellent electrical conductivity and high luminous efficiency, and an electroluminescent device comprising the same as a host material.

### 2. Description of the Related Art

The most important factor that determines luminous efficiency of an OLED is the electroluminescent material. At present, fluorescent materials are widely used for the electroluminescent material. But, phosphorescent materials are better when considering the electroluminescence mechanism. Theoretically, phosphorescent materials can improve the luminous efficiency by 4-fold.

Until now, iridium (III) complex-based phosphorescent materials are widely known. Such materials as (acac)Ir(btp)₂, Ir(ppy)₃ and Firpicare known for red, green and blue colors, respectively. Recently, a lot of researches on phosphorescent materials are underway, especially in Japan, Europe and the US.

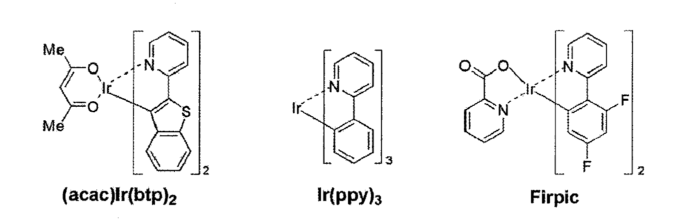

At present, CBP is the most widely known as a host material for a phosphorescent material. High-efficiency OLEDs using a hole blocking layer comprising BCP, BAlq, etc. are reported. High-performance OLEDs using BAlq derivatives as a host were reported by Pioneer (Japan) and others.

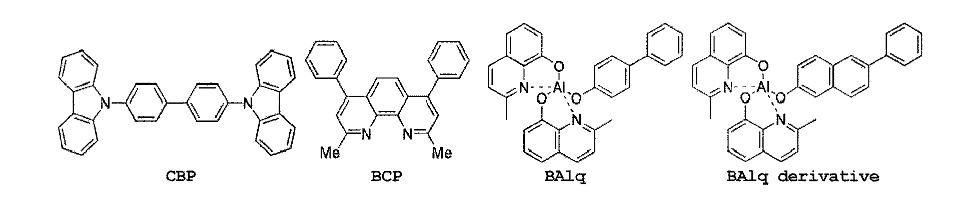

Although these materials provide good electroluminescence characteristics, they are disadvantageous in that degradation may occur during the high-temperature deposition process in vacuum because of low glass transition temperature and poor thermal stability. Since the power efficiency of an OLED is given by (π / voltage) × current efficiency, the power efficiency is inversely proportional to the voltage. High power efficiency is required to reduce the power consumption of an OLED. Actually, OLEDs using phosphorescent materials provide much better current efficiency (cd/A) than those using fluorescent materials. However, when the existing materials such as BAlq, CBP, etc. are used as a host of the phosphorescent material, there is no significant advantage in power efficiency (lm/W) over the OLEDs using fluorescent materials because of high driving voltage.

Further, the OLED devices do not have satisfactory operation life. Therefore, development of more stable, higher-performance host materials is required.

With regard thereto, many studies have been carried out since mid-1990s, including complexes for blue electroluminescent materials. However, they are simply used for the electroluminescent material, and little is known about application as a host material.

### SUMMARY

In order to solve the aforesaid problems, in an aspect, the present invention provides an electroluminescent compound having a novel ligand metal complex with very superior electroluminescence characteristics and physical properties as compared to existing organic host materials as a backbone. In another aspect, the present invention provides an electroluminescent device comprising the electroluminescent compound as a host material. In another aspect, the present invention provides an organic solar cell comprising the electroluminescent compound.

### BRIEF DESCRIPTION OF THE DRAWINGS

The above and other aspects, features and advantages of the present invention will become apparent from the following description of preferred embodiments given in conjunction with the accompanying drawing, in which:

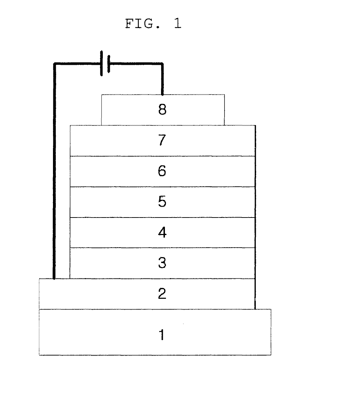

FIG. 1 is a cross-sectional view of an OLED device.

### DETAILED DESCRIPTION

Exemplary embodiments now will be described more fully hereinafter with reference to the accompanying drawings, in which exemplary embodiments are shown. This disclosure may, however, be embodied in many different forms and should not be construed as limited to the exemplary embodiments set forth therein. Rather, these exemplary embodiments are provided so that this disclosure will be thorough and complete, and will fully convey the scope of this disclosure to those skilled in the art. In the description, details of well-known features and techniques may be omitted to avoid unnecessarily obscuring the presented embodiments.

The terminology used herein is for the purpose of describing particular embodiments only and is not intended to be limiting of this disclosure. As used herein, the singular forms "a", "an" and "the" are intended to include the plural forms as well, unless the context clearly indicates otherwise. Furthermore, the use of the terms a, an, etc. does not denote a limitation of quantity, but rather denotes the presence of at least one of the referenced item. The use of the terms "first", "second", and the like does not imply any particular order, but they arr included to identify individual elements. Moreover, the use of the terms first, second, etc. does not denote any order or importance, but rather the terms first, second, etc. are used to distinguish one element from another. It will be further understood that the terms "comprises" and/or "comprising", or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

The shape, size and regions, and the like, of the drawing may be exaggerated for clarity.

The present invention relates to an electroluminescent compound represented by Chemical Formula 1 and an electroluminescent device comprising the same as a host material: 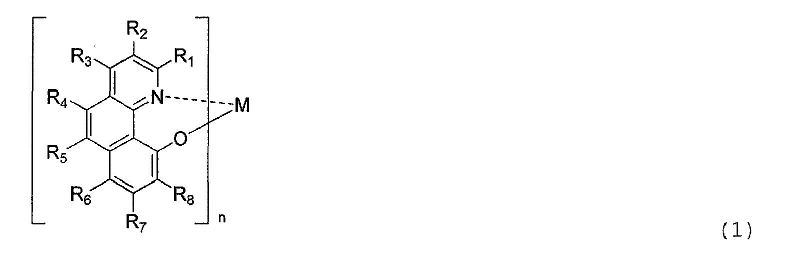 wherein
M represents a divalent or trivalent metal;
n represents the cationic valence of M;
R₁ through R₈ independently represent hydrogen, (C1-C60)alkyl, halogen, cyano, (C3-C60)cycloalkyl, 5- or 6-membered heterocycloalkyl containing one or more heteroatom(s) selected from N, O and S, (C7-C60)bicycloalkyl, adamantyl, (C2-C60)alkenyl, (C2-C60)alkynyl, (C6-C60)aryl, (C1-C60)alkoxy, (C1-C60)alkylthio, (C6-C60)aryloxy, (C3-C60)heteroaryl containing one or more heteroatom(s) selected from N, O and S, (C6-C60)arylthio, (C1-C60)alkoxycarbonyl, (C1-C60)alkylcarbonyl, (C6-C60)arylcarbonyl, mono- or di(C1-C30)alkylamino, mono- or di(C6-C30)arylamino, tri(C1-C30)alkylsilyl, di(C1-C30)alkyl(C6-C30)arylsilyl, tri(C6-C30)arylsilyl, di(C6-C60)arylboranyl, di(C1-C60)alkylboranyl, carboxyl, nitro or hydroxyl; and
the alkyl, cycloalkyl, heterocycloalkyl, bicycloalkyl, adamantyl, alkenyl, alkynyl, aryl, alkoxy, alkylthio, aryloxy, heteroaryl, arylthio, alkoxycarbonyl, alkylcarbonyl, arylcarbonyl, alkylamino, arylamino, trialkylsilyl, dialkylarylsilyl, triarylsilyl, diarylboranyl or dialkylboranyl of R₁ through R₈ may be further substituted by one or more substituent(s) selected from a group consisting of (C1-C60)alkyl, halogen, cyano, (C3-C60)cycloalkyl, 5- or 6-membered heterocycloalkyl containing one or more heteroatom(s) selected from N, O and S, (C7-C60)bicycloalkyl, adamantyl, (C2-C60)alkenyl, (C2-C60)alkynyl, (C6-C60)aryl, (C1-C60)alkoxy, (C1-C60)alkylthio, (C6-C60)aryloxy, (C3-C60) heteroaryl containing one or more heteroatom (s) selected from N, O and S, (C6-C60)arylthio, (C1-C60)alkoxycarbonyl, (C1-C60)alkylcarbonyl, (C6-C60)arylcarbonyl, mono- or di(C1-C30)alkylamino, mono- or di(C6-C30)arylamino, tri(C1-C30)alkylsilyl, di(C1-C30)alkyl(C6-C30)arylsilyl, tri(C6-C30)arylsilyl, carboxyl, nitro and hydroxyl,
except for the case where all of R₁ through R₈ are hydrogens.

In the present invention, "alkyl", "alkoxy" and other substituents including "alkyl" moiety may be either linear or branched.

In the present invention, "aryl" means an organic radical derived from an aromatic hydrocarbon by the removal of one hydrogen, and may include a 4- to 7-membered, particularly 5- or 6-membered, single ring or fused ring. Specific examples include phenyl, naphthyl, biphenyl, anthryl, indenyl, fluorenyl, phenanthryl, triphenylenyl, pyrenyl, perylenyl, chrysenyl, naphthacenyl, fluoranthenyl, etc., but not limited thereto. The naphthyl includes 1-naphthyl and 2-naphthyl, the anthryl includes 1-anthryl, 2-anthryl and 9-anthryl, and the fluorenyl includes all of 1-fluorenyl, 2-fluorenyl, 3-fluorenyl, 4-fluorenyl and 9-fluorenyl.

In the present invention, "heteroaryl" means an aryl group containing 1 to 4 heteroatom(s) selected from N, O, S and Si as aromatic ring backbone atom(s), other remaining aromatic ring backbone atoms being carbon. It may be 5- or 6-membered monocyclic heteroaryl or polycyclic heteroaryl resulting from condensation with a benzene ring, and may be partially saturated. The heteroaryl includes a divalent aryl group wherein the heteroatom(s) in the ring may be oxidized or quaternized toform,for example,N-oxide or quaternarysalt. Specific examples include monocyclic heteroaryl such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl, etc., polycyclic heteroaryl such as benzofuranyl, benzothiophenyl, isobenzofuranyl, benzimidazolyl, benzothiazolyl, benzoisothiazolyl, benzoisoxazolyl, benzoxazolyl, isoindolyl, indolyl, indazolyl, benzothiadiazolyl, quinolyl, isoquinolyl, cinnolinyl, quinazolinyl, quinoxalinyl, carbazolyl, phenanthridinyl, benzodioxolyl, etc., N-oxide thereof (e.g., pyridyl N-oxide, quinolyl N-oxide, etc.), quaternary salt thereof, etc., but not limited thereto.

And, in the present invention, the substituents including "(C1-C60)alkyl" moiety may have 1 to 60 carbon atoms, 1 to 20 carbon atoms, or 1 to 10 carbon atoms. The substituents including "(C6-C60)aryl" moiety may have 6 to 60 carbon atoms, 6 to 20 carbon atoms, or 6 to 12 carbon atoms. The substituents including "(C3-C60)heteroaryl" moiety may have 3 to 60 carbon atoms, 4 to 20 carbon atoms, or 4 to 12 carbon atoms. The substituents including "(C3-C60)cycloalkyl" moiety may have 3 to 60 carbon atoms, 3 to 20 carbon atoms, or 3 to 7 carbon atoms. The substituents including "(C2-C60)alkenyl or alkynyl" moiety may have 2 to 60 carbon atoms, 2 to 20 carbon atoms, or 2 to 10 carbon atoms.

Specifically, R₁ through R₈ may independently represent hydrogen, methyl, ethyl, n-propyl, i-propyl, n-butyl, i-butyl, t-butyl, trifluoromethyl, perfluoroethyl, perfluorobutyl, chloro, fluoro, cyano, cyclopropyl, cyclobutyl, cyclopentyl, cyclohexyl, cycloheptyl, cyclooctyl, pyrrolidinyl, imidazolidinyl, pyrazolidinyl, piperidinyl, piperazinyl, morpholinyl, thiomorpholinyl, pyridyl, furyl, thienyl, pyrrolyl, pyrazolyl, oxazolyl, isoxazolyl, thiazolyl, isothiazolyl, imidazolyl, oxadiazolyl, thiadiazolyl, pyrazinyl, pyrimidinyl, pyridazinyl, benzoxazolyl, benzothiazolyl, benzimidazolyl, carbazolyl, phenanthridinyl, quinolyl, isoquinolyl, adamantyl, ethenyl, methylethenyl, phenylethenyl, ethynyl, methylethynyl, phenylethynyl, phenyl, biphenyl, naphthyl, 9,9-dimethylfluorenyl, 9,9-diphenylfluorenyl, methoxy, ethoxy, n-propoxy, i-propoxy, n-butoxy, i-butoxy, t-butoxy, hexyloxy, methylthio, phenyloxy, phenylthio, methoxycarbonyl, butoxycarbonyl, methylcarbonyl, ethylcarbonyl, butylcarbonyl, phenylcarbonyl, naphthylcarbonyl, dimethylamino, diethylamino, diphenylamino, trimethylsilyl, triphenylsilyl, t-butyldimethylsilyl, diphenylboranyl, dimethylboranyl, carboxyl, nitro or hydroxyl; and

The pyrrolidinyl, imidazolidinyl, pyrazolidinyl, piperidinyl, piperazinyl, morpholinyl, thiomorpholinyl, pyridyl, furyl, thienyl, pyrrolyl, pyrazolyl, oxazolyl, isoxazolyl, thiazolyl, isothiazolyl, imidazolyl, oxadiazolyl, thiadiazolyl, pyrazinyl, pyrimidinyl, pyridazinyl, benzoxazolyl, benzothiazolyl, benzimidazolyl, carbazolyl, phenanthridinyl, quinolyl, isoquinolyl, phenyl, biphenyl, naphthyl, fluorenyl, diphenylboranyl or dimethylboranyl of R₁ through R₈ may be further substituted by one or more substituent (s) selected from a group consisting of methyl, ethyl, n-propyl, i-propyl, n-butyl, i-butyl, t-butyl, trifluoromethyl, perfluoroethyl, perfluorobutyl, chloro, fluoro, cyano, cyclopropyl, cyclobutyl, cyclopentyl, cyclohexyl, cycloheptyl, cyclooctyl, pyrrolidinyl, imidazolidinyl, pyrazolidinyl, piperidinyl, piperazinyl, morpholinyl, thiomorpholinyl, pyridyl, furyl, thienyl, pyrrolyl, pyrazolyl, oxazolyl, isoxazolyl, thiazolyl, isothiazolyl, imidazolyl, oxadiazolyl, thiadiazolyl, pyrazinyl, pyrimidinyl, pyridazinyl, benzoxazolyl, benzothiazolyl, benzimidazolyl, carbazolyl, phenanthridinyl, quinolyl, isoquinolyl, adamantyl, ethenyl, methylethenyl, phenylethenyl, ethynyl, methylethynyl, phenylethynyl, phenyl, biphenyl, naphthyl, 9,9-dimethylfluorenyl, 9,9-diphenylfluorenyl, methoxy, ethoxy, n-propoxy, i-propoxy, n-butoxy, i-butoxy, t-butoxy, hexyloxy, methylthio, phenyloxy, phenylthio, methoxycarbonyl, butoxycarbonyl, methylcarbonyl, ethylcarbonyl, butylcarbonyl, phenylcarbonyl, naphthylcarbonyl, dimethylamino, diethylamino, diphenylamino, trimethylsilyl, triphenylsilyl, t-butyldimethylsilyl, carboxyl, nitro and hydroxyl.

In Chemical Formula 1, M represents a divalent metal selected from a group consisting of Be, Zn, Mg, Ca, Cu and Ni, or a trivalent metal selected from a group consisting of Al, In, Co, Ga and B.

More specifically, the electroluminescent compound represented by Chemical Formula 1 may be exemplified by following compounds, but not limited thereto:

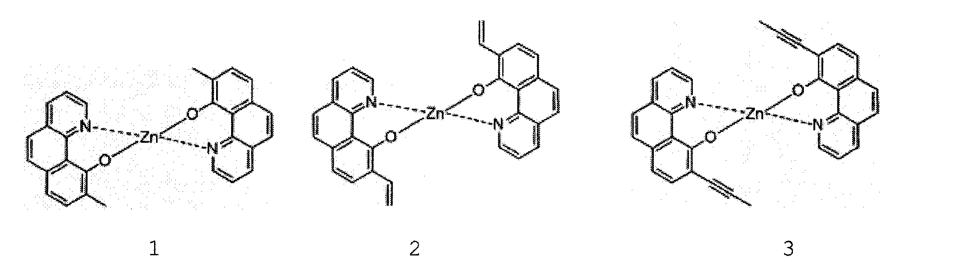

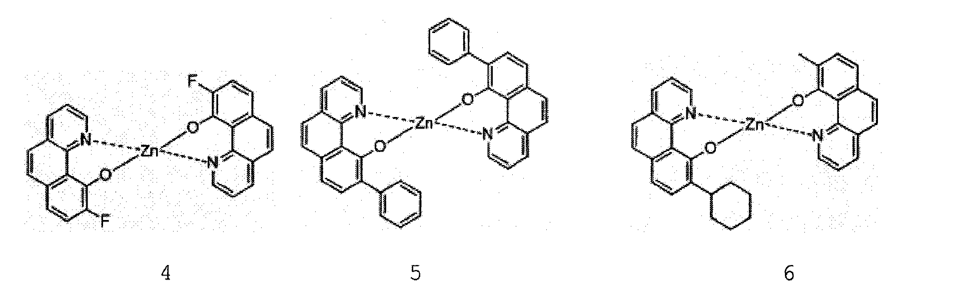

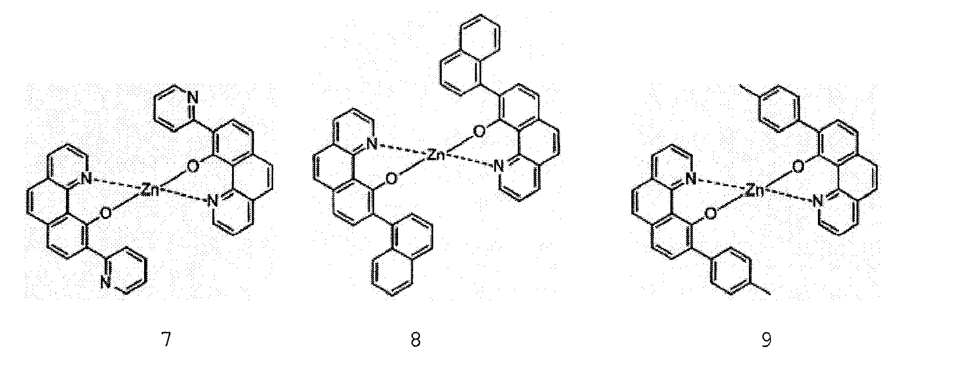

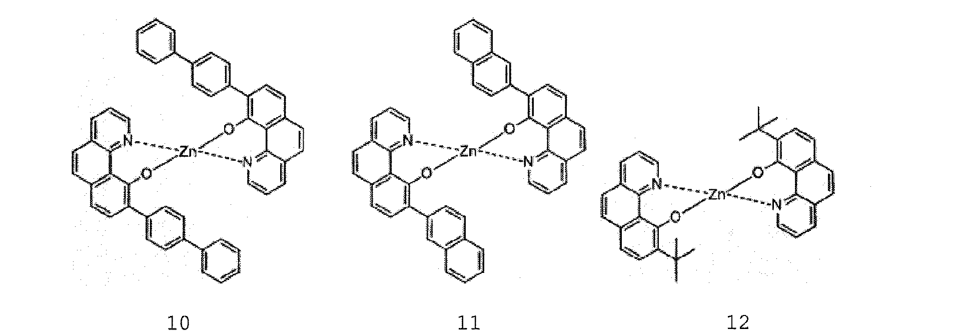

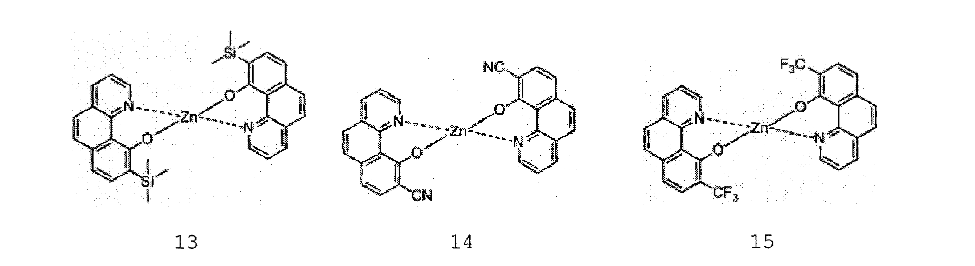

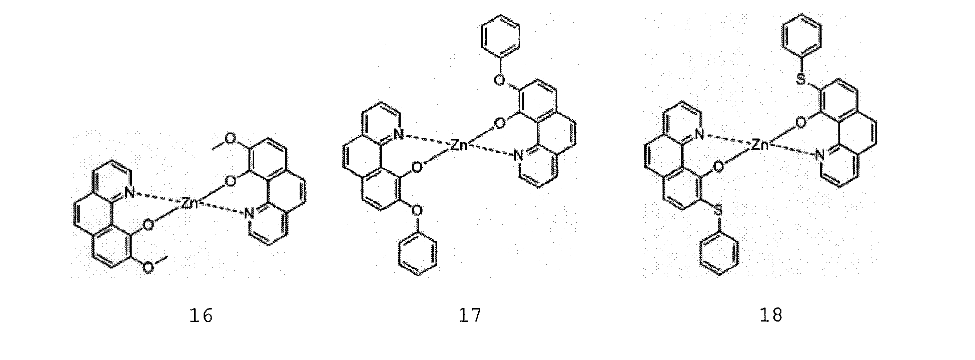

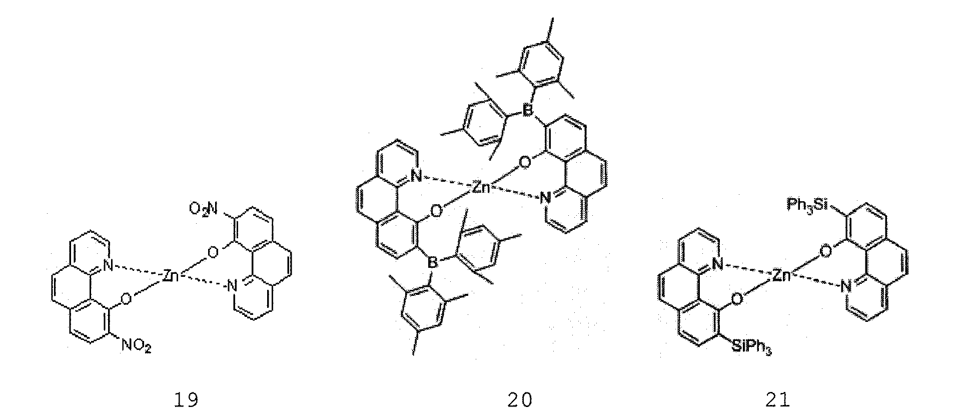

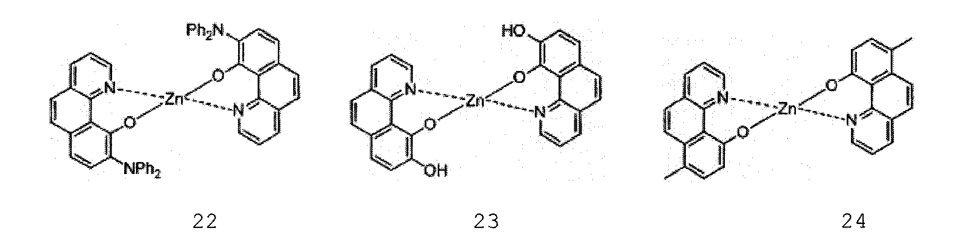

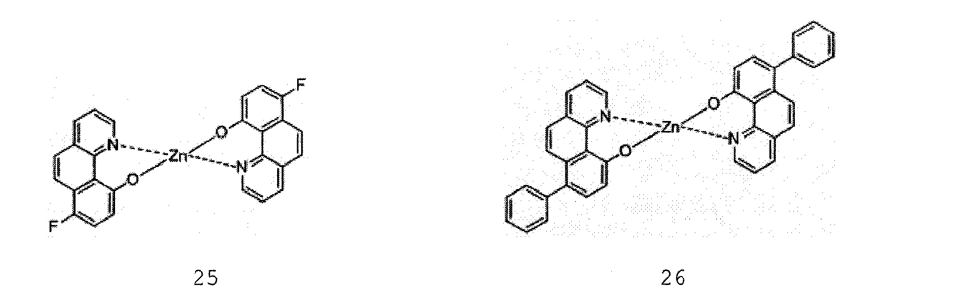

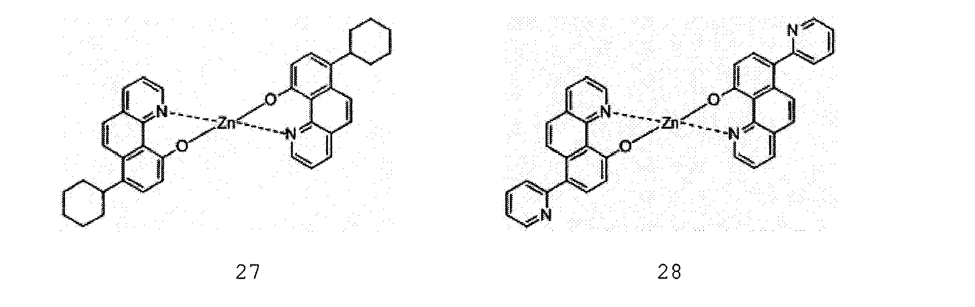

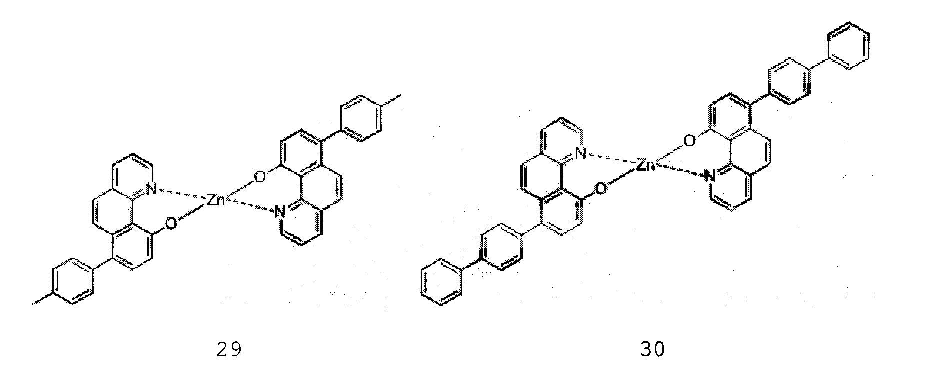

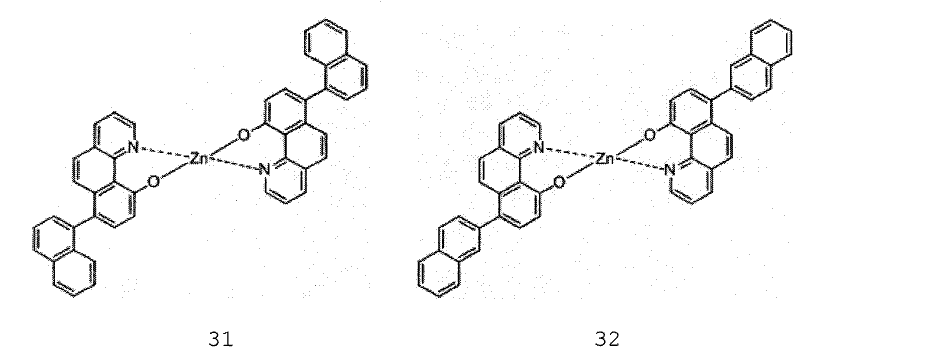

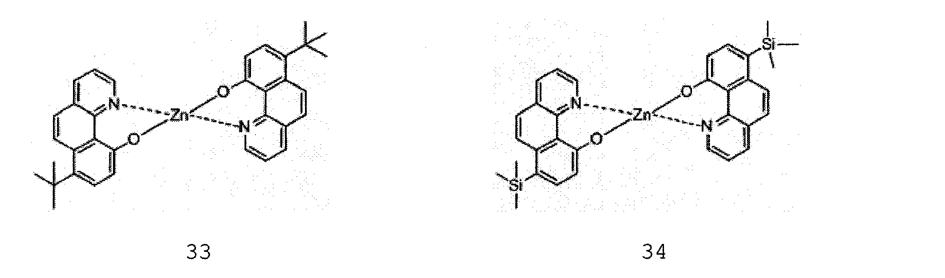

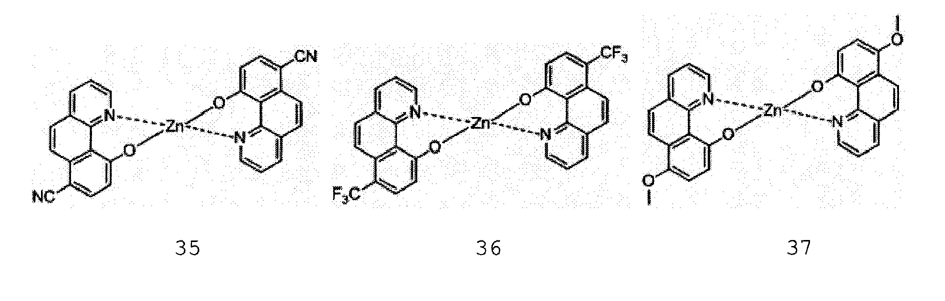

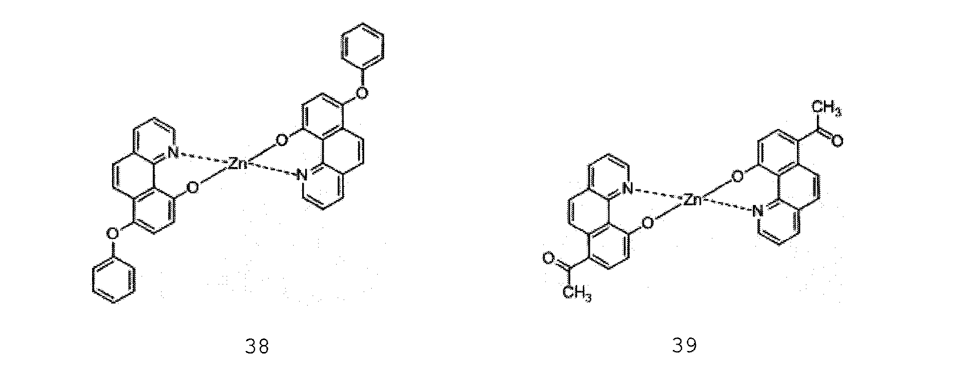

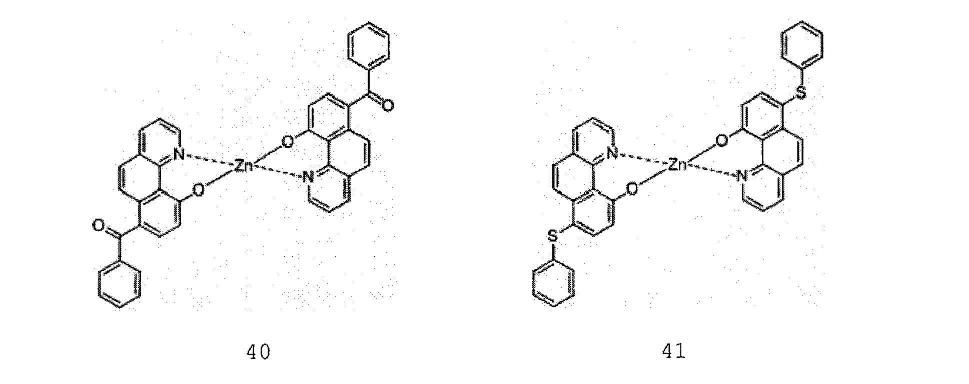

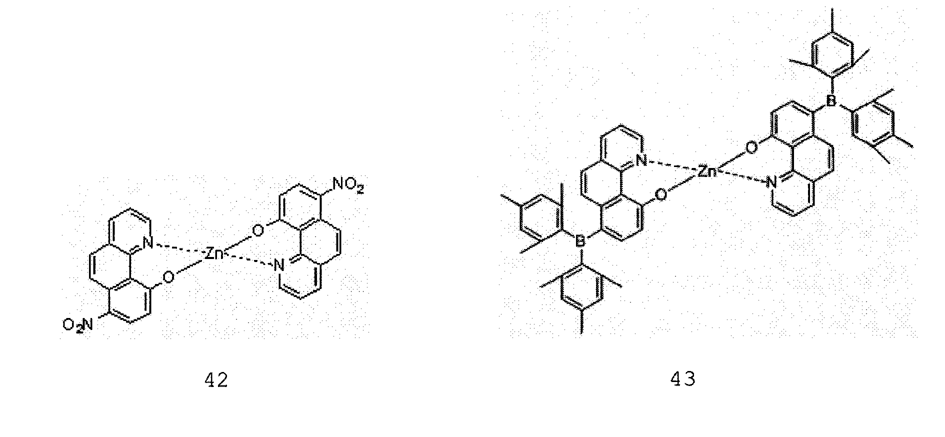

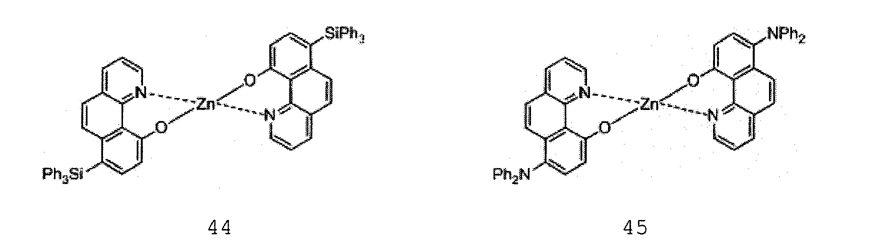

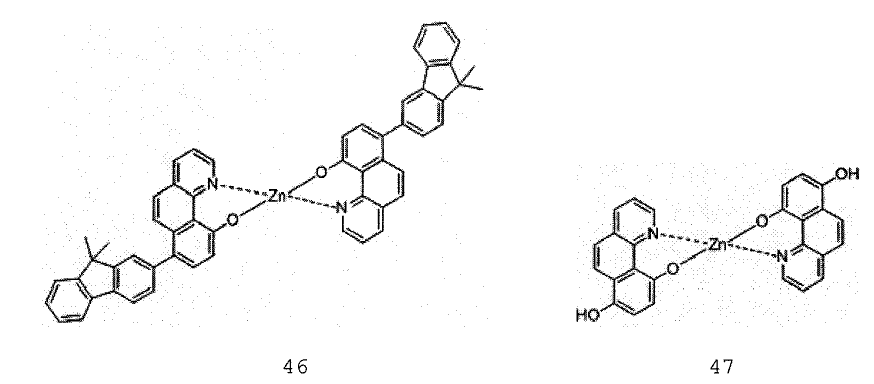

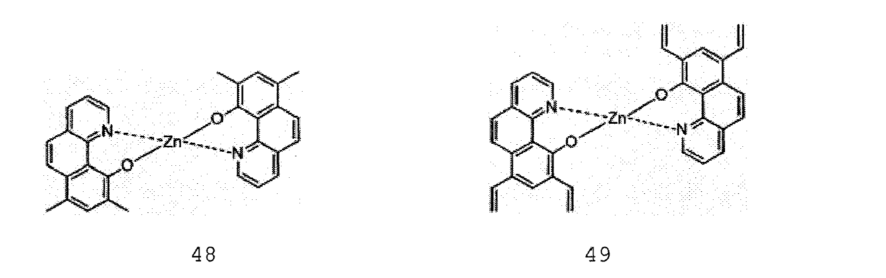

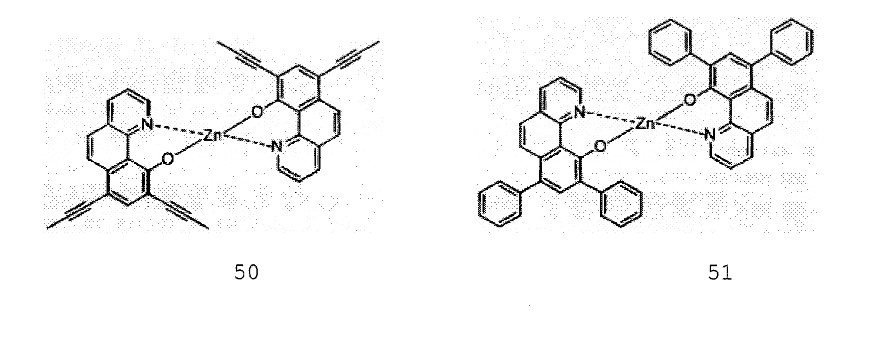

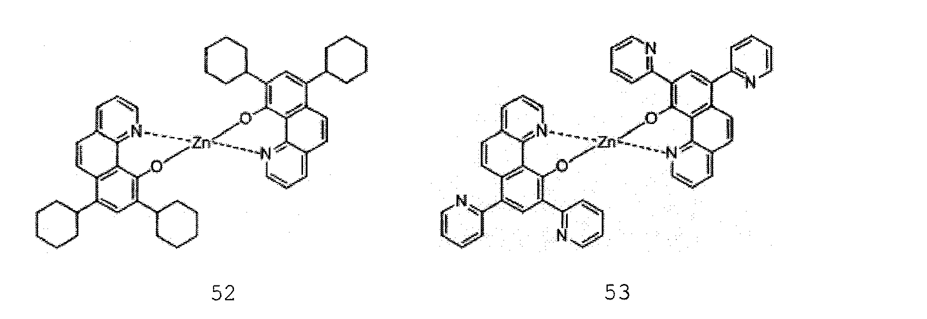

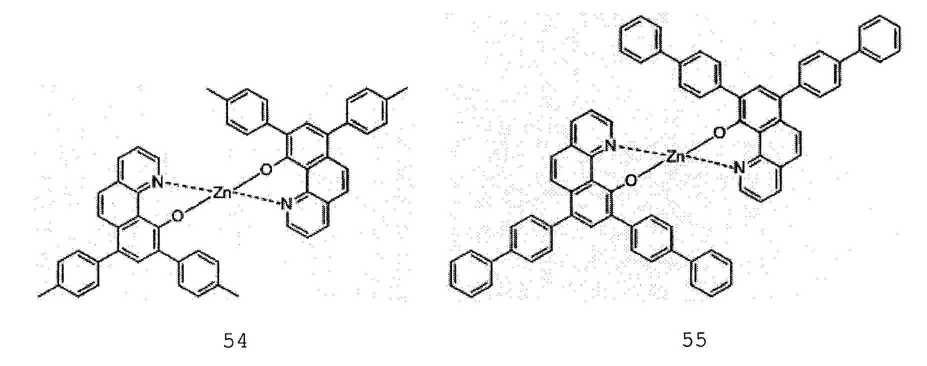

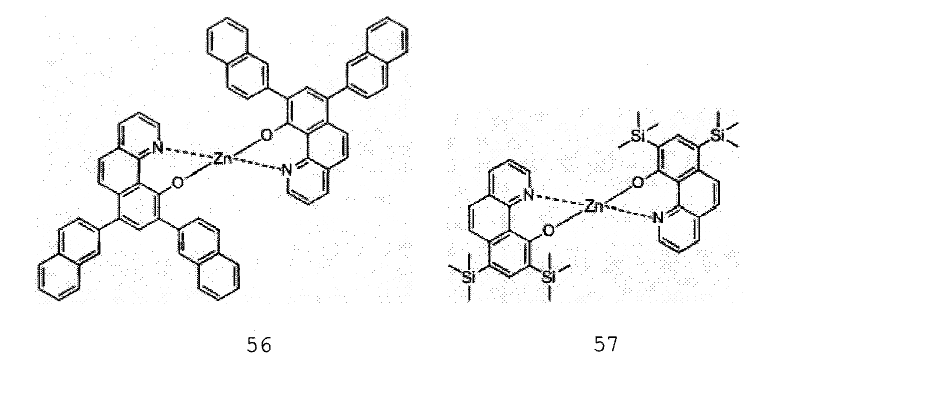

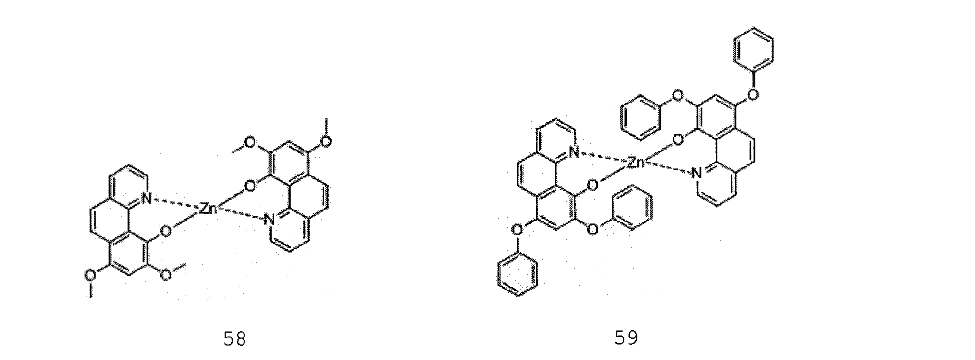

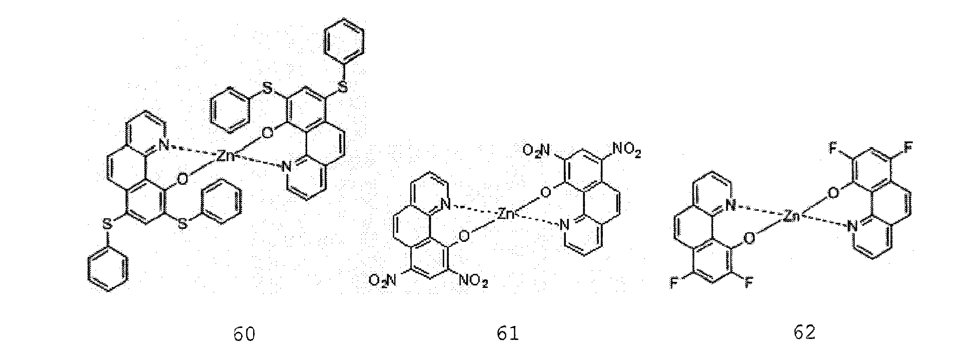

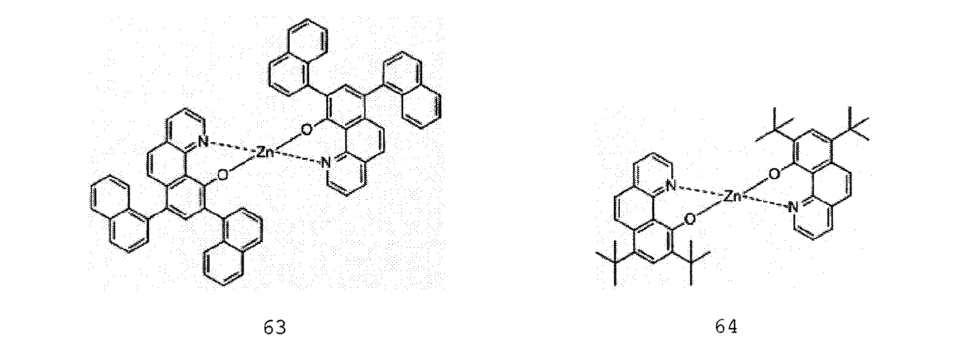

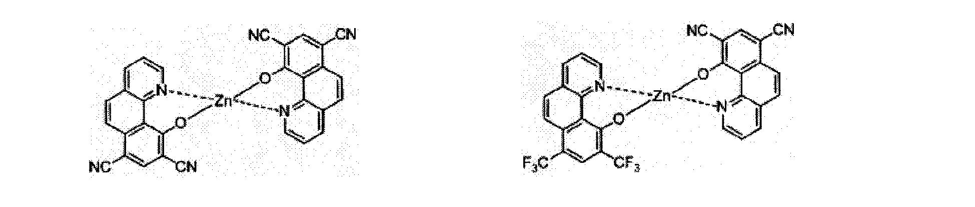

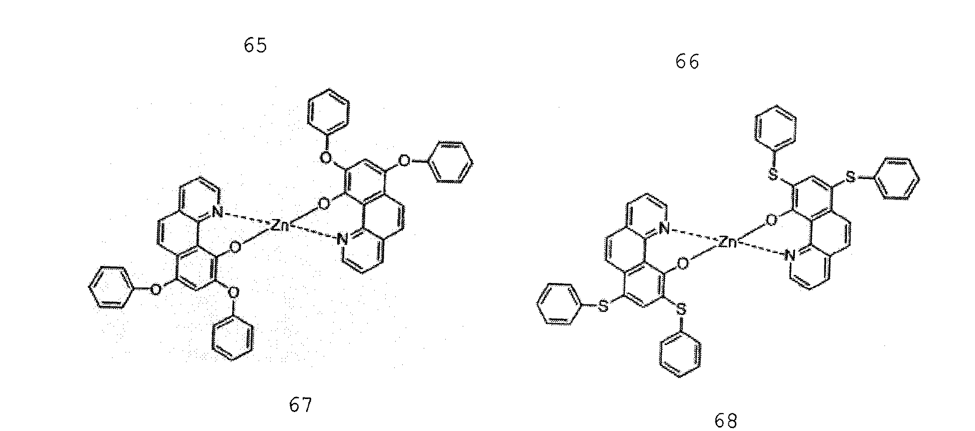

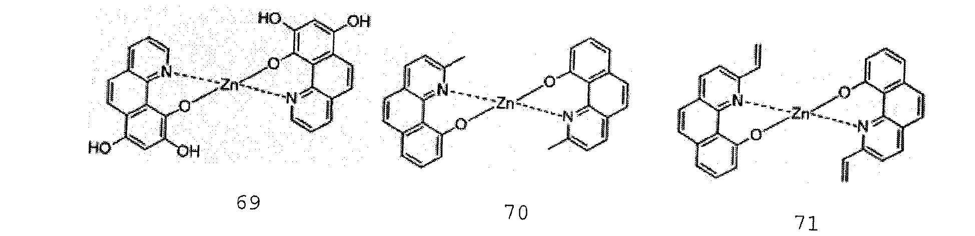

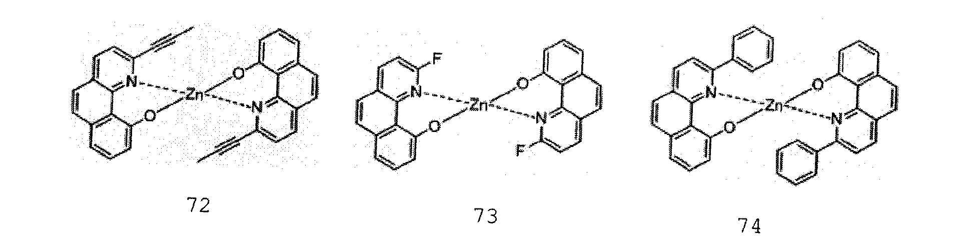

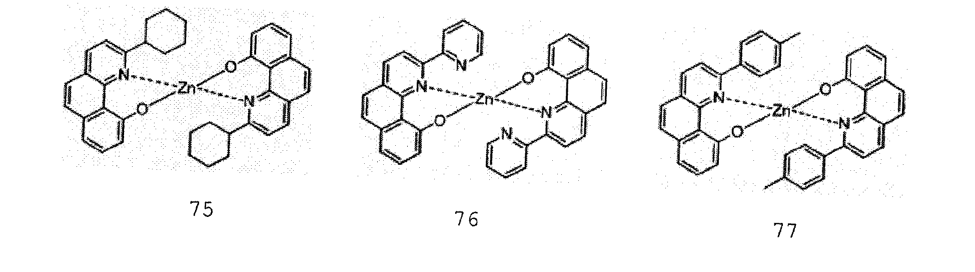

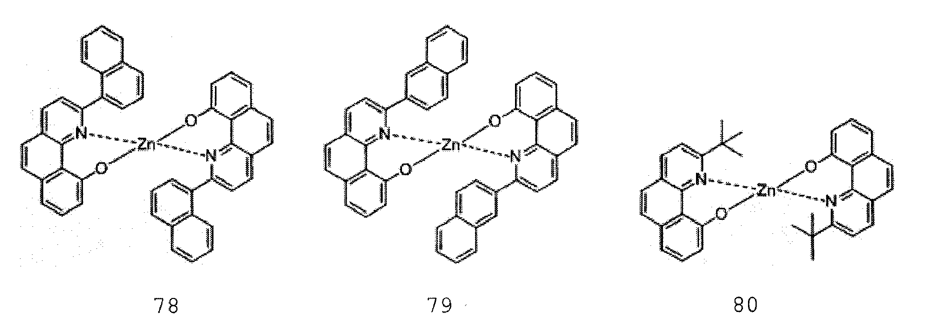

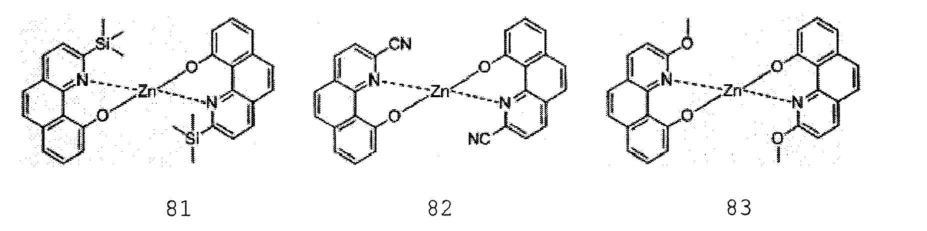

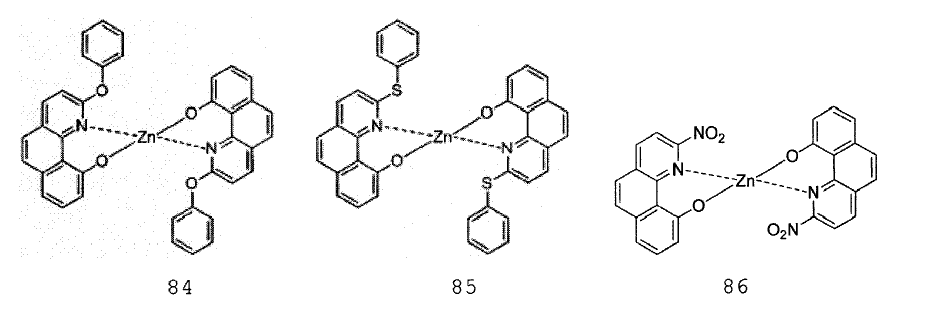

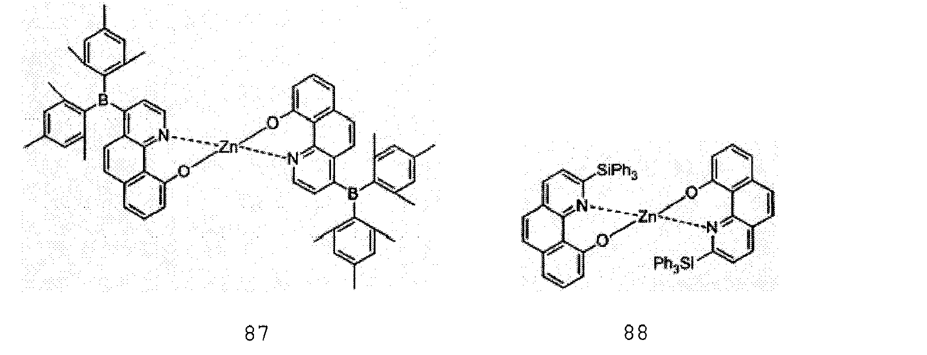

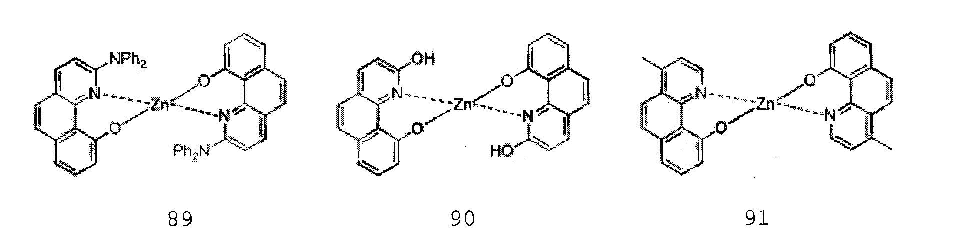

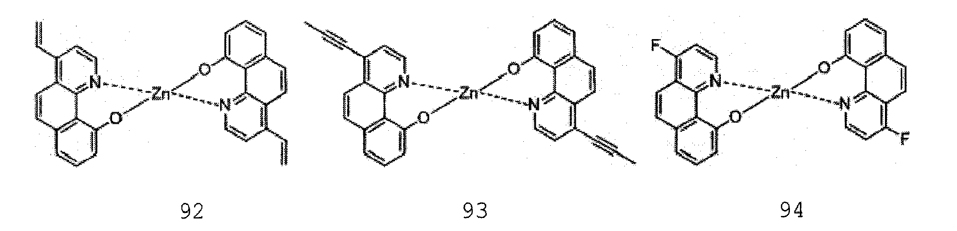

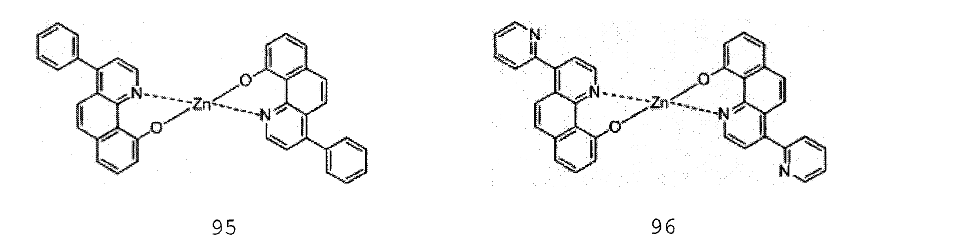

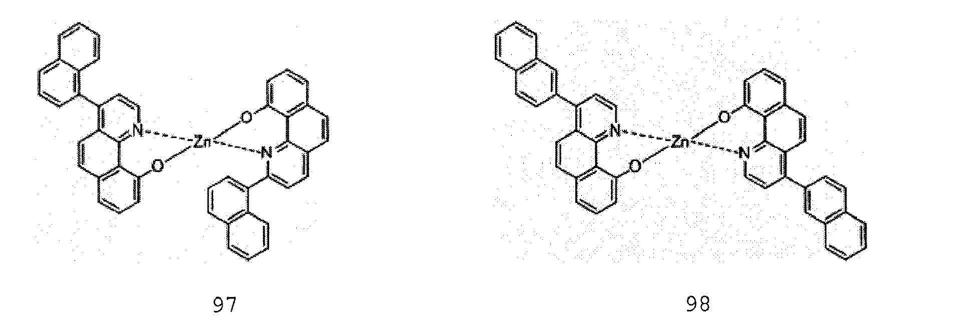

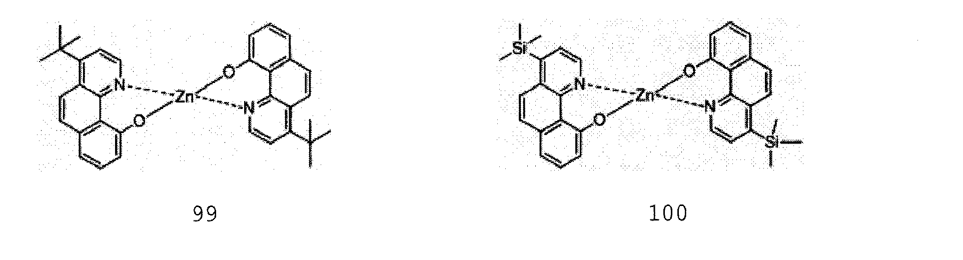

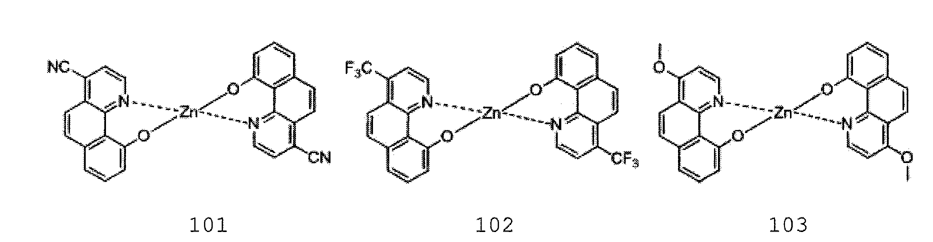

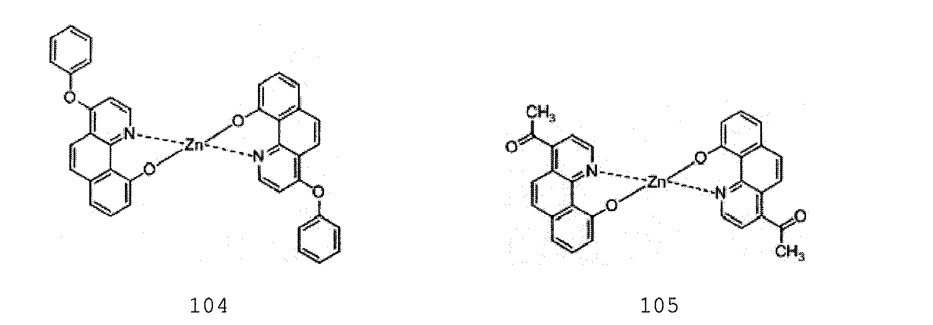

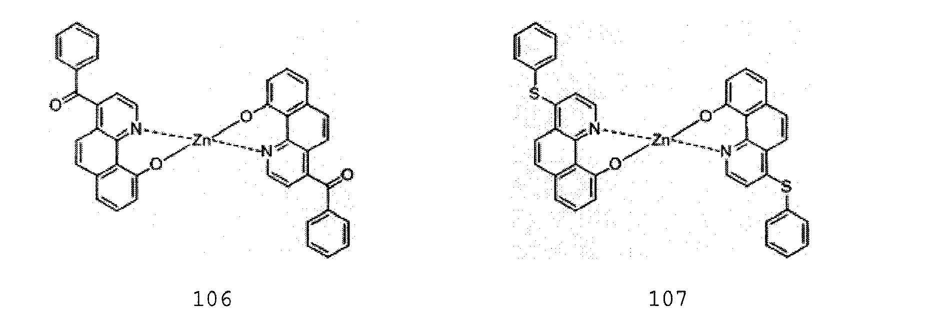

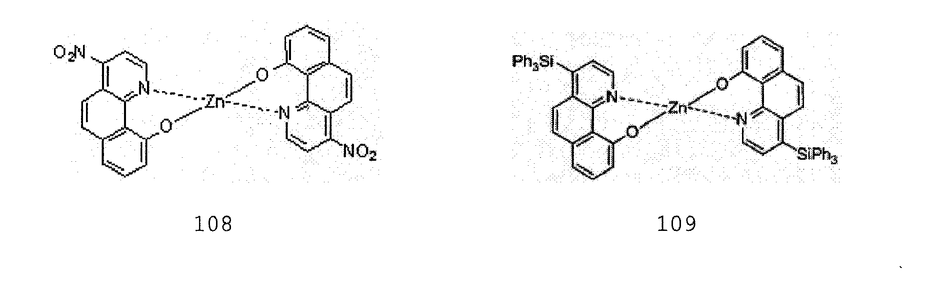

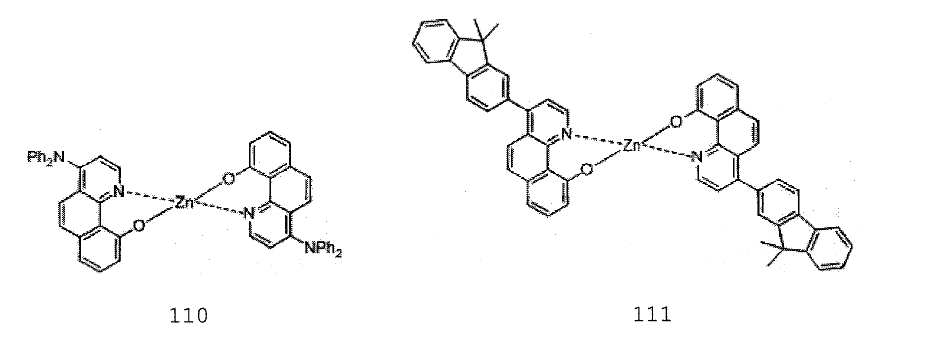

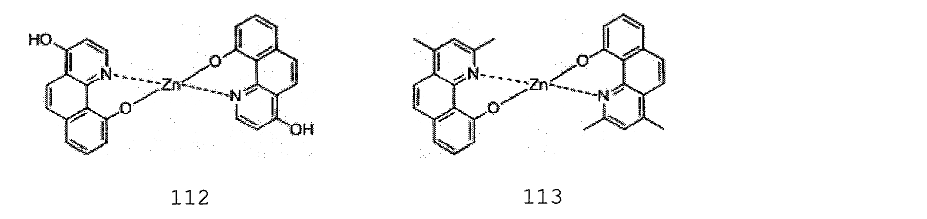

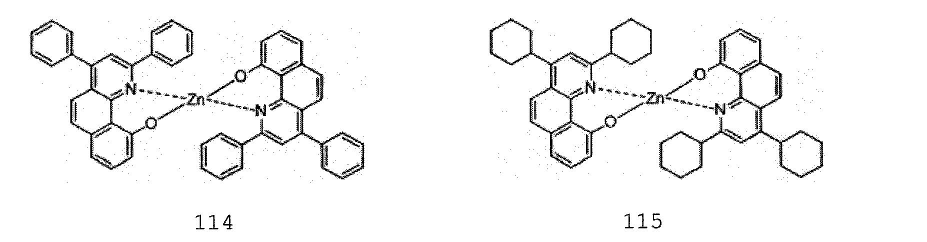

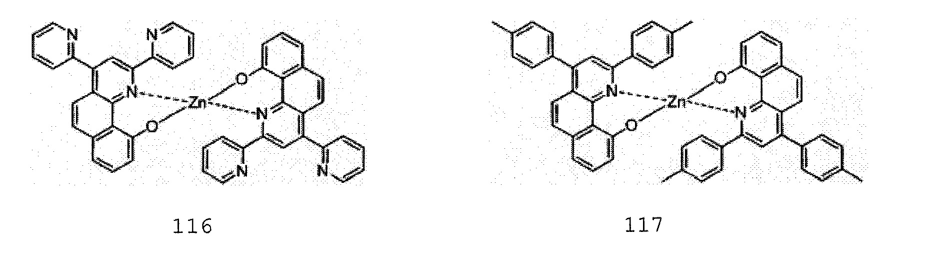

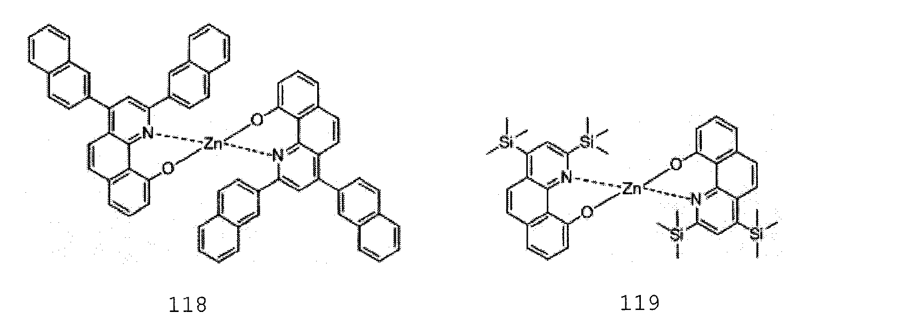

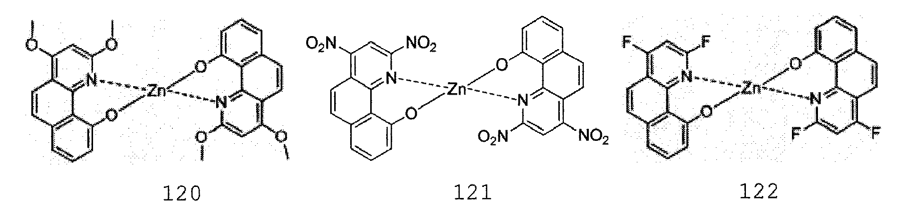

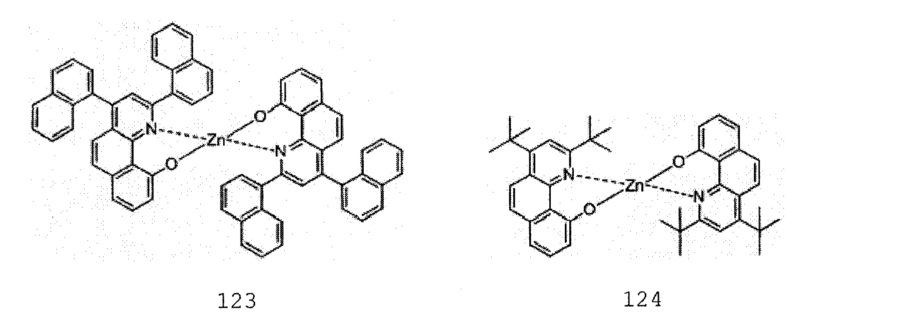

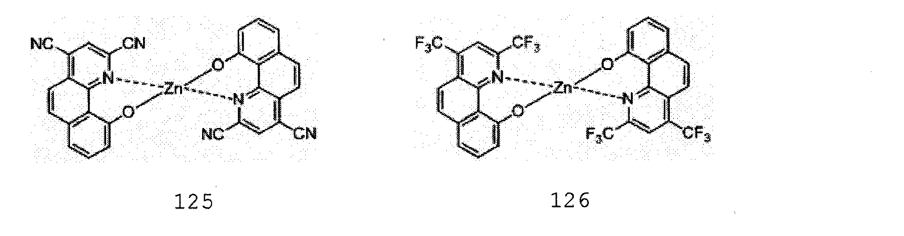

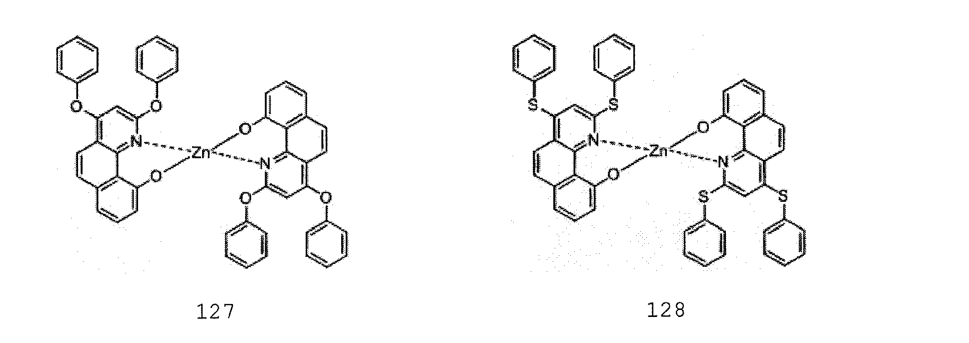

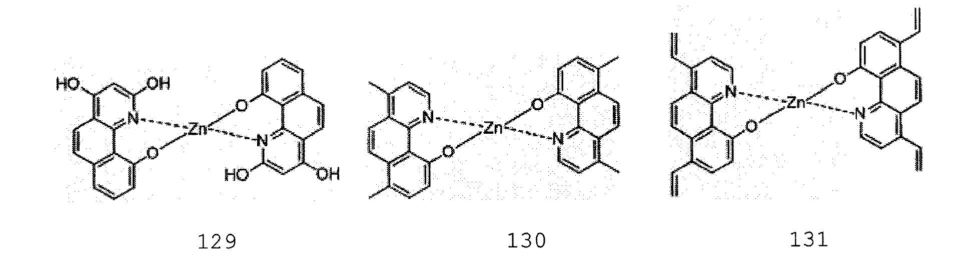

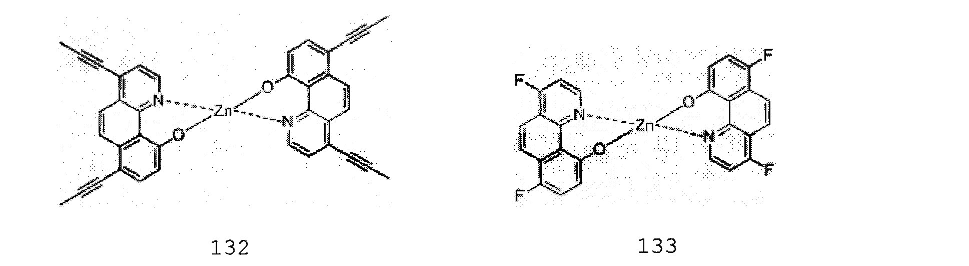

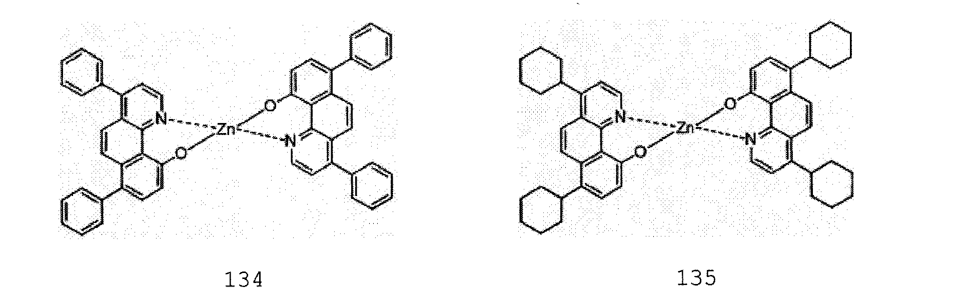

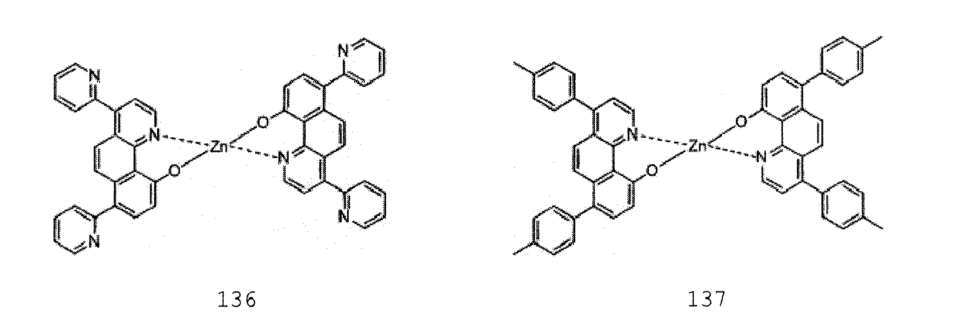

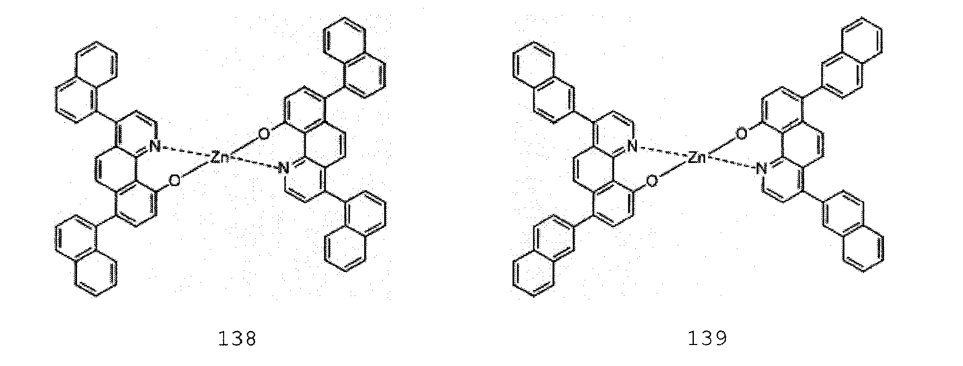

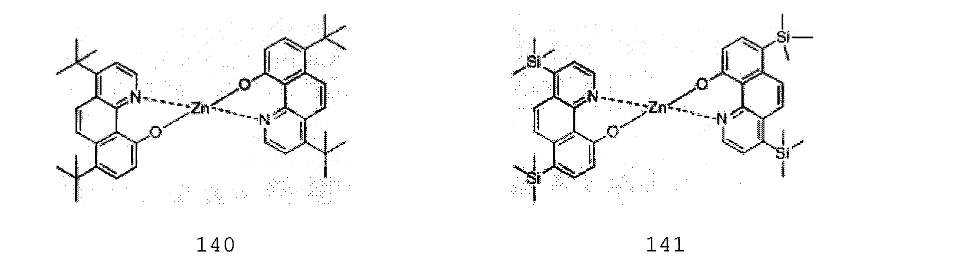

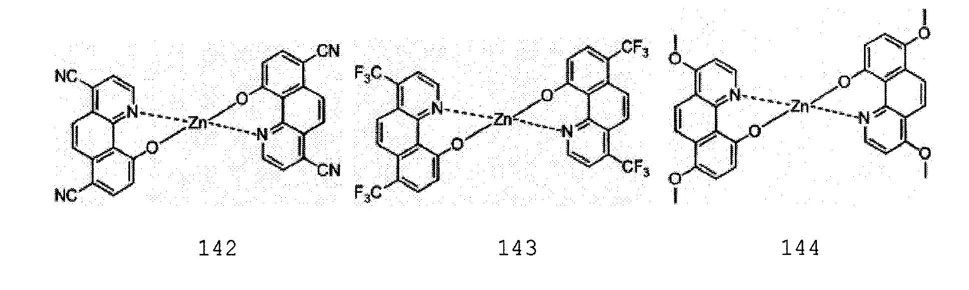

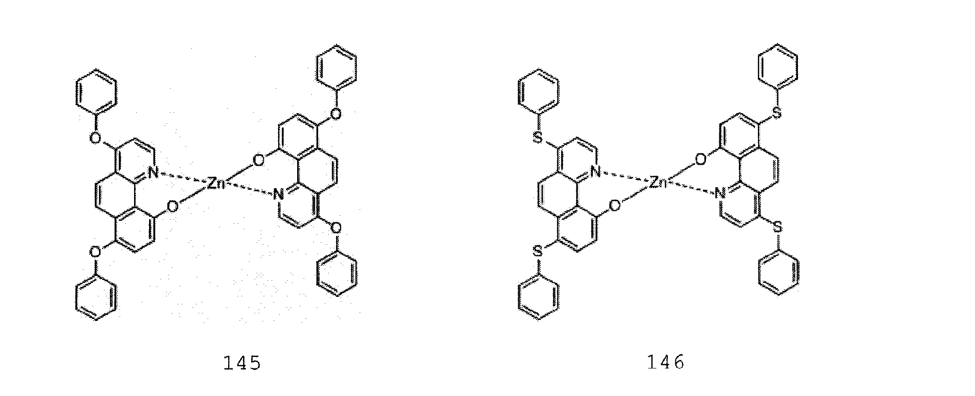

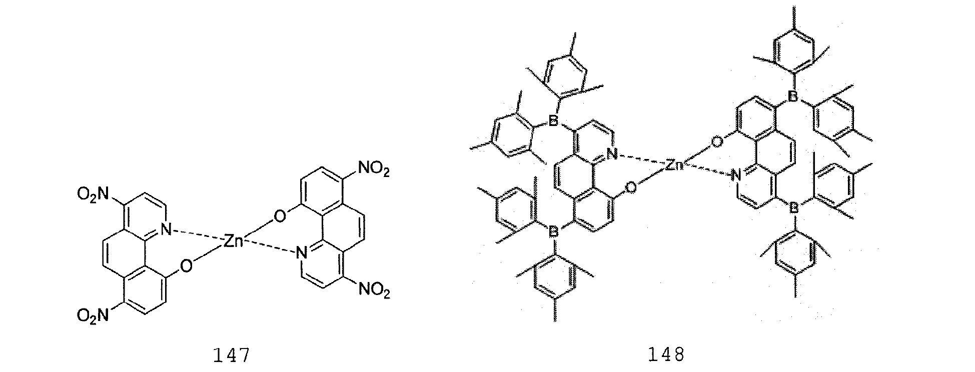

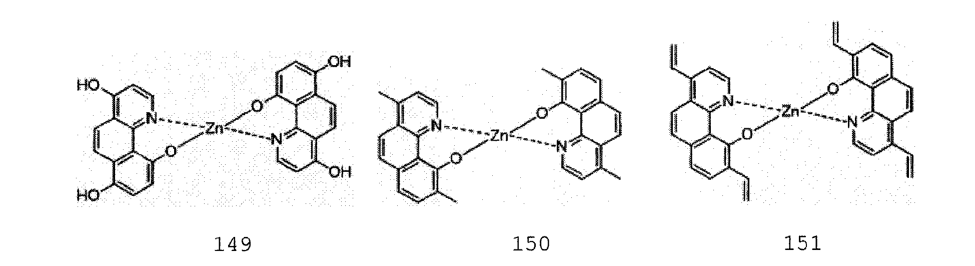

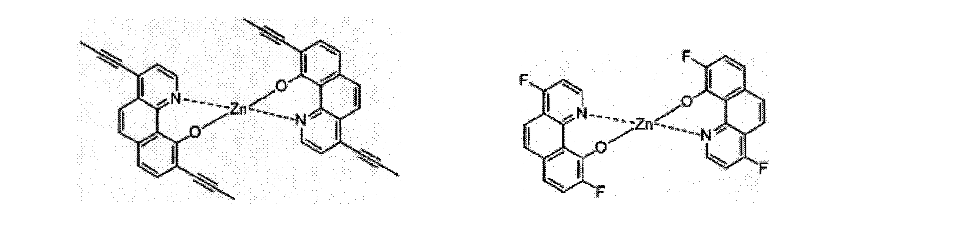

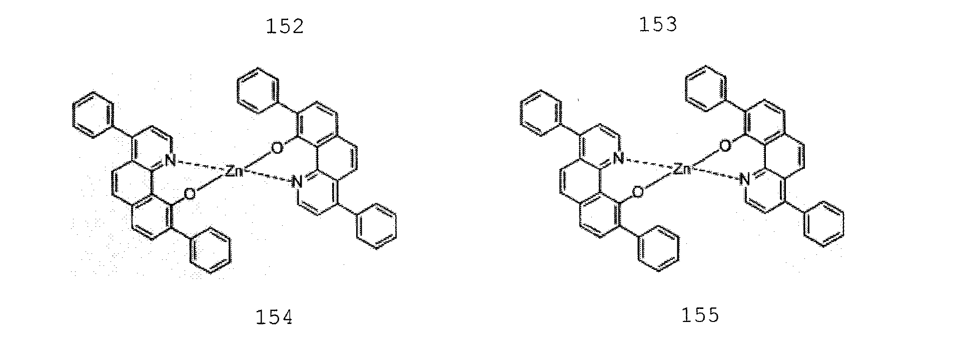

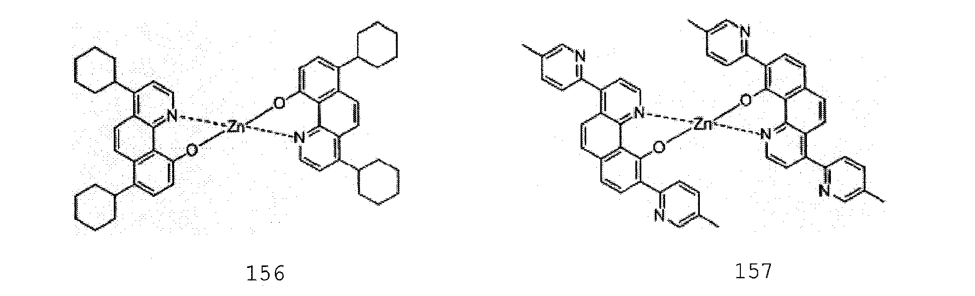

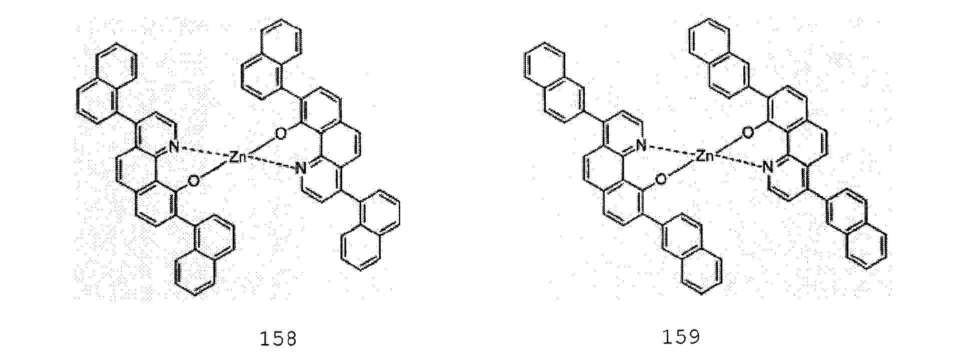

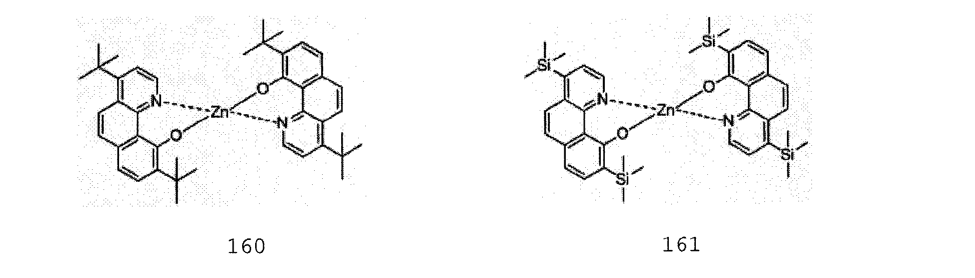

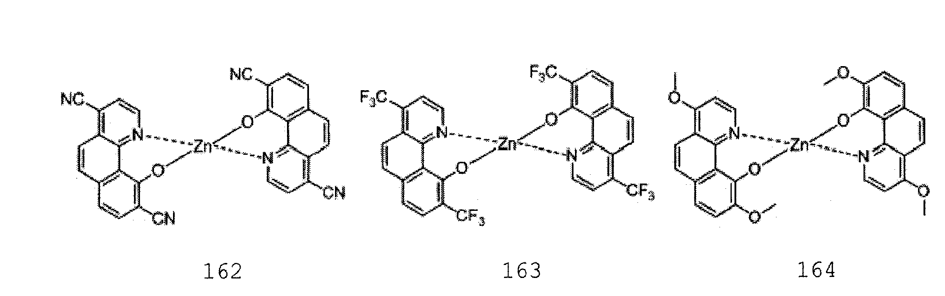

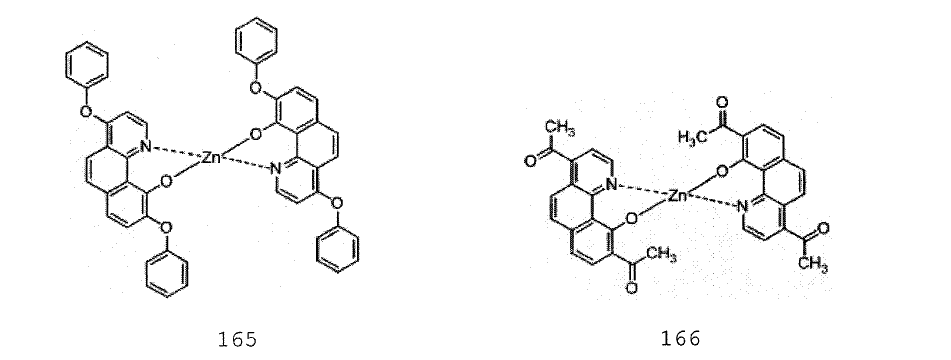

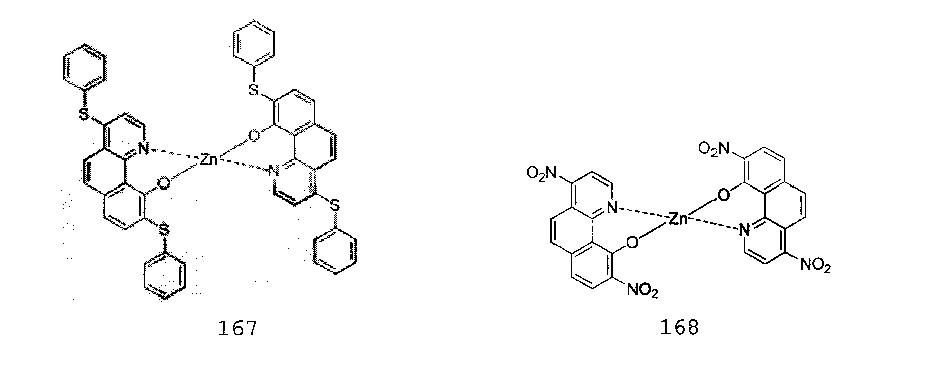

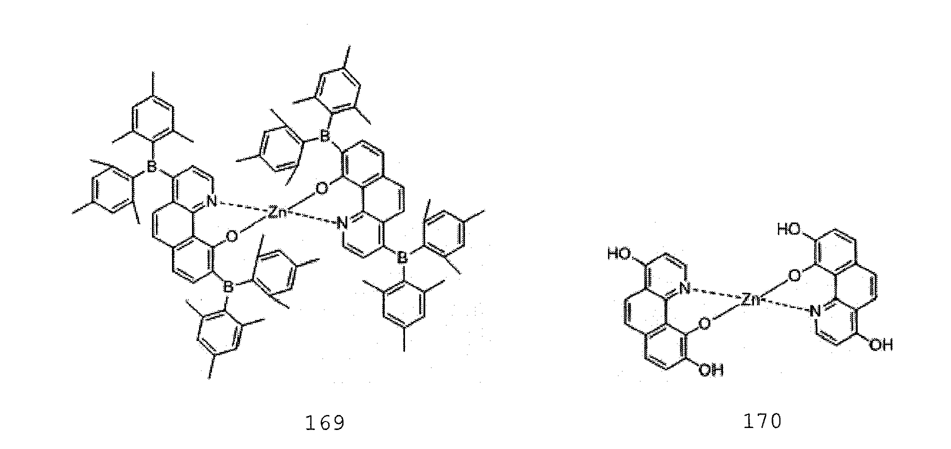

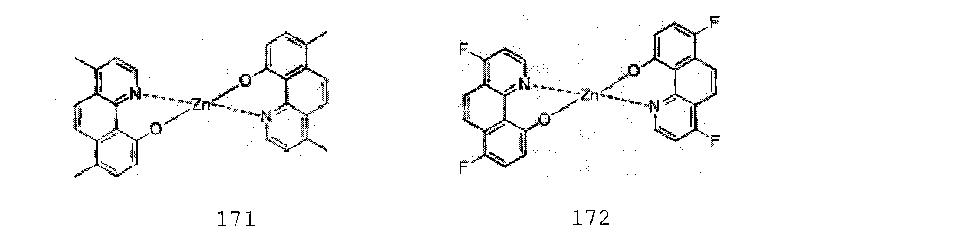

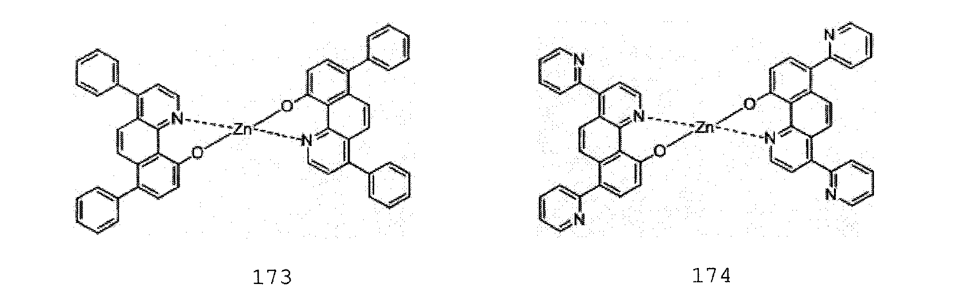

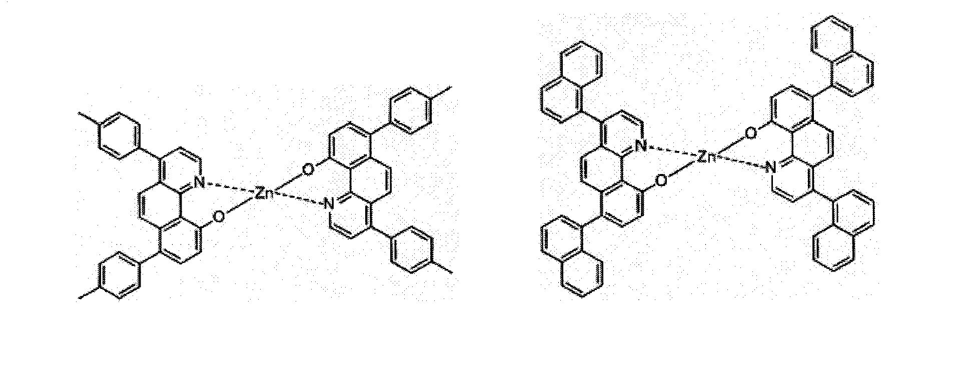

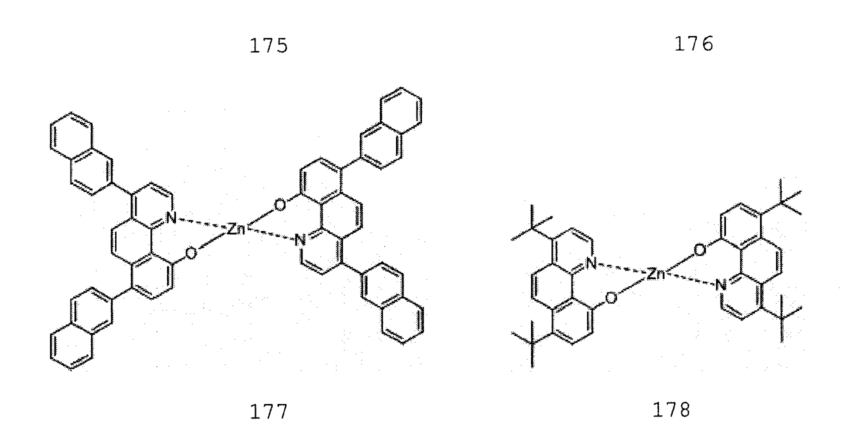

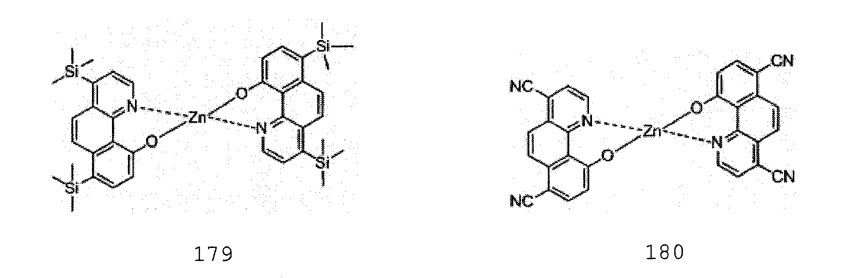

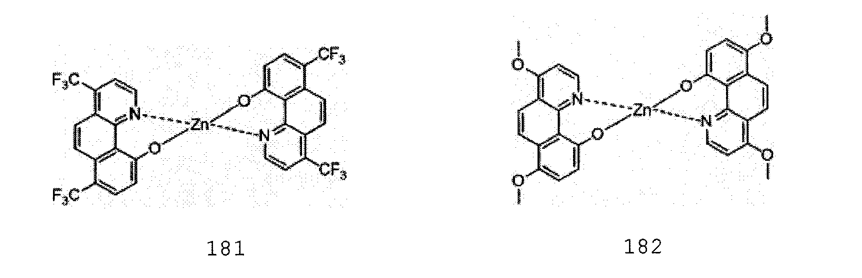

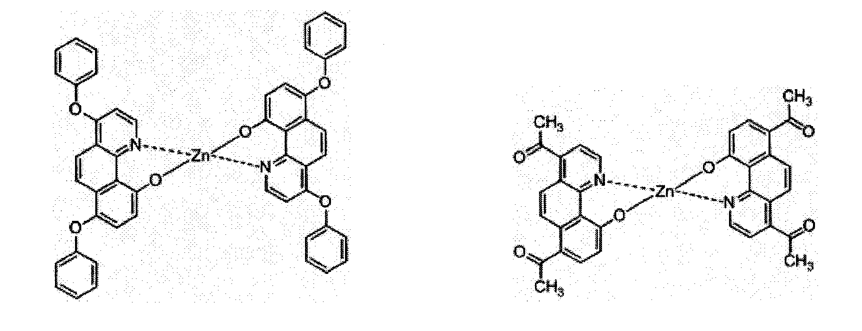

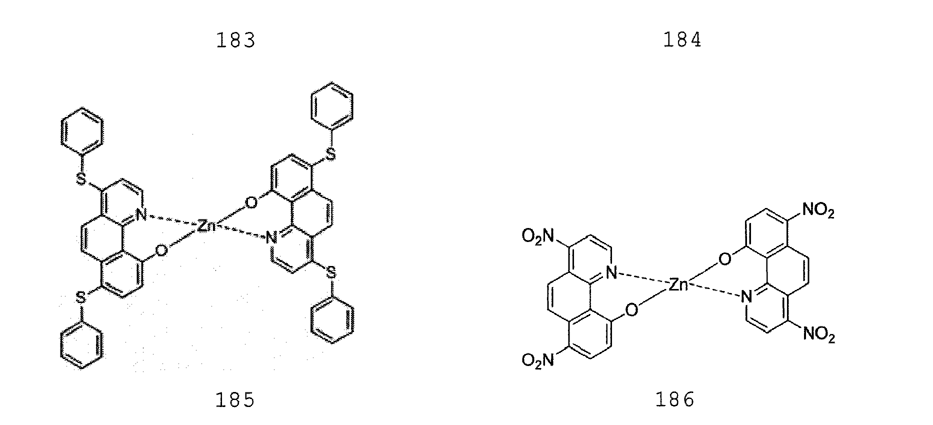

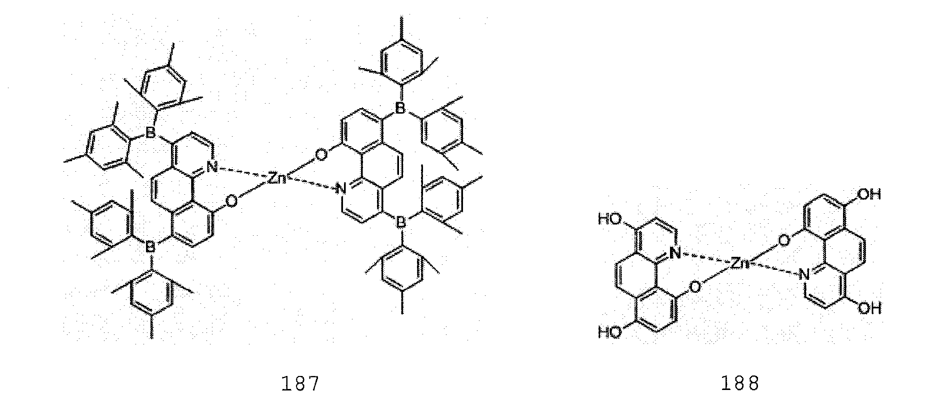

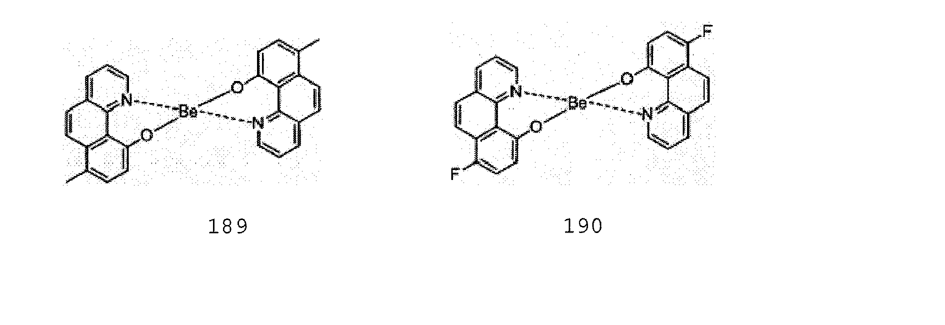

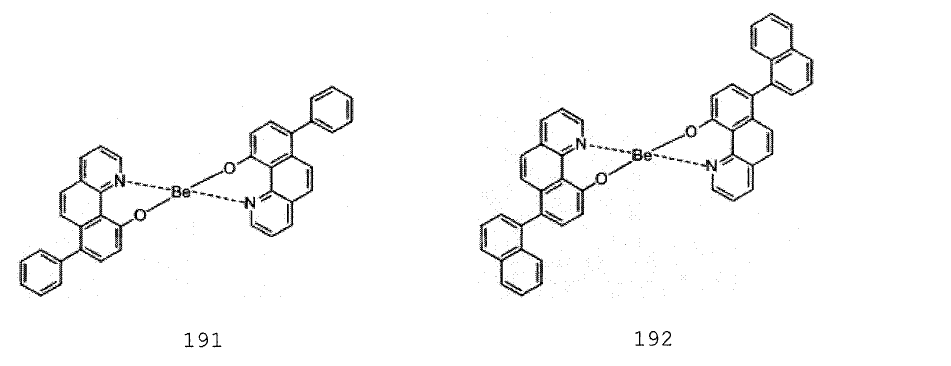

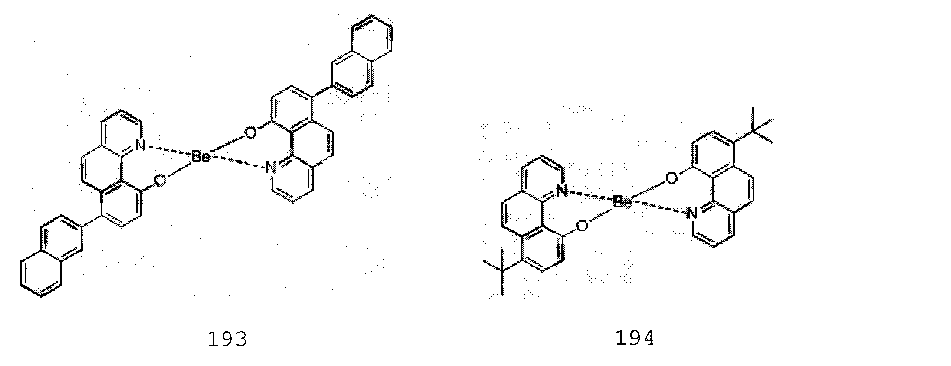

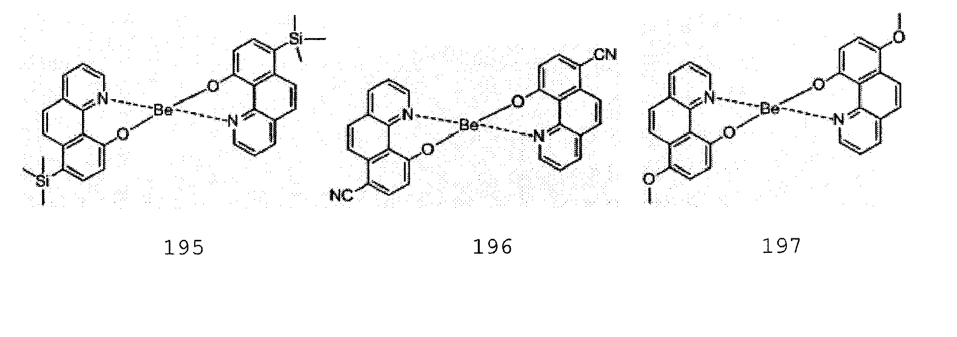

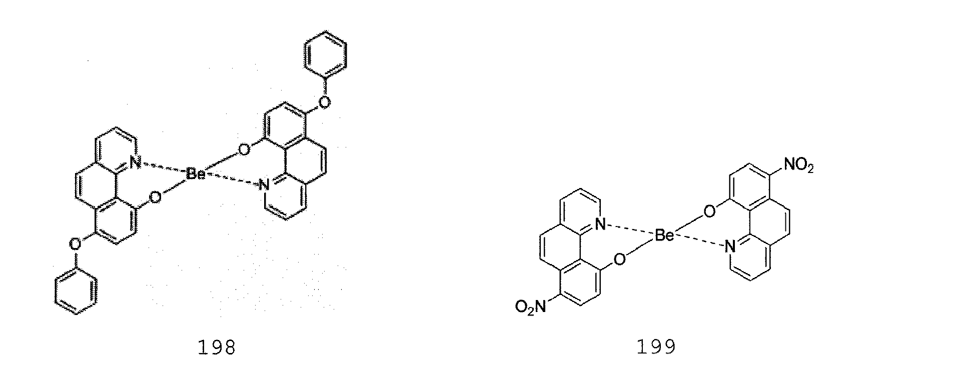

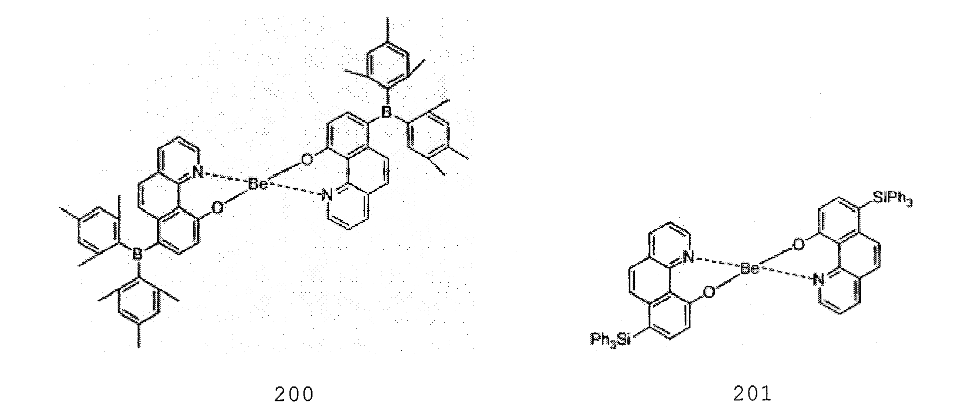

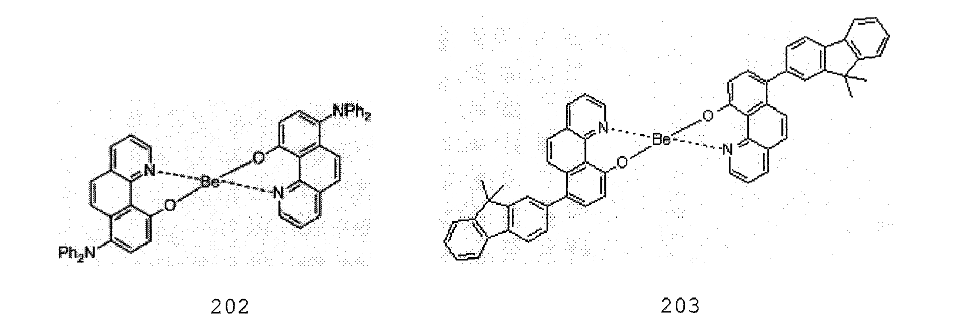

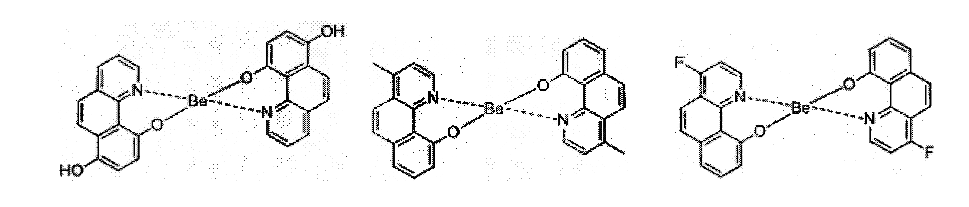

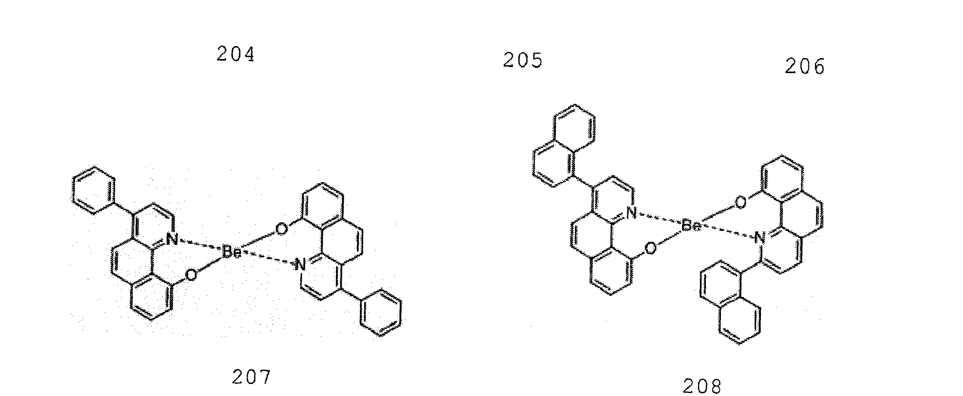

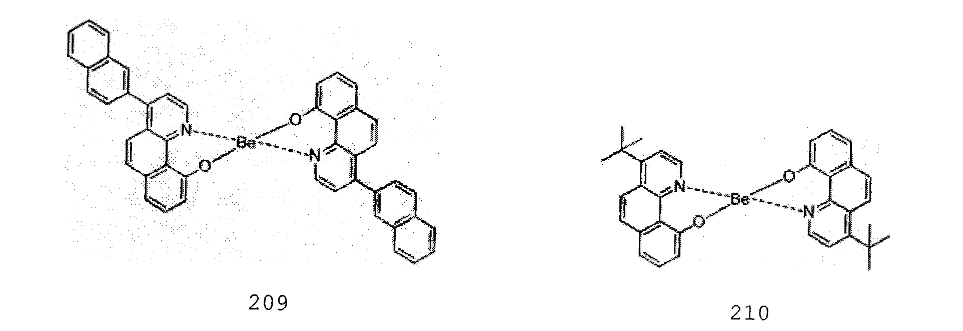

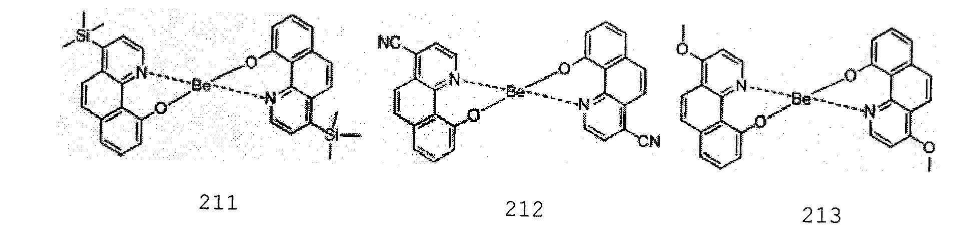

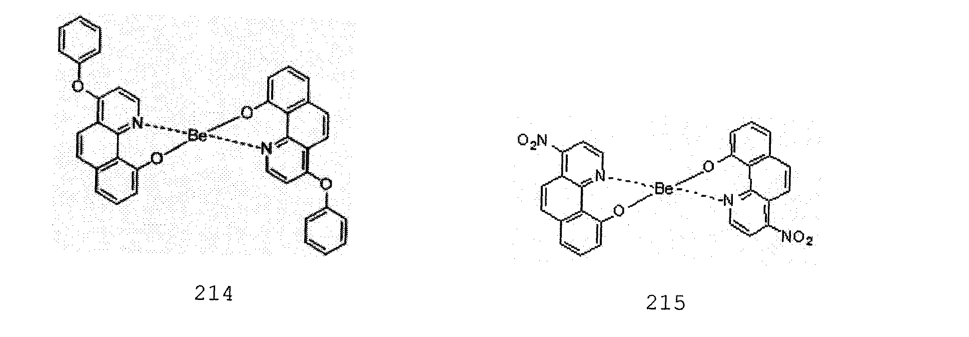

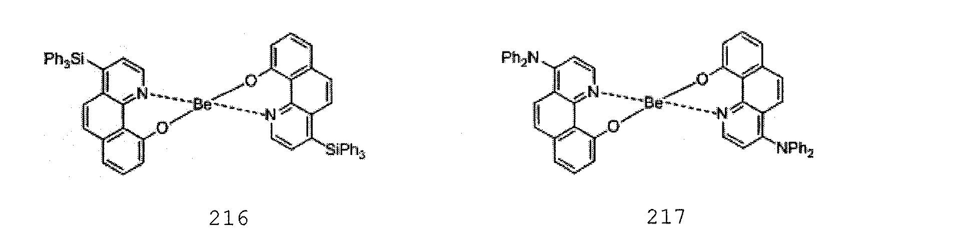

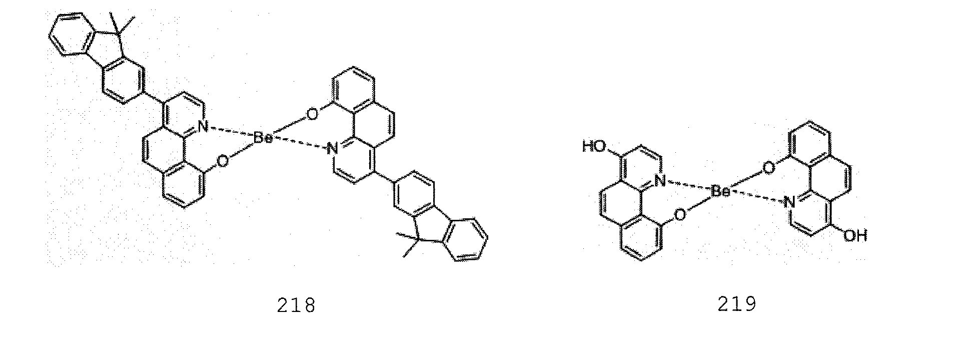

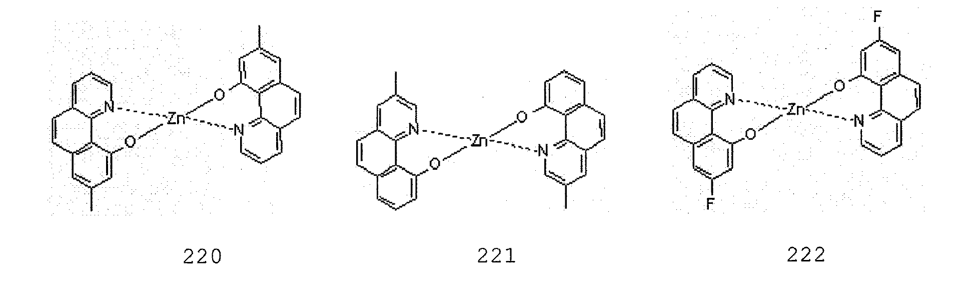

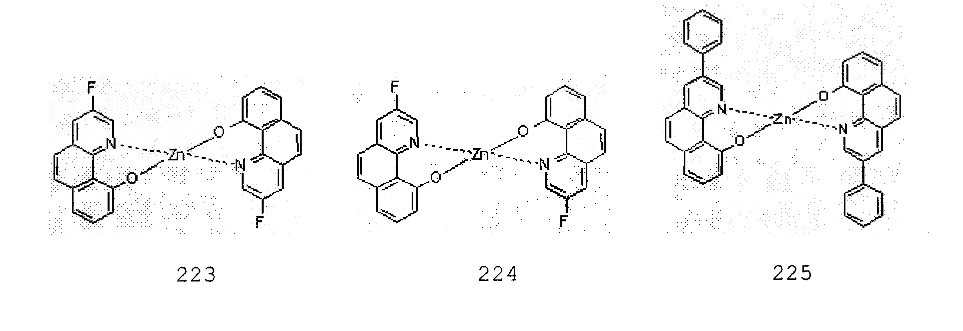

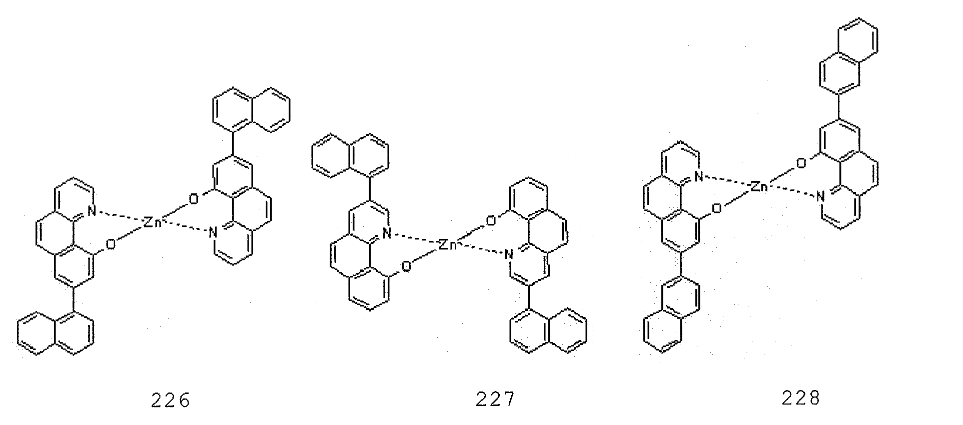

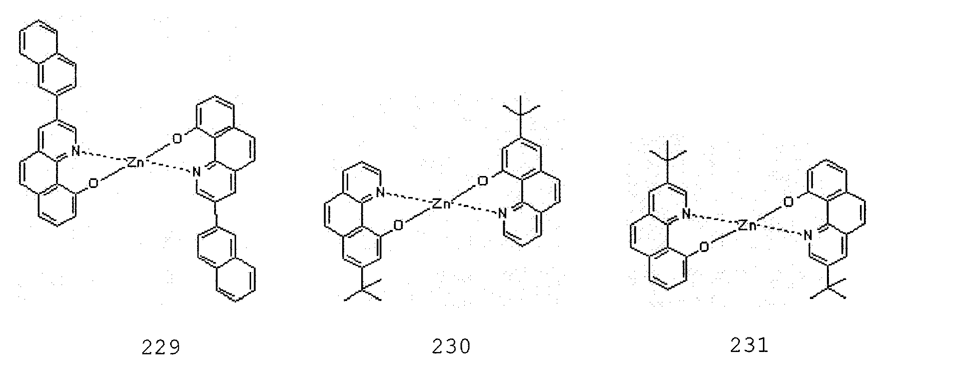

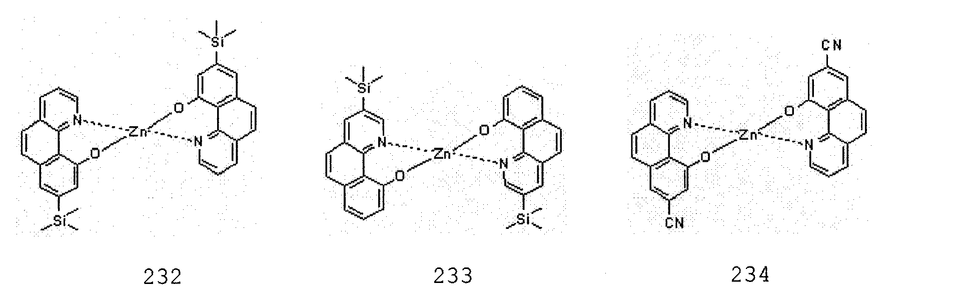

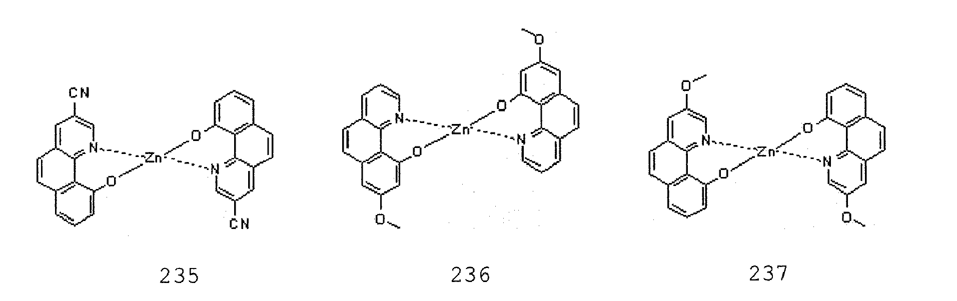

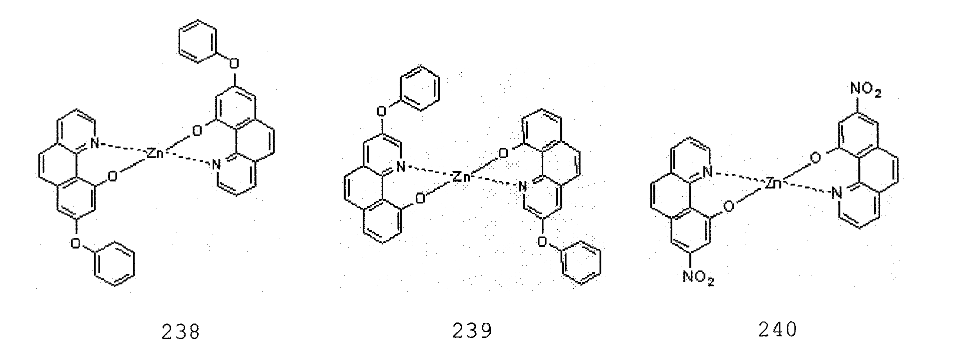

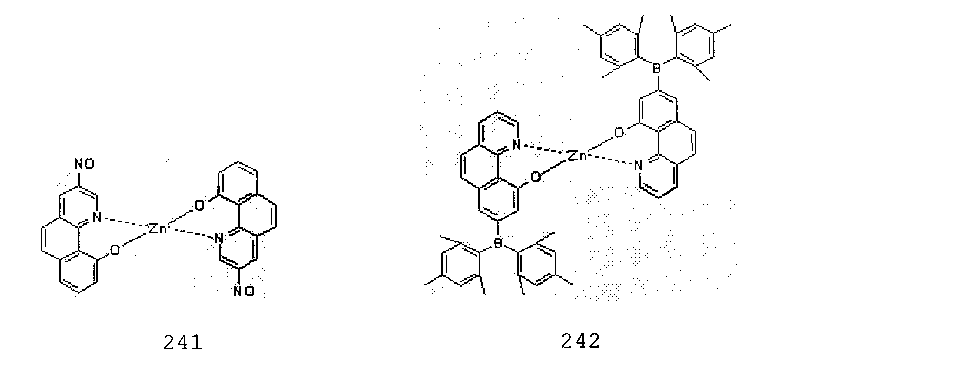

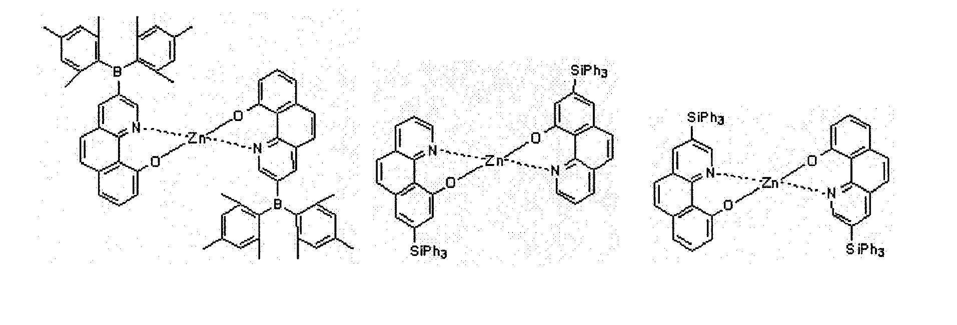

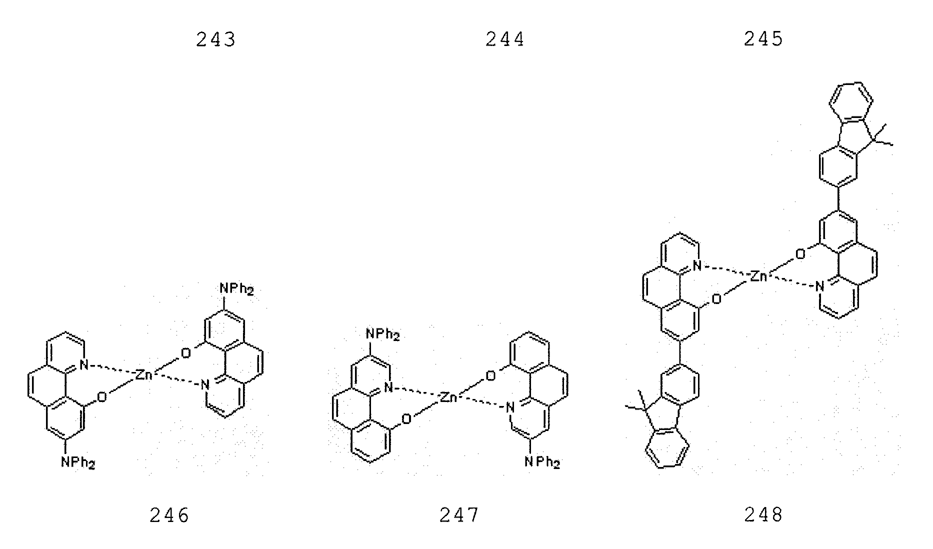

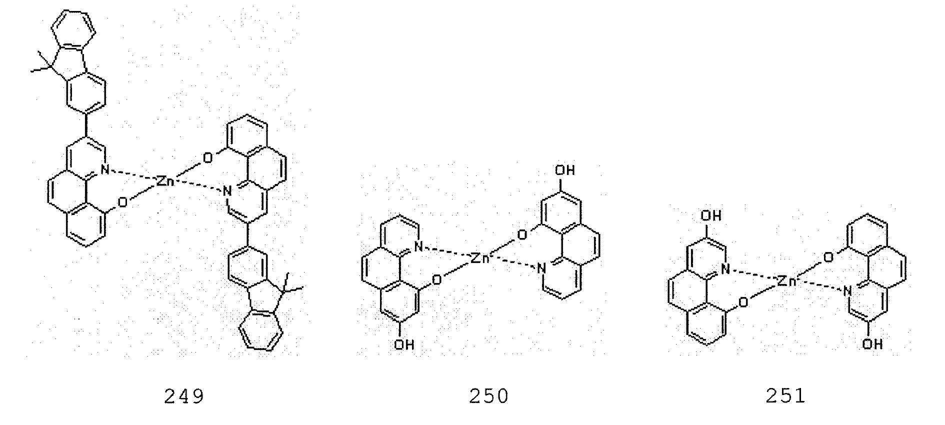

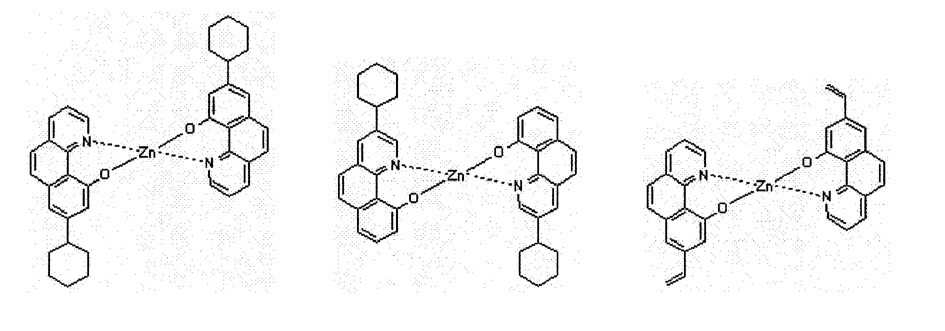

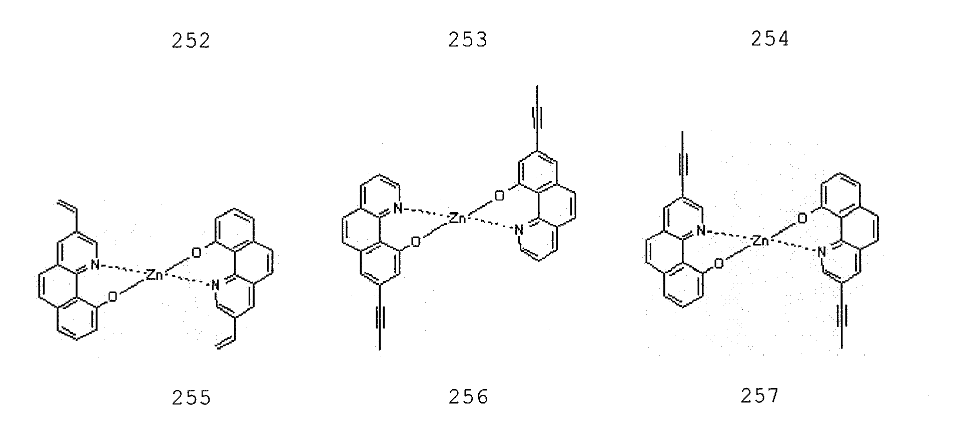

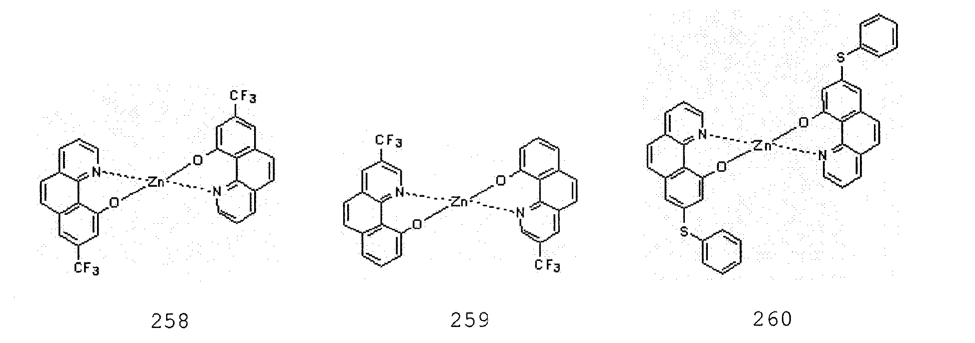

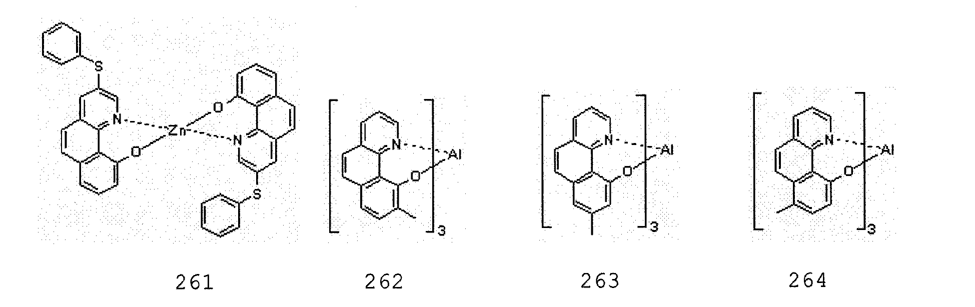

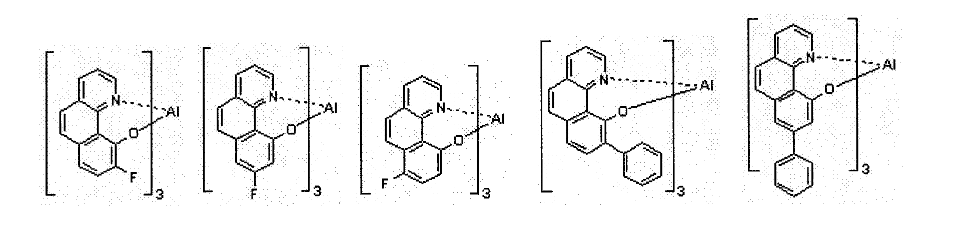

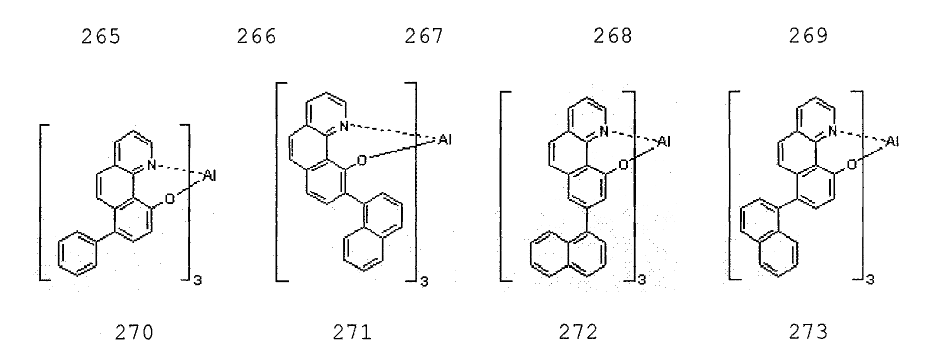

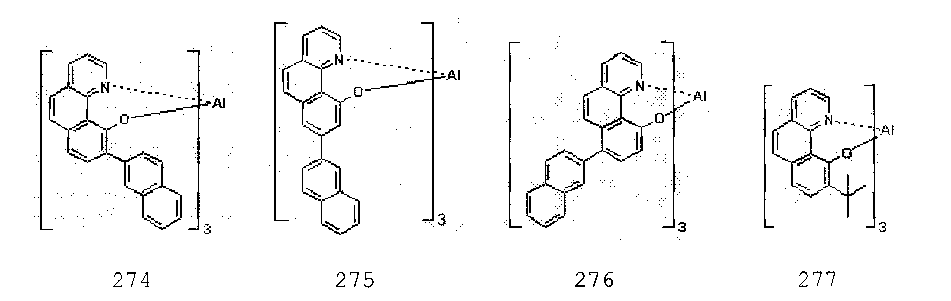

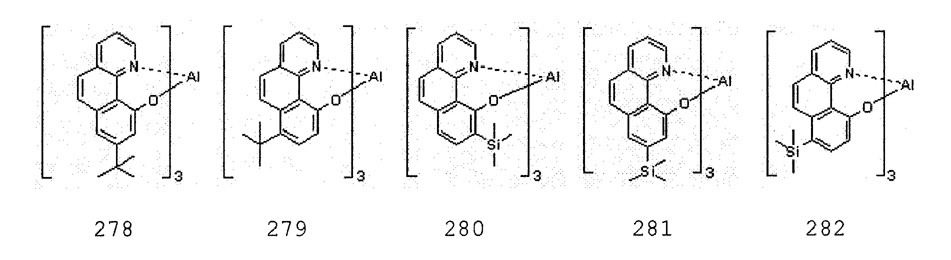

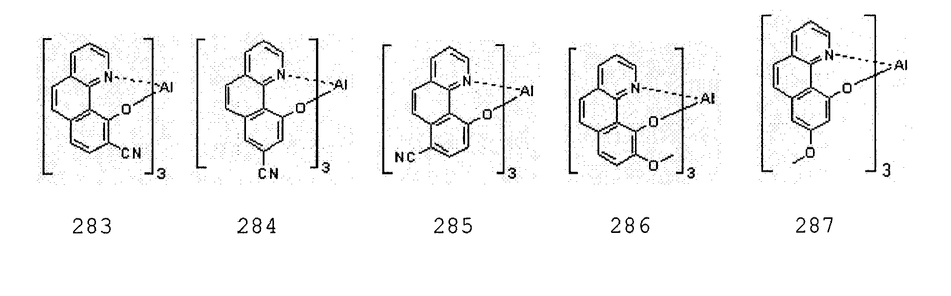

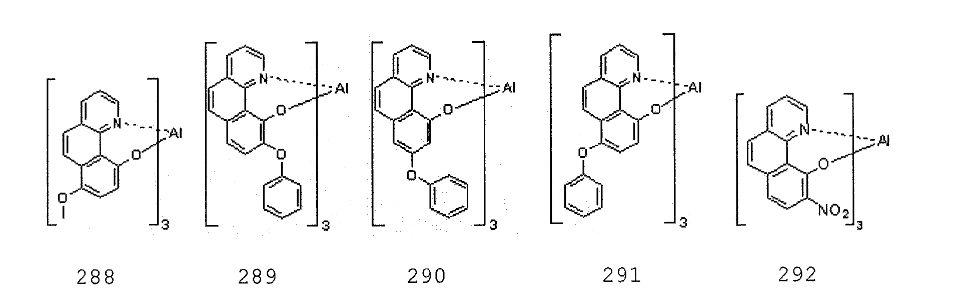

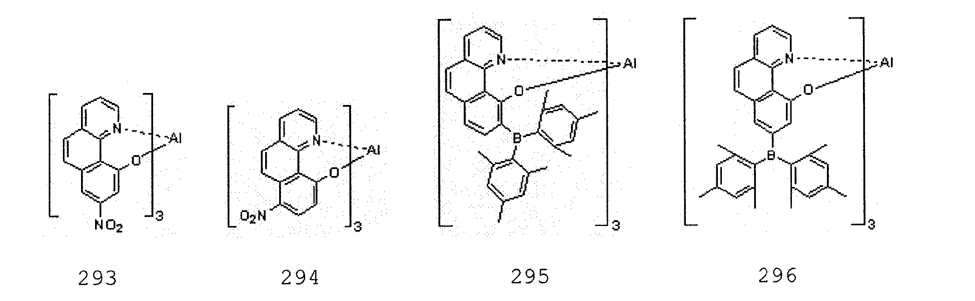

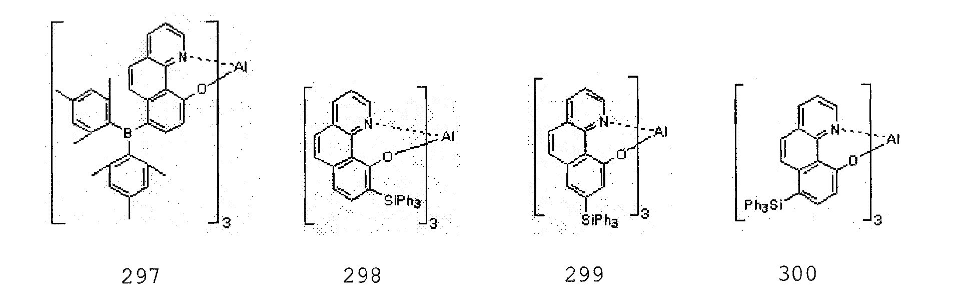

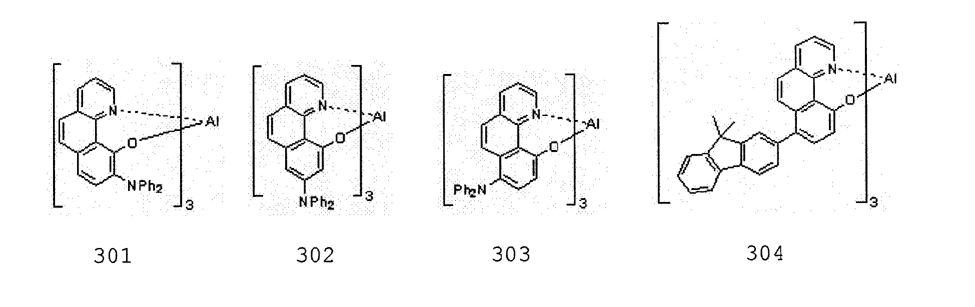

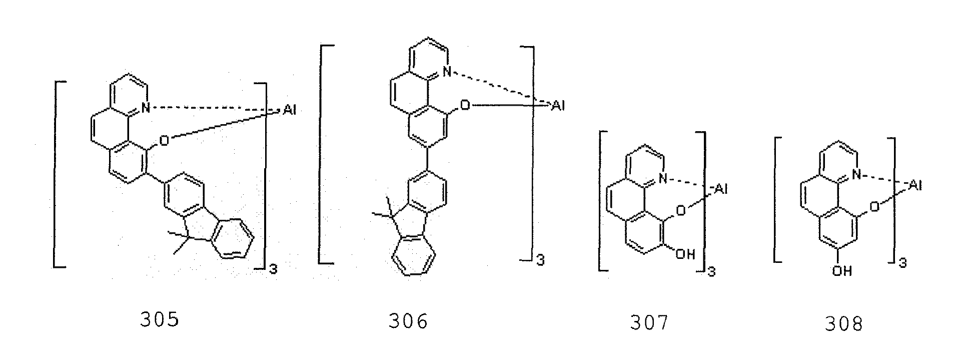

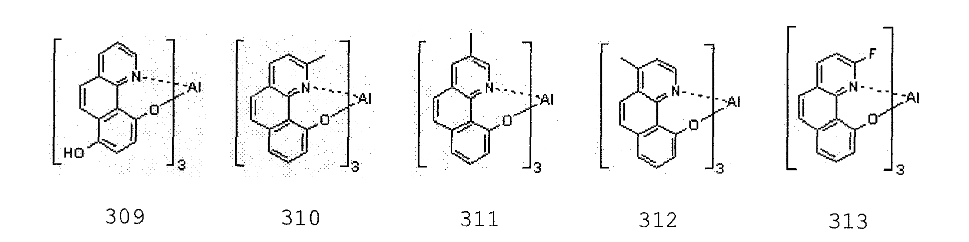

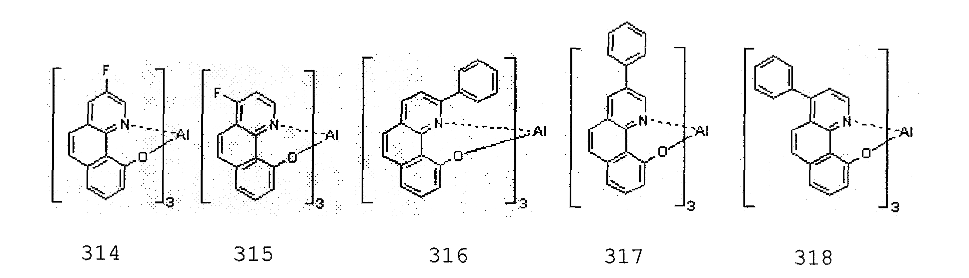

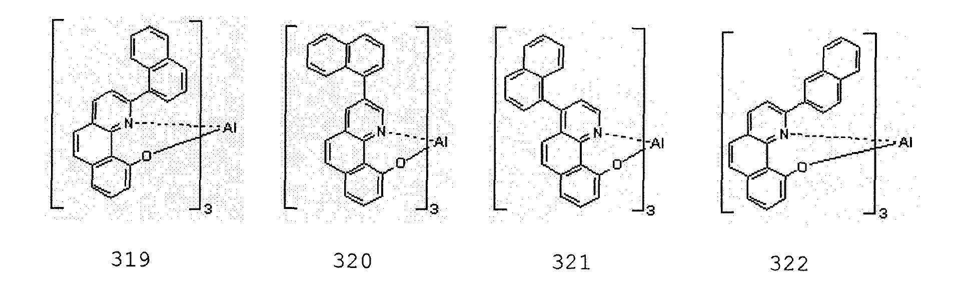

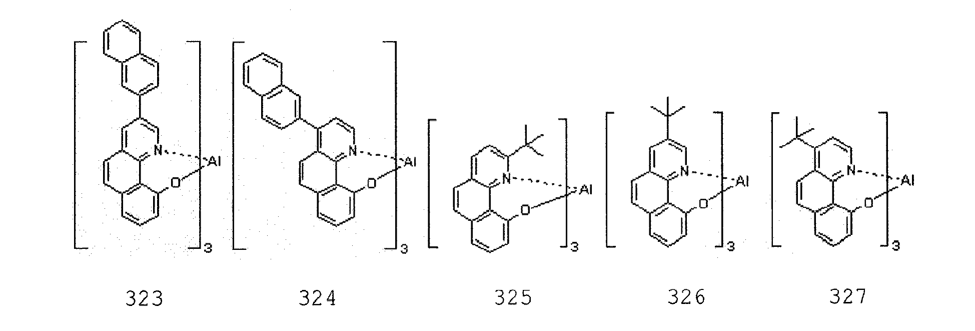

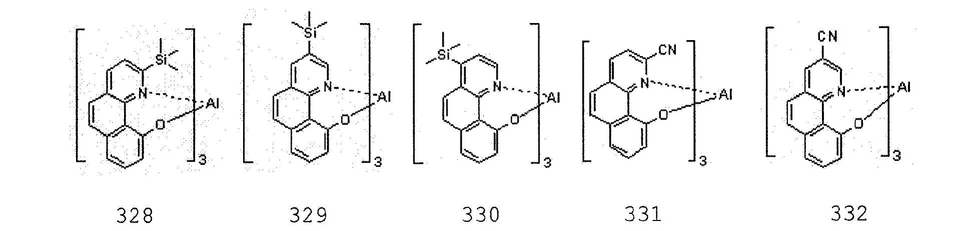

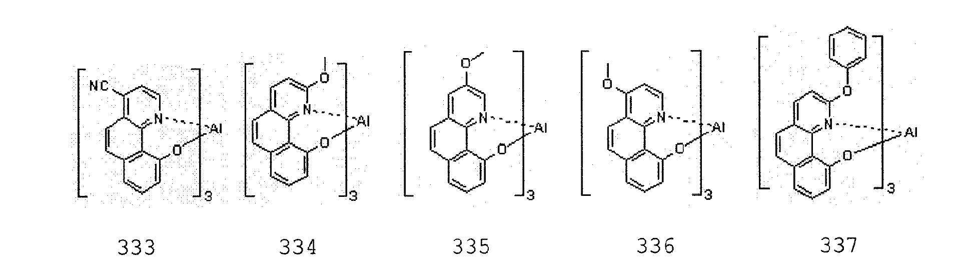

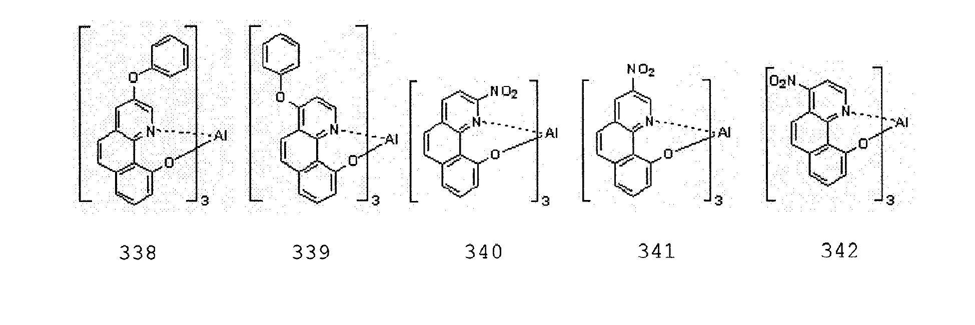

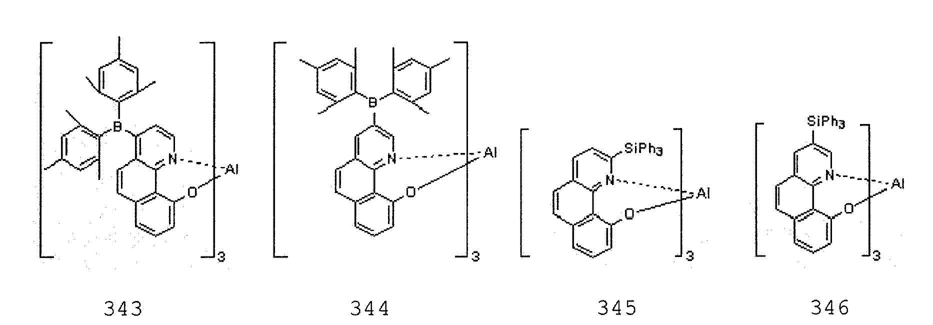

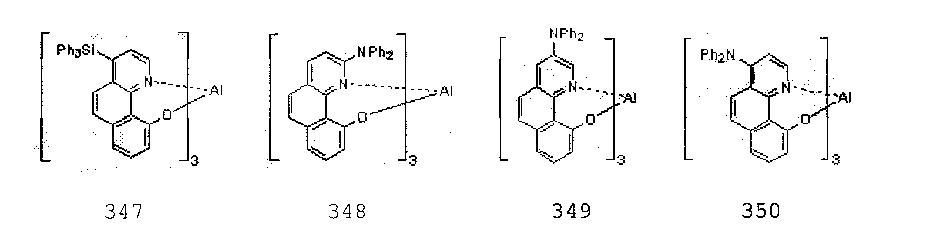

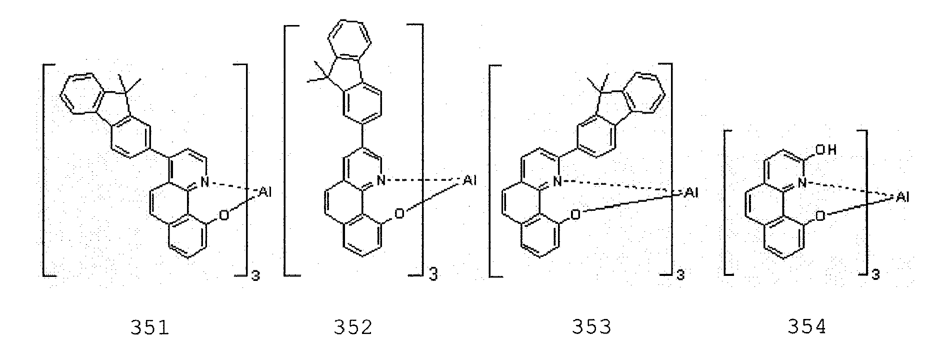

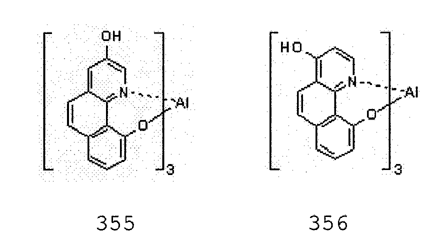

The present invention further provides an organic solar cell. The organic solar cell according to the present invention comprises one or more electroluminescent compound(s) represented by Chemical Formula 1.

The present invention further provides an electroluminescent device. The electroluminescent device according to the present invention comprises a first electrode; a second electrode; and one or more organic layer(s) interposed between the first electrode and the second electrode, wherein the organic layer comprises one or more electroluminescent compound(s) represented by Chemical Formula 1.

In the electroluminescent device according to the present invention, the organic layer further comprises an electroluminescent layer. The electroluminescent layer comprises one or more electroluminescent compound(s) represented by Chemical Formula 1, as an electroluminescent host, and one or more electroluminescent dopant(s). The electroluminescent dopant used in the electroluminescent device according to the present invention is not particularly limited, but may be exemplified by the compounds represented by Chemical Formula 2:

**M¹L¹⁰¹L¹⁰²L¹⁰³** (2)

wherein
M¹ is a metal selected from a group consisting of Group 7, Group 8, Group 9, Group 10, Group 11, Group 13, Group 14, Group 15 and Group 16 metals;
ligands L¹⁰¹, L¹⁰² and L¹⁰³ are independently selected from the following structures: 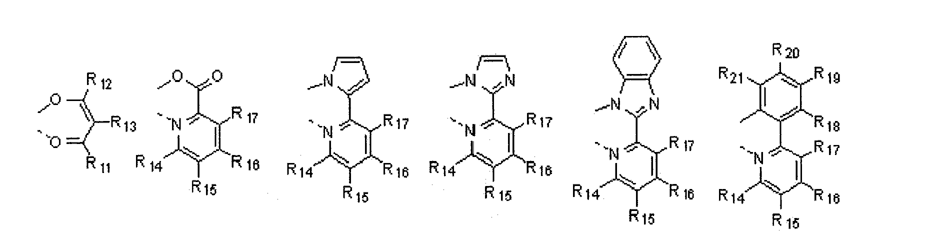 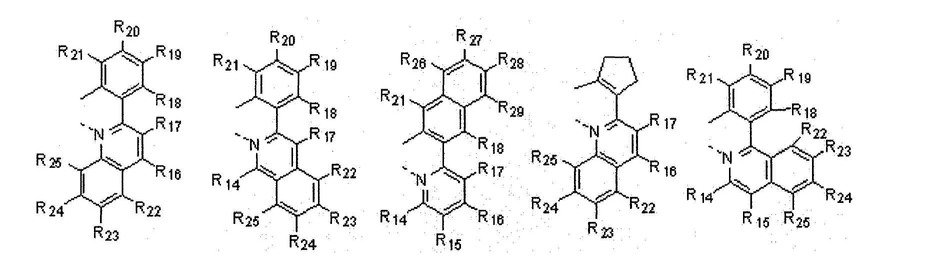 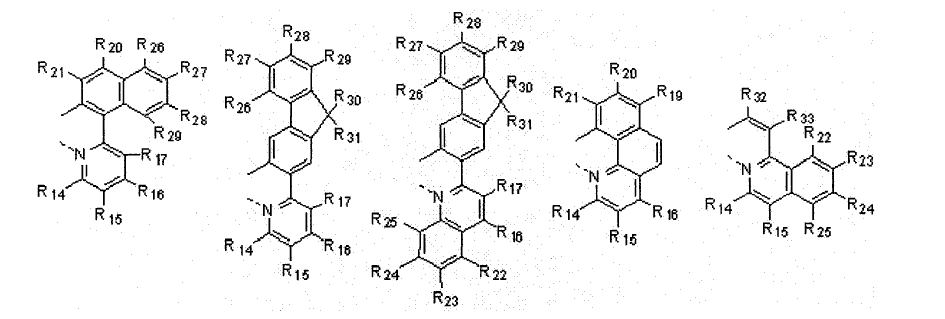 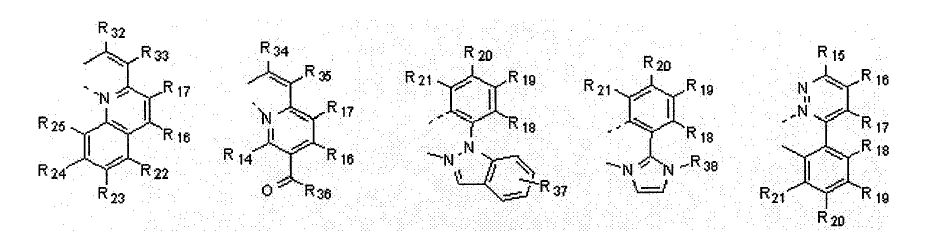 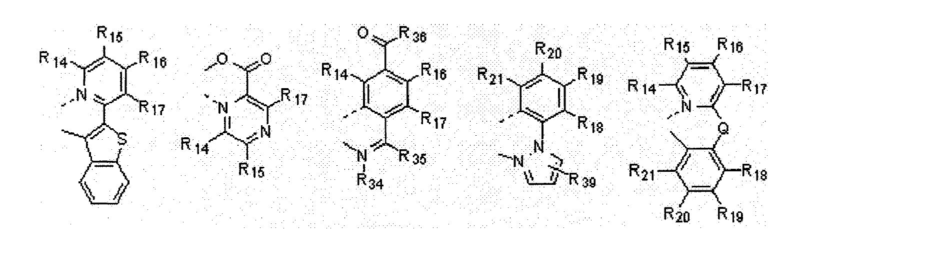 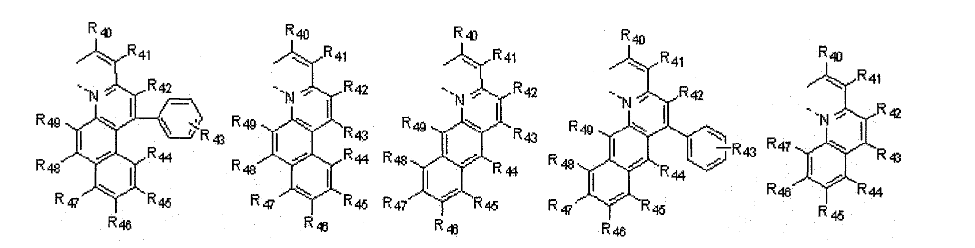 wherein
   R₁₁ through R₁₃ independently represent hydrogen, (C1-C60)alkyl with or without halogen substituent, (C6-C60)aryl with or without (C1-C60)alkyl substituent or halogen;
   R₁₄ through R₂₉ independently represent hydrogen, (C1-C60)alkyl, (C1-C30)alkoxy, (C3-C60)cycloalkyl, (C2-C30)alkenyl, (C6-C60)aryl, mono- or di(C1-C30)alkylamino, mono- or di(C6-C30)arylamino, SF₅, tri(C1-C30)alkylsilyl, di(C1-C30)alkyl(C6-C30)arylsilyl, tri(C6-C30)arylsilyl, cyano or halogen, and the alkyl, cycloalkyl, alkenyl or aryl of R₁₄ through R₂₉ may be further substituted by one or more substituent (s) selected from (C1-C60)alkyl, (C6-C60) aryl and halogen;
   R₃₀ through R₃₃ independently represent hydrogen, (C1-C60)alkyl with or without halogen substituent or (C6-C60)aryl with or without (C1-C60)alkyl substituent;
   R₃₄ and R₃₅ independently represent hydrogen, (C1-C60)alkyl, (C6-C60)aryl or halogen, or R₃₄ and R₃₅ may be linked via (C3-C12)alkylene or (C3-C12) alkenylene with or without a fused ring to form an alicyclic ring or a monocyclic or polycyclic aromatic ring, and the alkyl or aryl of R₃₄ and R₃₅, or the alicyclic ring or the monocyclic or polycyclic aromatic ring formed as they are linked via (C3-C12)alkylene or (C3-C12)alkenylene with or without a fused ring may be further substituted by one or more substituent (s) selected from (C1-C60)alkyl with or without halogen substituent, (C1-C30)alkoxy, halogen, tri(C1-C30)alkylsilyl, tri(C6-C30)arylsilyl and (C6-C60)aryl;
   R₃₆ represents (C1-C60)alkyl, (C6-C60)aryl, (C5-C60)heteroaryl containing one or more heteroatom (s) selected from N, O and S or halogen;
   R₃₇ through R₃₉ independently represent hydrogen, (C1-C60)alkyl, (C6-C60)aryl or halogen, and the alkyl or aryl of R₃₆ through R₃₉ may be further substituted halogen or (C1-C60)alkyl;
   R₄₀ and R₄₁ independently represent hydrogen, (C1-C20)alkyl with or without halogen substituent, (C6-C20)aryl, halogen, cyano, tri(C1-C20)alkylsilyl, di(C1-C20)alkyl(C6-C20)arylsilyl, tri(C6-C20)arylsilyl, (C1-C20)alkoxy, (C1-C20)alkylcarbonyl, (C6-C20)arylcarbonyl, di(C1-C20)alkylamino or di(C6-C20)arylamino, or R₄₀ and R₄₁ may be linked via (C3-C12)alkylene or (C3-C12)alkenylene with or without a fused ring to form an alicyclic ring or a monocyclic or polycyclic aromatic ring;
   the alkyl or aryl of R₄₀ and R₄₁, or the alicyclic ring or the monocyclic or polycyclic aromatic ring formed as they are linked via (C3-C12)alkylene or (C3-C12)alkenylene with or without a fused ring may be further substituted by one or more substituent(s) selected from (C1-C20)alkyl with or without halogen substituent, halogen, cyano, tri(C1-C20)alkylsilyl, di(C1-C20)alkyl(C6-C20)arylsilyl, tri(C6-C20)arylsilyl, (C1-C20)alkoxy, (C1-C20)alkylcarbonyl, (C6-C20)arylcarbonyl, di(C1-C20)alkylamino, di(C6-C20)arylamino, phenyl, naphthyl, anthryl, fluorenyl and spirobifluorenyl, or may be further substituted by phenyl or fluorenyl with one or more substituent(s) selected from a group consisting of (C1-C20)alkyl with or without halogen substituent, halogen, cyano, tri(C1-C20)alkylsilyl, di(C1-C20)alkyl(C6-C20)arylsilyl, tri(C6-C20)arylsilyl, (C1-C20)alkoxy, (C1-C20)alkylcarbonyl, (C6-C20)arylcarbonyl, di(C1-C20)alkylamino, di(C6-C20)arylamino, phenyl, naphthyl, anthryl, fluorenyl and spirobifluorenyl;
   R₄₂ through R₄₉ independently represent hydrogen, (C1-C20)alkyl with or without halogen substituent, (C1-C20)alkoxy, (C3-C12)cycloalkyl, halogen, cyano, (C6-C20)aryl, (C4-C20)heteroaryl, tri(C1-C20)alkylsilyl, di(C1-C20)alkyl(C6-C20)arylsilyl or tri(C6-C20)arylsilyl; and
   Q represents 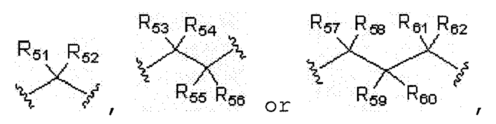 wherein R₅₁ through R₆₂ 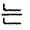 independently represent hydrogen, (C1-C60)alkyl with or without halogen substituent, (C1-C30)alkoxy, halogen, (C6-C60)aryl, cyano or (C5-C60)cycloalkyl, or each of R₅₁ through R₆₂ may be linked to an adjacent substituent via alkylene or alkenylene to form a (C5-C7)spiro ring or a (C5-C9)fused ring, or may be linked to R₁₇ or R₁₈ via alkylene or alkenylene to form a (C5-C7)fused ring.
   M¹ is selected from Ir, Pt, Pd, Rh, Re, Os, Tl, Pb, Bi, In, Sn, Sb, Te, Au and Ag. Specifically, the compound represented by Chemical Formula 2 may be exemplified the following compounds, but not limited thereto:

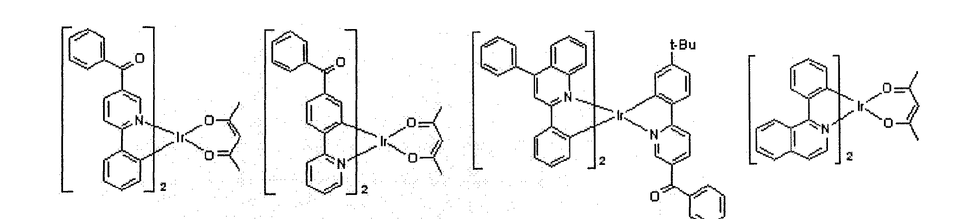

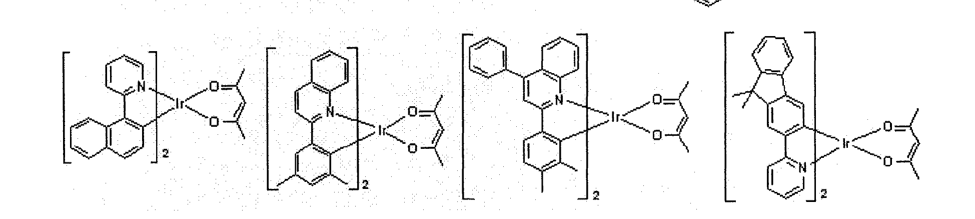

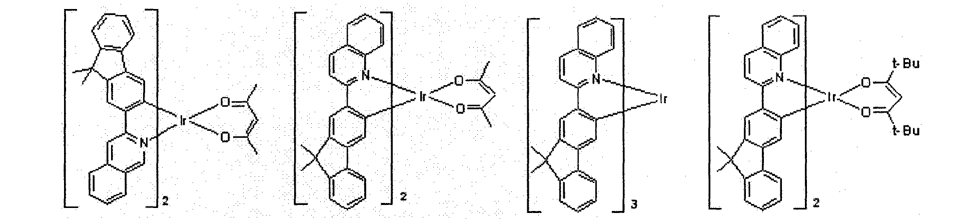

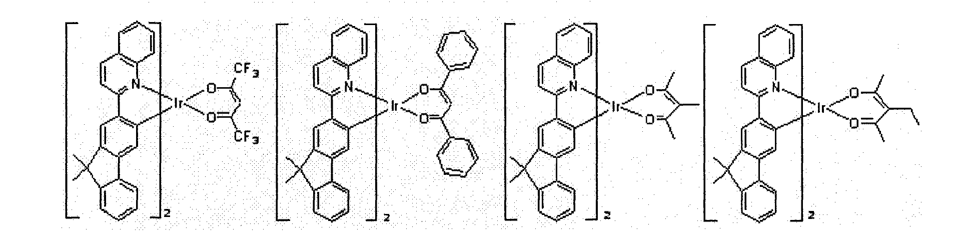

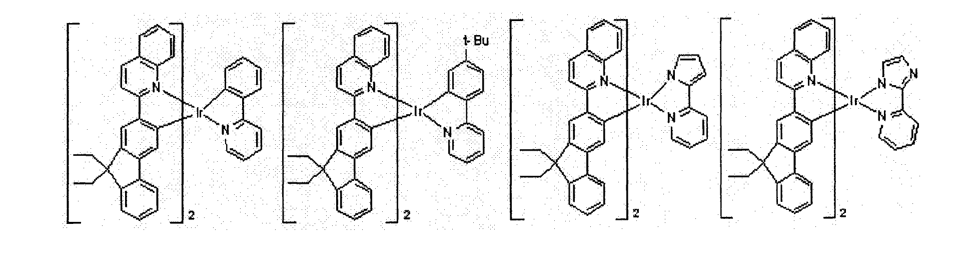

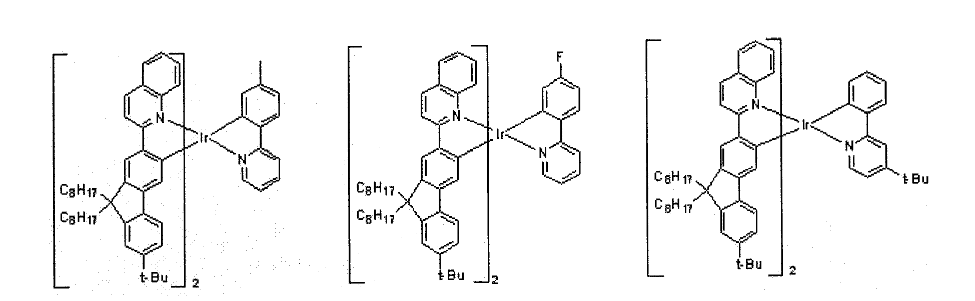

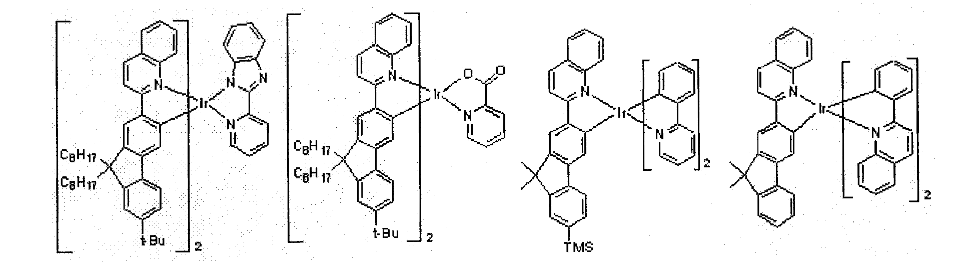

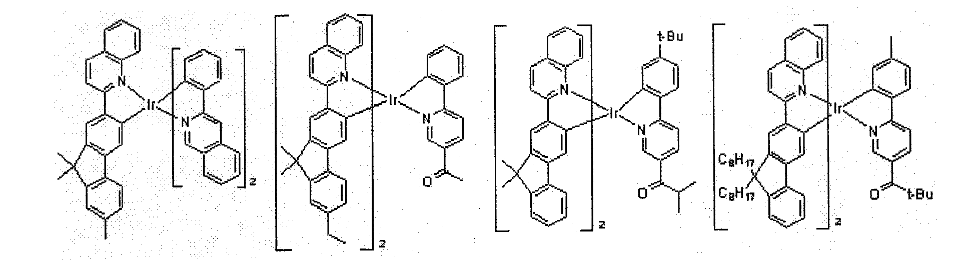

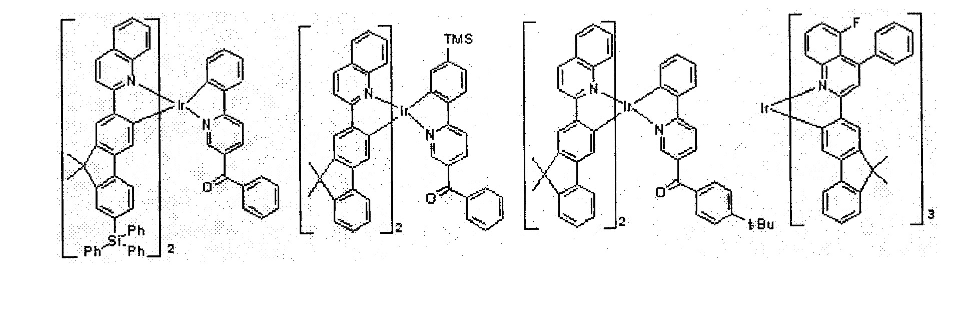

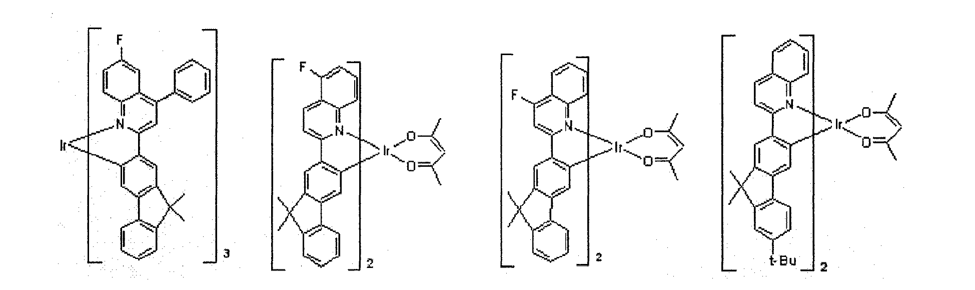

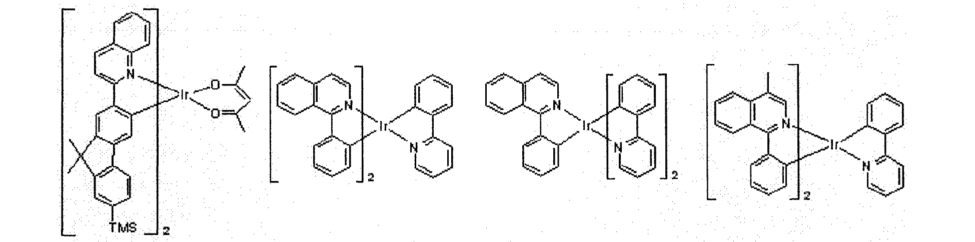

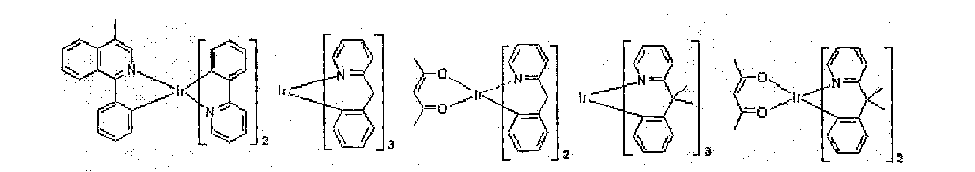

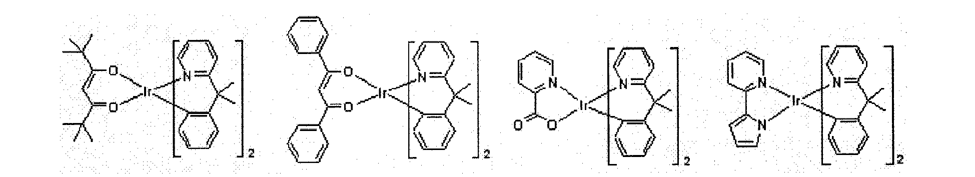

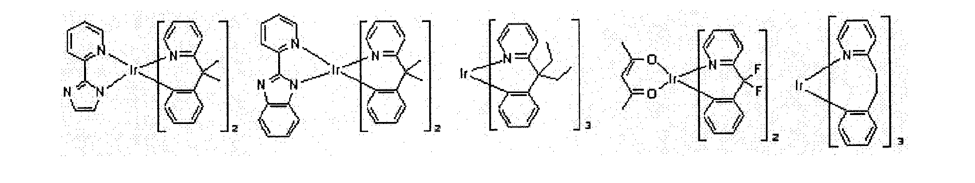

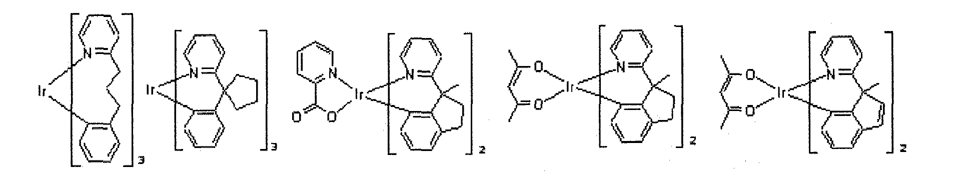

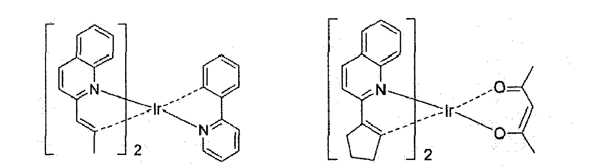

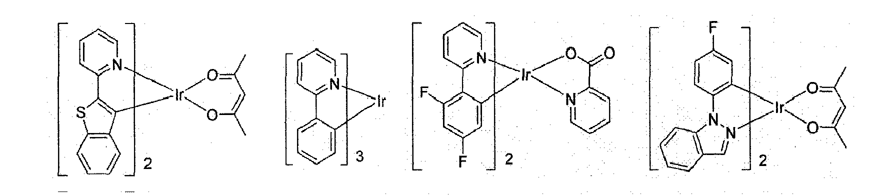

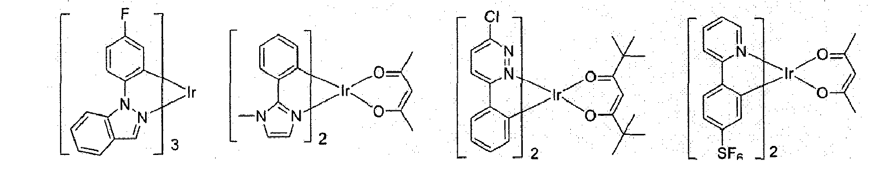

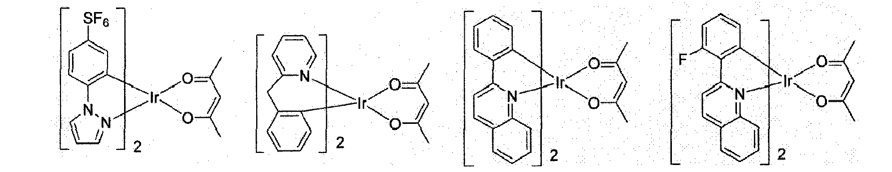

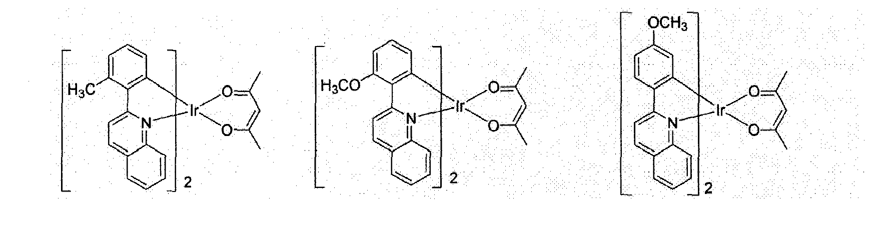

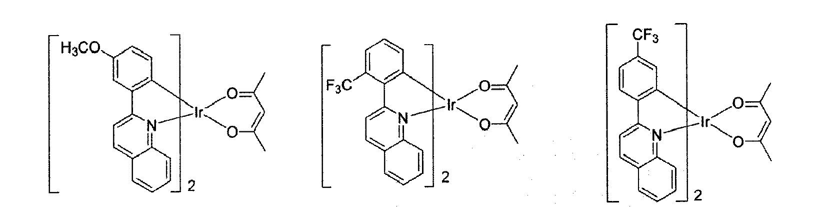

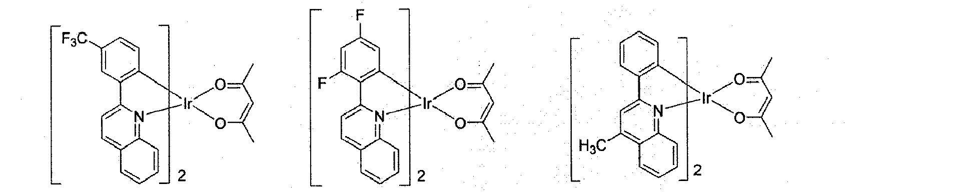

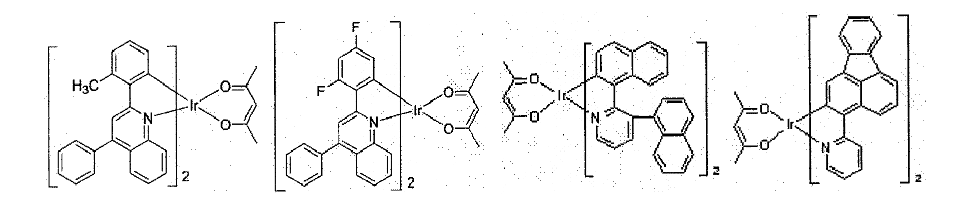

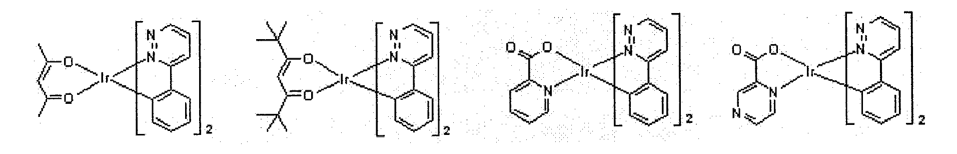

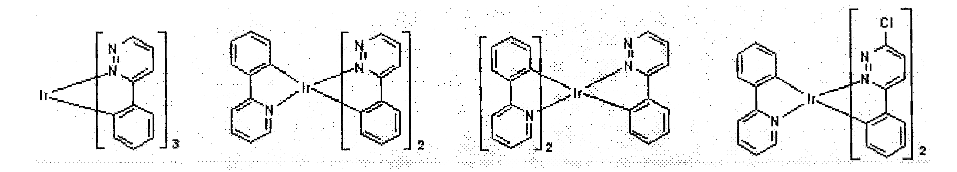

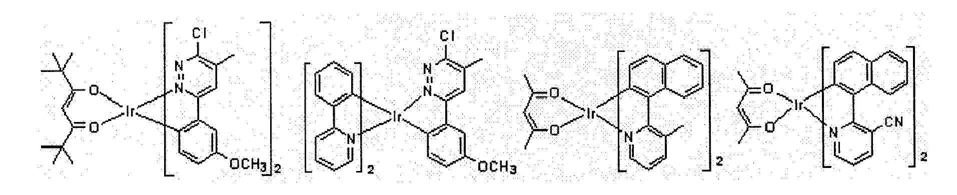

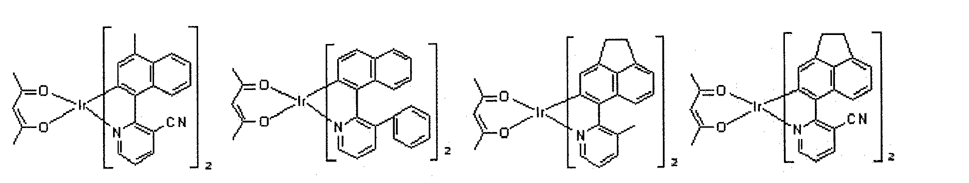

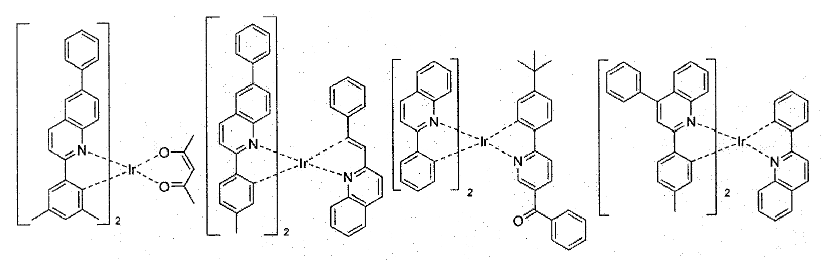

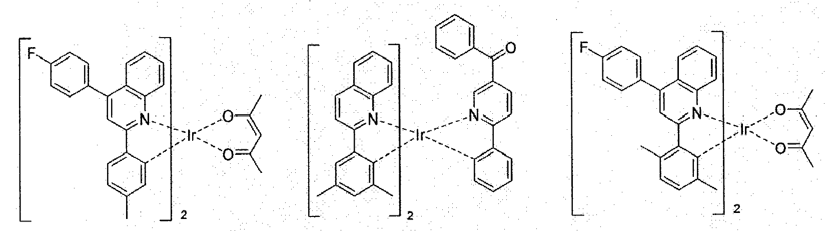

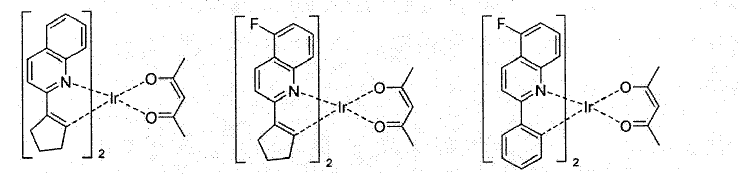

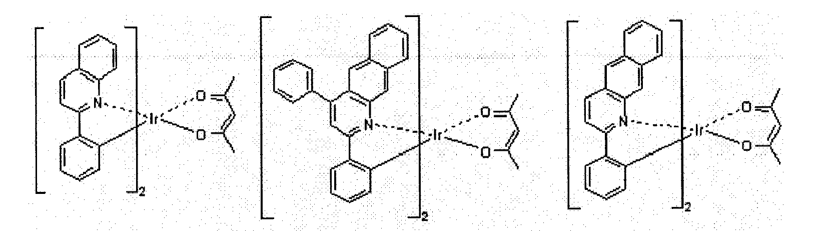

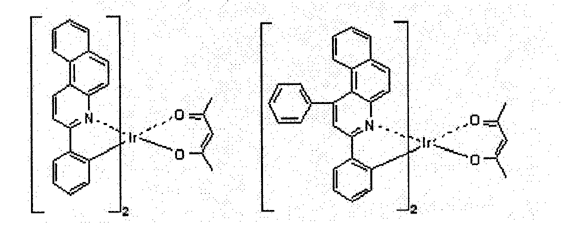

The electroluminescent device according to the present invention comprises the electroluminescent compound represented by Chemical Formula 1, and may further comprise one or more compound(s) selected from a group consisting of arylamine compounds and styrylarylamine compounds at the same time. For example, the arylamine compound or the styrylarylamine compound may be a compound represented by Chemical Formula 3, but not limited thereto: 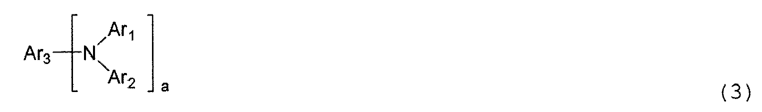 wherein
Ar₁ and Ar₂ independently represent hydrogen, deuterium, halogen, (C1-C60)alkyl, (C6-C60)aryl, (C4-C60)heteroaryl, (C6-C60)arylamino, (C1-C60)alkylamino, 5- or 6-membered heterocycloalkyl containing one or more heteroatom(s) selected from N, O and S or (C3-C60) cycloalkyl, and Ar₁ and Ar₂ may be linked via (C3-C60)alkylene or (C3-C60)alkenylene with or without a fused ring to form an alicyclic ring or a monocyclic or polycyclic aromatic ring;
Ar₃ represents (C6-C60)aryl, (C4-C60) heteroaryl or a substituent selected from the following structures, when a is 1: 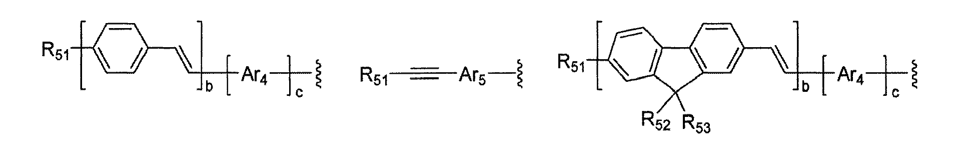
Ar₃ represents (C6-C60)arylene, (C4-C60)heteroarylene or a substituent selected from the following structures, when a is 2: 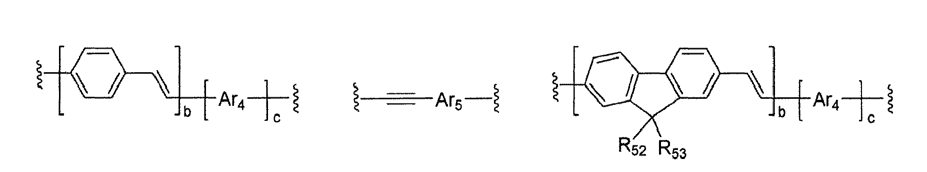
Ar₄ and Ar₅ independently represent (C6-C60)arylene or (C4-C60)heteroarylene;
R₅₁, R₅₂ and R₅₃ independently represent hydrogen, halogen, deuterium, (C1-C60)alkyl or (C6-C60)aryl;
b represents an integer from 1 to 4;
c represents an integer 0 or 1; and
the alkyl, aryl, heteroaryl, arylamino, alkylamino, cycloalkyl or heterocycloalkyl of Ar₁ and Ar₂, the aryl, heteroaryl, arylene or heteroarylene of Ar₃, the arylene or heteroarylene of Ar₄ and Ar₅, or the alkyl or aryl of R₅₁ through R₅₃ may be further substituted by one or more substituent (s) selected froma group consisting of deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C6-C60)arylthio, (C1-C60)alkylthio, (C1-C60)alkoxycarbonyl, (C1-C60)alkylcarbonyl, (C6-C60)arylcarbonyl, carboxyl, nitro and hydroxyl.

More specifically, the arylamine compound or the styrylarylamine compoundmay be exemplified by the following compounds, but not limited thereto:

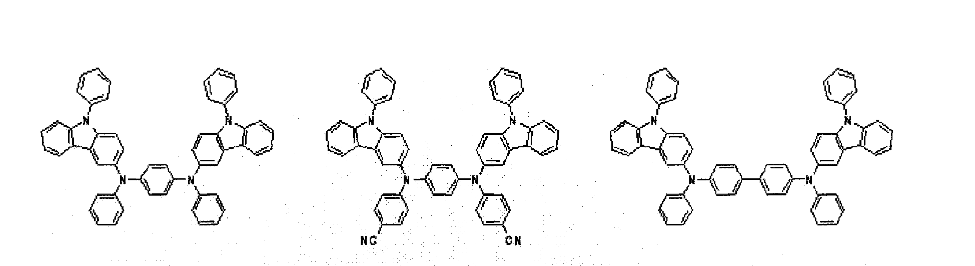

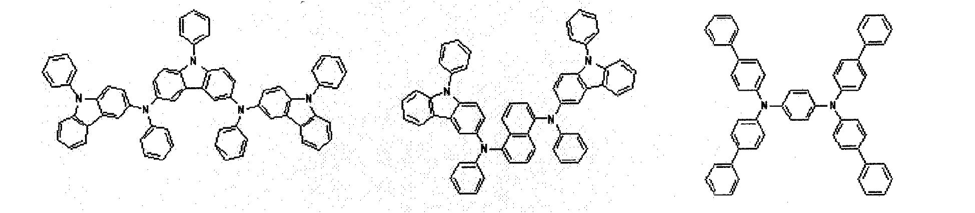

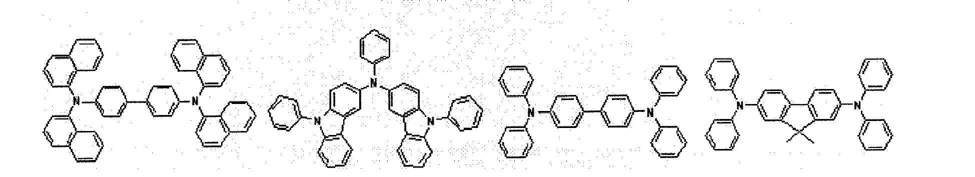

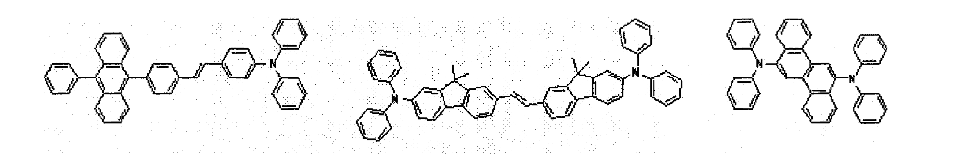

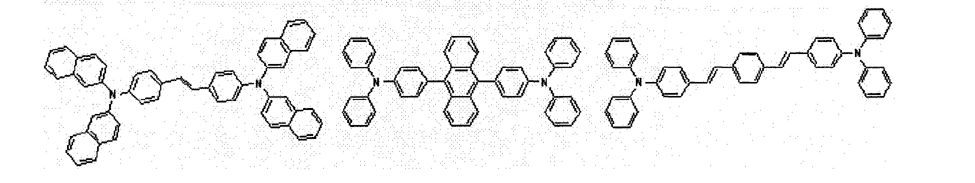

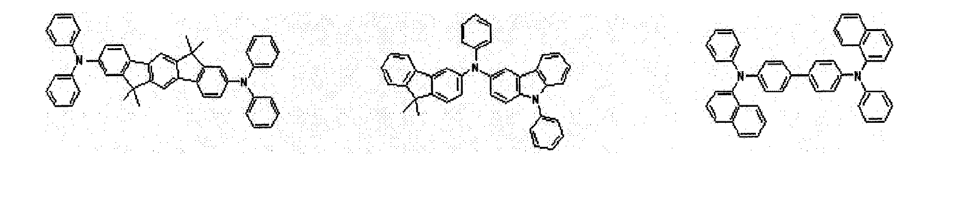

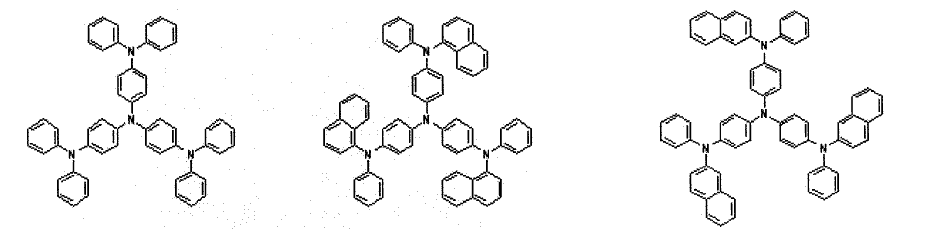

In the electroluminescent device according to the present invention, the organic layer may further comprise, in addition to the electroluminescent compound represented by Chemical Formula 1, one or more metal (s) selected from a group consisting of organic metals of Group 1, Group 2, 4th period and 5th period transition metals, lanthanide metals and d-transition elements. Also, the organic layer may comprise an electroluminescent layer and a charge generating layer at the same time.

An independent electroluminescence type electroluminescent device having a pixel structure in which the electroluminescent device according to the present invention comprising the electroluminescent compound represented by Chemical Formula 1 is used as a subpixel and one or more subpixel(s) comprising one or more metal compound(s) selected from a group consisting of Ir, Pt, Pd, Rh, Re, Os, Tl, Pb, Bi, In, Sn, Sb, Te, Au and Ag is patterned in parallel may be manufactured.

The organic layer may further comprise, in addition to the electroluminescent compound, one or more green- or blue-emitting organic compound(s) or organometallic compound(s) to form a white-emitting electroluminescent device. The green- or blue-emitting organic compound or organometallic compound may be exemplified by the compounds represented by Chemical Formulas 4 to 8, but not limited thereto: 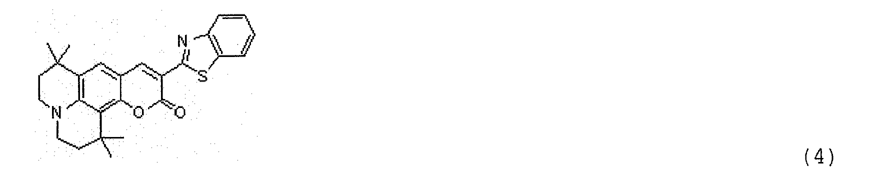 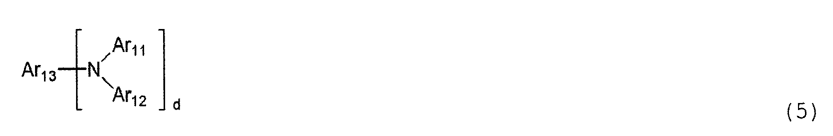 wherein
Ar₁₁ and Ar₁₂ independently represent (C1-C60)alkyl, (C6-C60)aryl, (C4-C60)heteroaryl, (C6-C60)arylamino, (C1-C60)alkylamino, 5- or 6-membered heterocycloalkyl containing one or more heteroatom(s) selected from N, O and S or (C3-C60)cycloalkyl, and Ar₁₁ and Ar₁₂ may be linked via (C3-C60) alkylene or (C3-C60)alkenylene with or without a fused ring to form an alicyclic ring or a monocyclic or polycyclic aromatic ring;
Ar₁₃ represents (C6-C60)aryl, (C4-C60)heteroaryl or a substituent selected from the following structures, when d is 1: 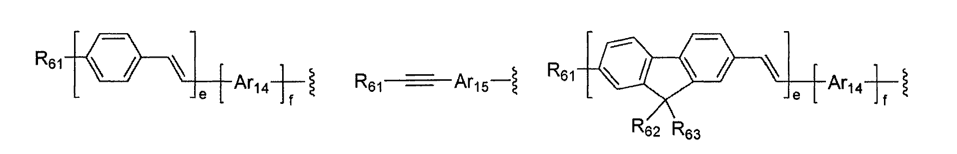
Ar₁₃ represents (C6-C60)arylene, (C4-C60)heteroarylene or a substituent selected from the following structures, when d is 2: 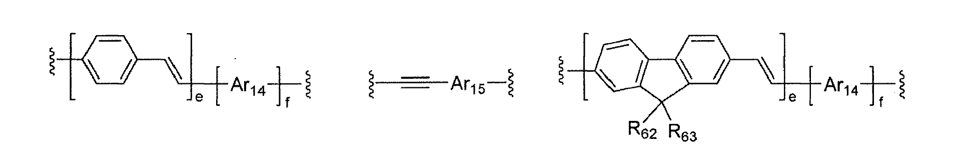
Ar₁₄ and Ar₁₅ independently represent (C6-C60)arylene or (C4-C60)heteroarylene;
R₆₁, R₆₂ and R₆₃ independently represent hydrogen, deuterium, (C1-C60)alkyl or (C6-C60)aryl;
e represents an integer from 1 to 4;
f represents an integer 0 or 1;
the alkyl, aryl, heteroaryl, arylamino, alkylamino, cycloalkyl or heterocycloalkyl of Ar₁₁ and Ar₁₂, the aryl, heteroaryl, arylene or heteroarylene of Ar₁₃, the arylene or heteroarylene of Ar₁₄ and Ar₁₅, or the alkyl or aryl of R₆₁ through R₆₃ may be further substituted by one or more substituent(s) selected from a group consisting of deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C6-C60)arylthio, (C1-C60)alkylthio, (C1-C60)alkoxycarbonyl, (C1-C60)alkylcarbonyl, (C6-C60)arylcarbonyl, carboxyl, nitro and hydroxyl; 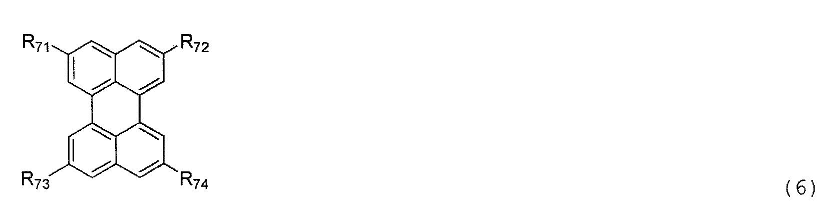
wherein
R₇₁ through R₇₄ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of R₇₁ through R₇₄ may be linked to an adjacent substituent via (C3-C60)alkylene or (C3-C60)alkenylene with or without a fused ring to form an alicyclic ring or a monocyclic or polycyclic aromatic ring;
the alkyl, alkenyl, alkynyl, cycloalkyl, heterocycloalkyl, aryl, heteroaryl, arylsilyl, alkylsilyl, alkylamino or arylamino of R₇₁ through R₇₄, or the alicyclic ring or the monocyclic or polycyclic aromatic ring formed as they are linked to an adjacent substituent via (C3-C60)alkylene or (C3-C60)alkenylene with or without a fused ring may be further substituted by one or more substituent (s) selected from deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl;

   **(Ar₂₁)_{g}-L₁₁-(Ar₂₂)ₕ** (7)

   **(Ar₂₃)ᵢ-L₁₂-(Ar₂₄)ⱼ** (8)

   wherein
   L₁₁ represents (C6-C60)arylene or (C4-C60)heteroarylene;
   L₁₂ represent anthracenylene;
   Ar₂₁ through Ar₂₄ independently represent hydrogen, deuterium, (C1-C60)alkyl, (C1-C60)alkoxy, halogen, (C4-C60)heteroaryl, (C5-C60)cycloalkyl or (C6-C60)aryl, or the cycloalkyl, aryl or heteroaryl of Ar₂₁ through Ar₂₄ may be further substituted by one or more substituent (s) selected from a group consisting of (C6-C60) aryl or (C4-C60)heteroaryl with or without one or more substituent(s) selected from a group consisting of (C1-C60)alkyl with or without halogen substituent, (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl, deuterium, (C1-C60)alkyl with or without halogen substituent, (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; and
   g, h, i and j independently represent an integer from 0 to 4.

The compound represented by Chemical Formula 7 or 8 may be exemplified by anthracene derivatives or benz[a]anthracene derivatives represented by Chemical Formulas 9 to 11: 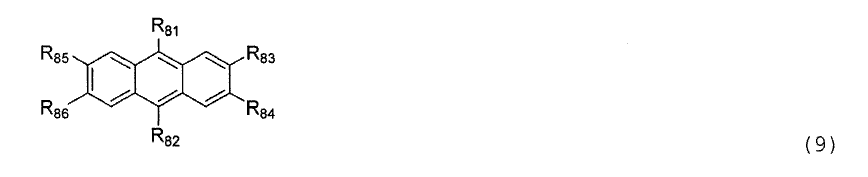 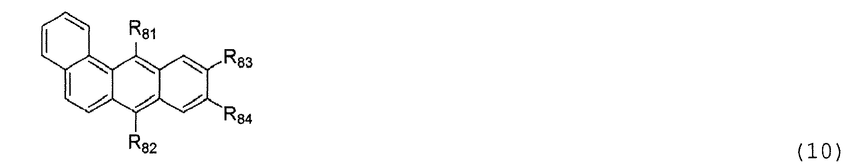 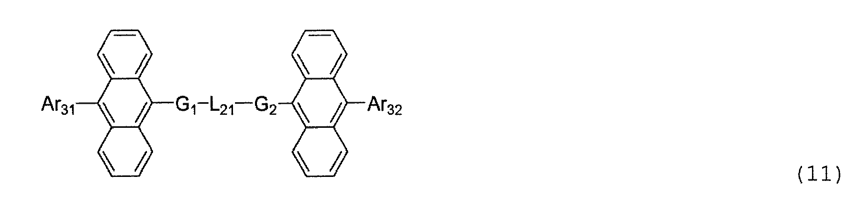 wherein
R₈₁ and R₈₂ independently represent (C6-C60)aryl, (C4-C60)heteroaryl, 5- or 6-membered heterocycloalkyl containing one or more heteroatom(s) selected from N, O and S or (C3-C60)cycloalkyl, and the aryl or heteroaryl of R₈₁ and R₈₂ may be further substituted by one or more substituent(s) selected from a group consisting of deuterium, (C1-C60)alkyl, halo(C1-C60)alkyl, (C1-C60)alkoxy, (C3-C60)cycloalkyl, (C6-C60)aryl, (C4-C60)heteroaryl, halogen, cyano, tri(C1-C60)alkylsilyl, di(C1-C60)alkyl(C6-C60)arylsilyl and tri(C6-C60)arylsilyl;
R₈₃ through R₈₆ independently represent hydrogen, deuterium, (C1-C60)alkyl, (C1-C60)alkoxy, halogen, (C4-C60)heteroaryl, (C5-C60)cycloalkyl or (C6-C60)aryl, and the heteroaryl, cycloalkyl or aryl of R₈₃ through R₈₆ may be further substituted by one or more substituent(s) selected from a group consisting of deuterium, (C1-C60)alkyl with or without halogen substituent, (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl;
G₁ and G₂ independently represent a chemical bond or (C6-C60)arylene with or without one or more substituent(s) selected from (C1-C60)alkyl, (C1-C60)alkoxy, (C6-C60)aryl, (C4-C60)heteroaryl and halogen;
Ar₃₁ and Ar₃₂ represent (C4-C60)heteroaryl or aryl selected from the following structures: 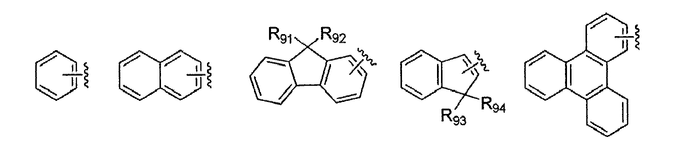 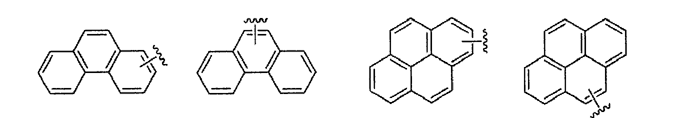 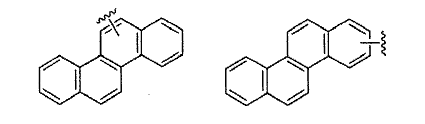
the aryl or heteroaryl of Ar₃₁ and Ar₃₂ may be substituted by one or more substituent(s) selected from deuterium, (C1-C60)alkyl, (C1-C60)alkoxy, (C6-C60)aryl and (C4-C60)heteroaryl;
L₂₁ represents (C6-C60)arylene, (C4-C60)heteroarylene or a compound having the following structure: 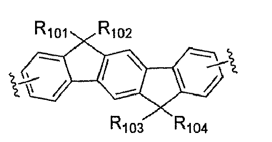
the arylene or heteroarylene of L₂₁ may be substituted by one or more substituent(s) selected from deuterium, (C1-C60)alkyl, (C1-C60)alkoxy, (C6-C60)aryl, (C4-C60)heteroaryl and halogen;
R₉₁, R₉₂, R₉₃ and R₉₄ independently represent hydrogen, deuterium, (C1-C60)alkyl or (C6-C60)aryl, or each of them may be linked to an adjacent substituent via (C3-C60)alkylene or (C3-C60)alkenylene with or without a fused ring to form an alicyclic ring or a monocyclic or polycyclic aromatic ring; and
R₁₀₁, R₁₀₂, R₁₀₃ and R₁₀₄ independently represent hydrogen, deuterium, (C1-C60)alkyl, (C1-C60)alkoxy, (C6-C60)aryl, (C4-C60)heteroaryl or halogen, or each of them may be linked to an adjacent substituent via (C3-C60)alkylene or (C3-C60)alkenylene with or without a fused ring to form an alicyclic ring or a monocyclic or polycyclic aromatic ring.

More specifically, the green- or blue-emitting organic compound or organometallic compound may be exemplified by the following compounds, but not limited thereto:

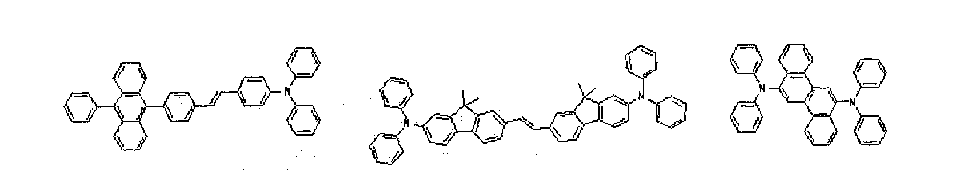

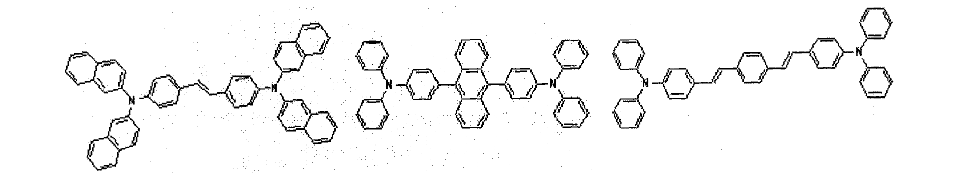

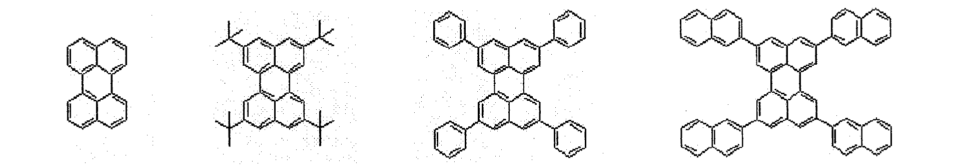

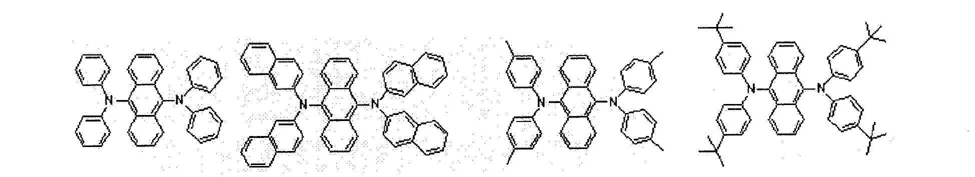

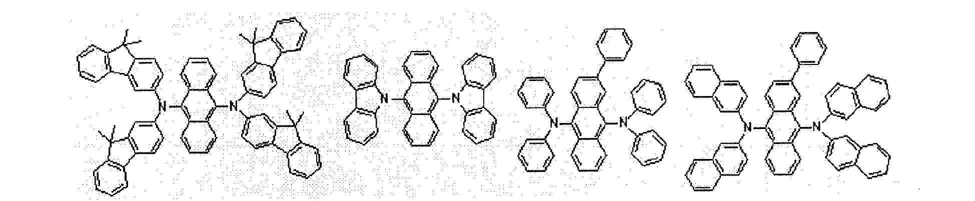

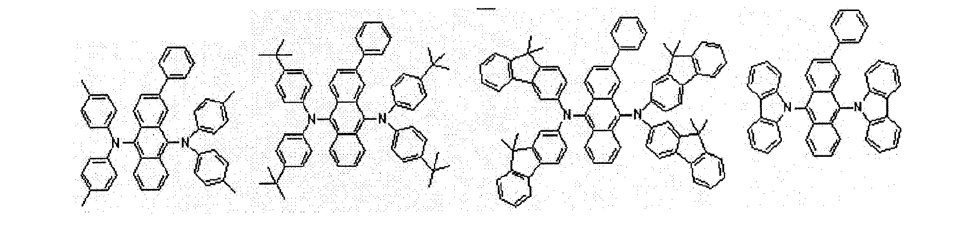

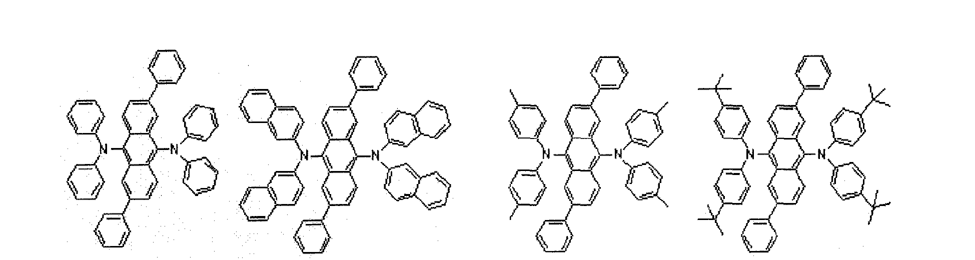

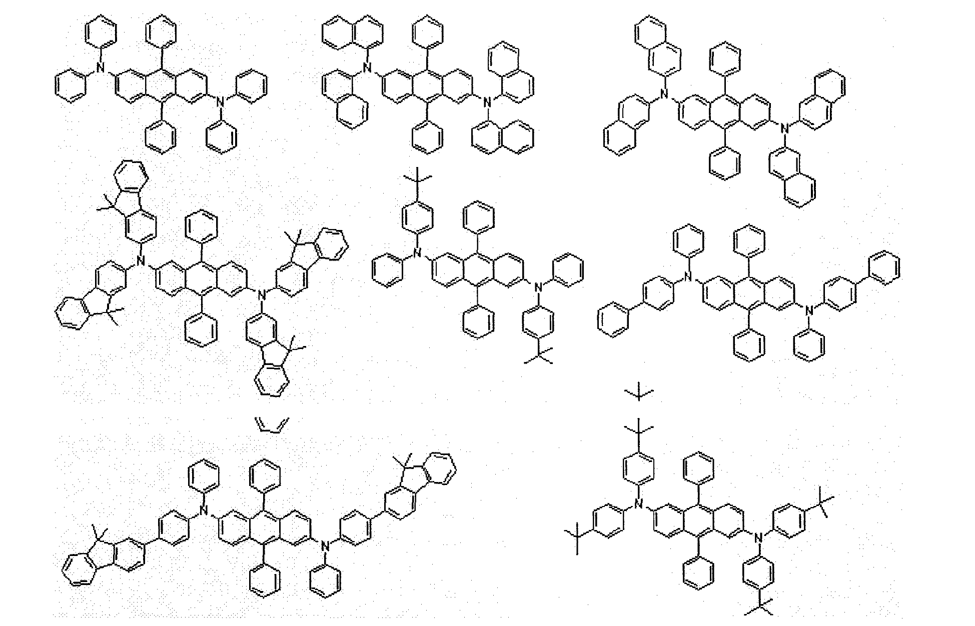

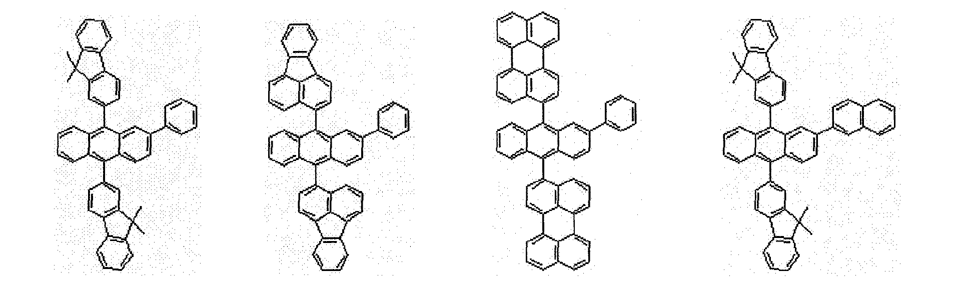

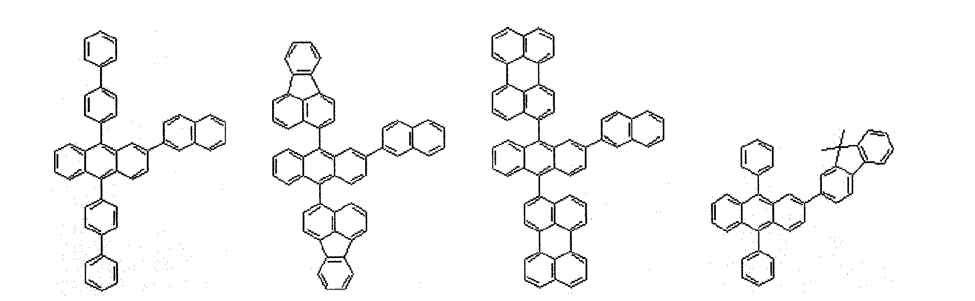

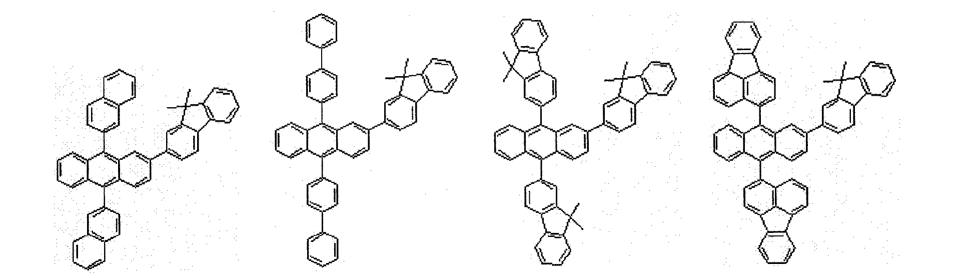

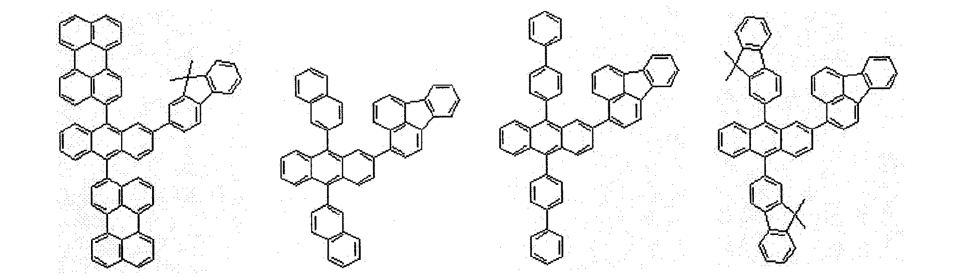

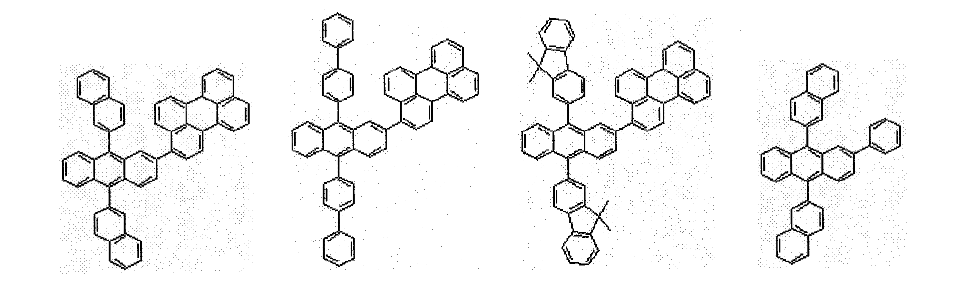

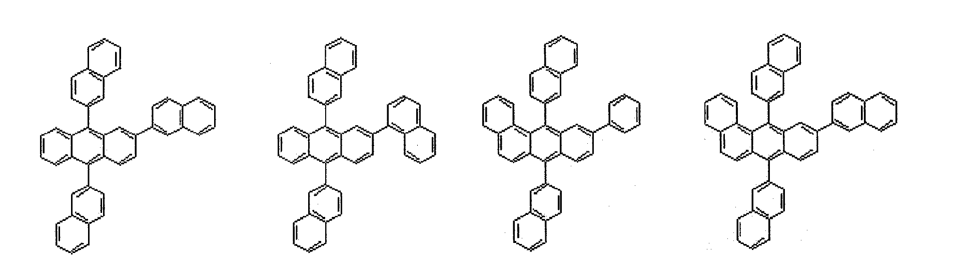

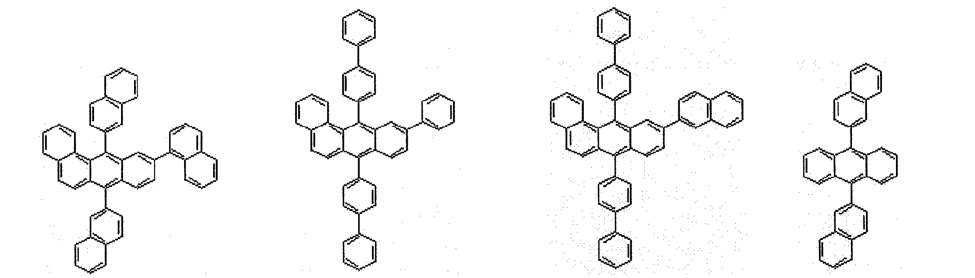

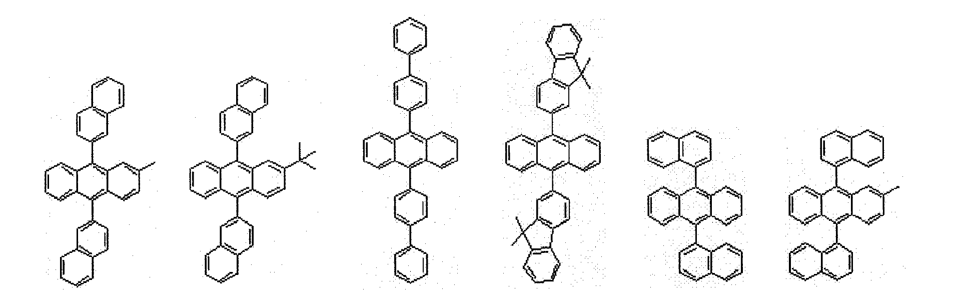

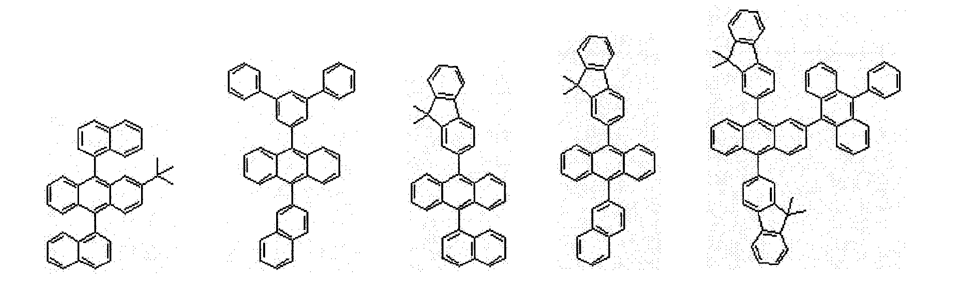

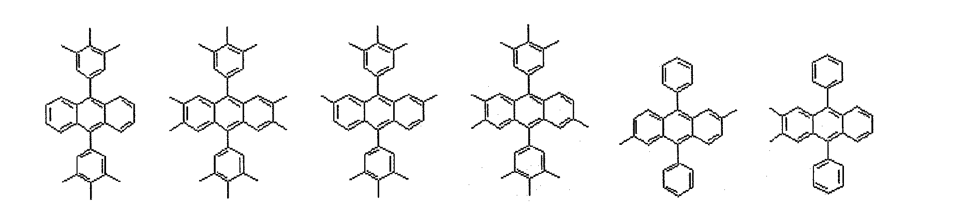

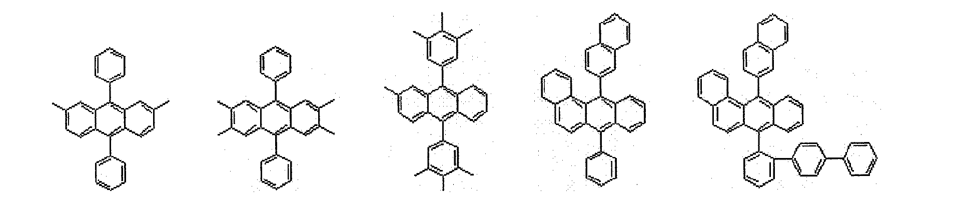

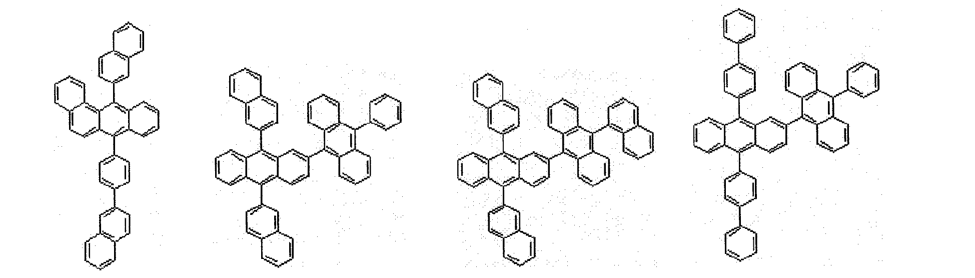

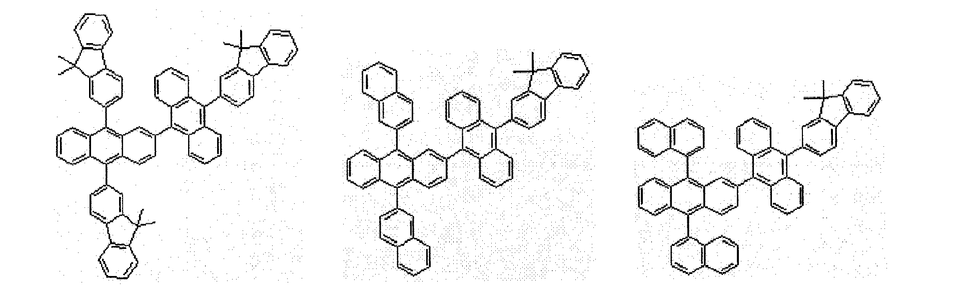

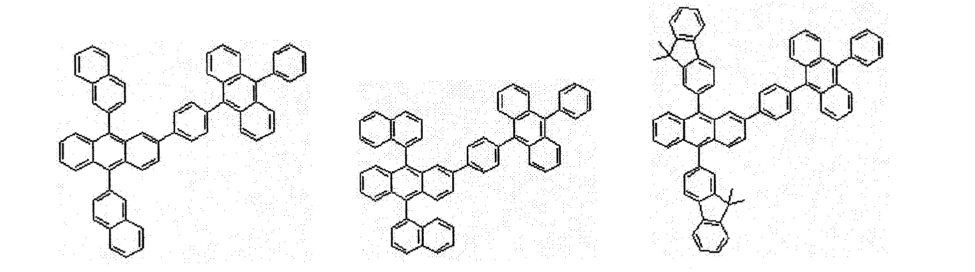

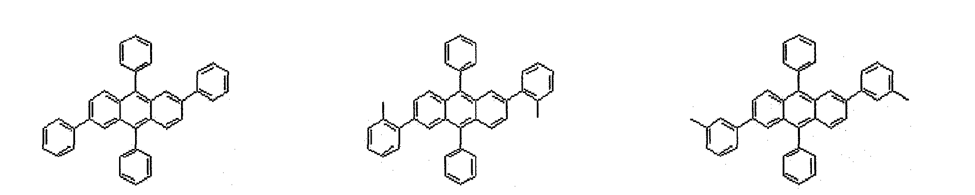

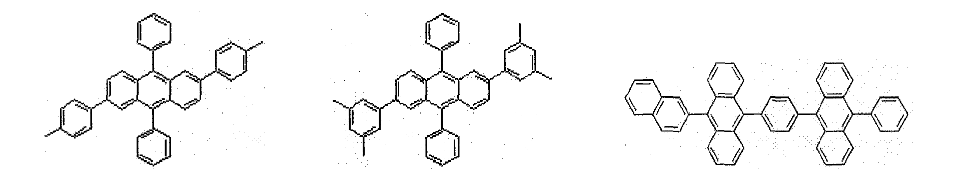

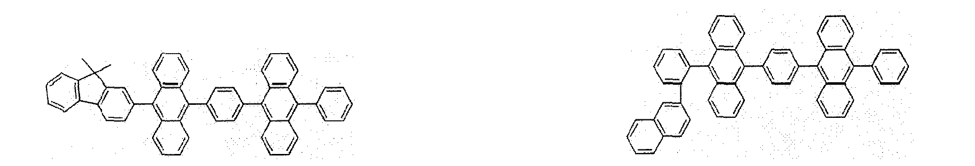

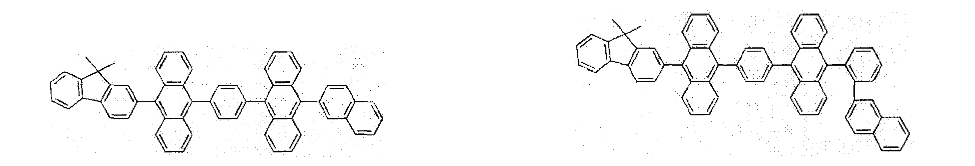

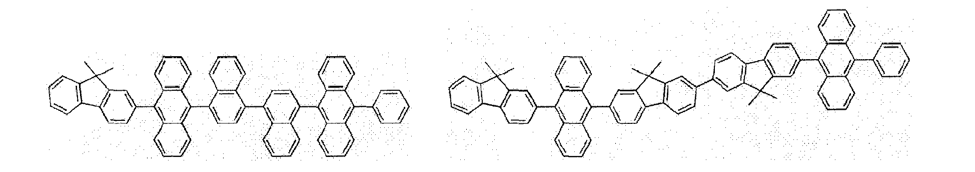

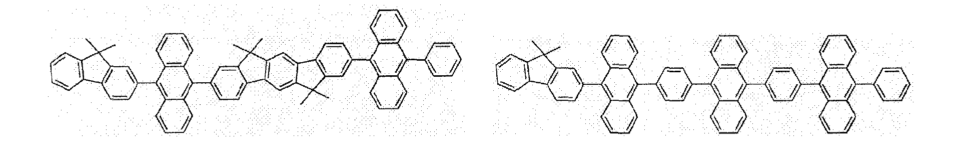

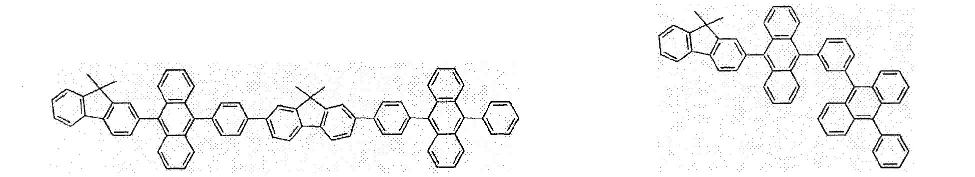

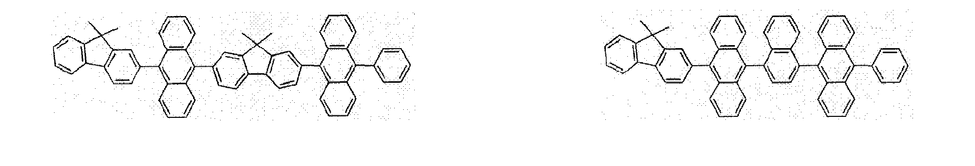

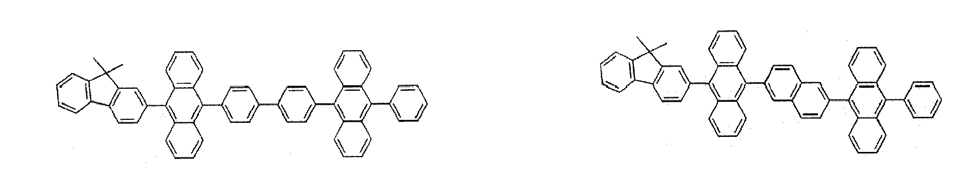

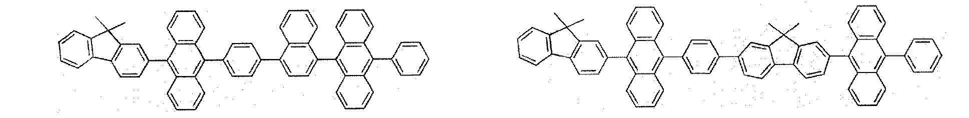

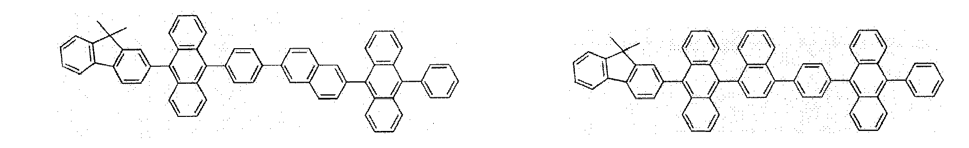

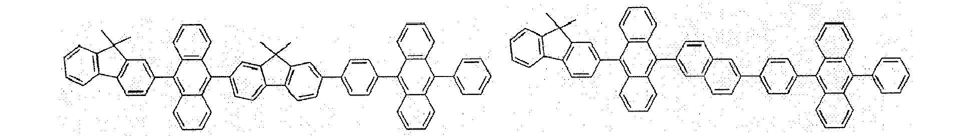

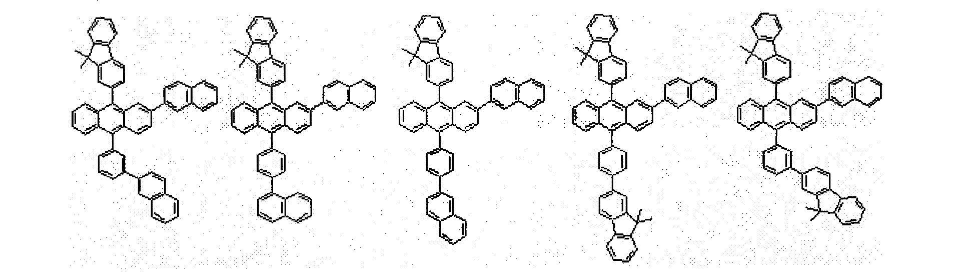

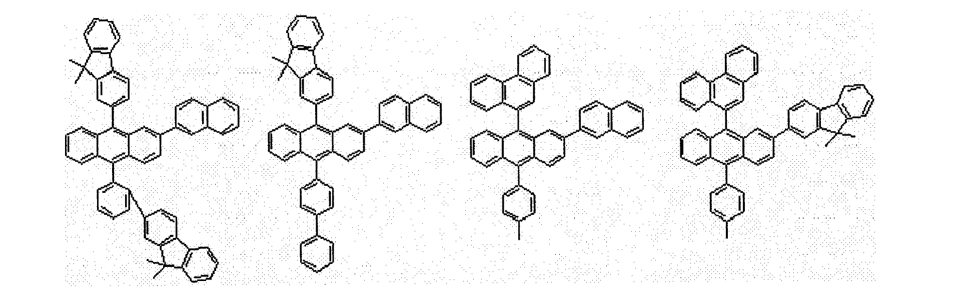

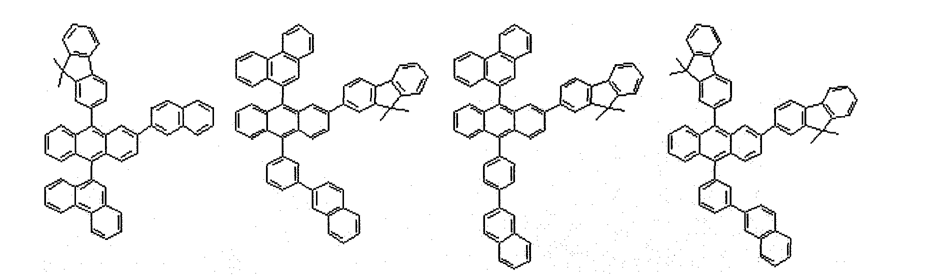

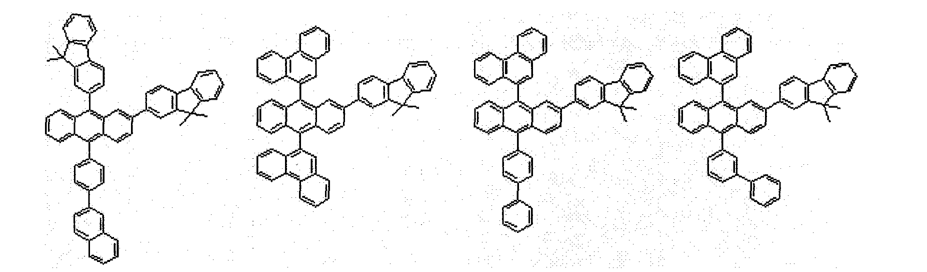

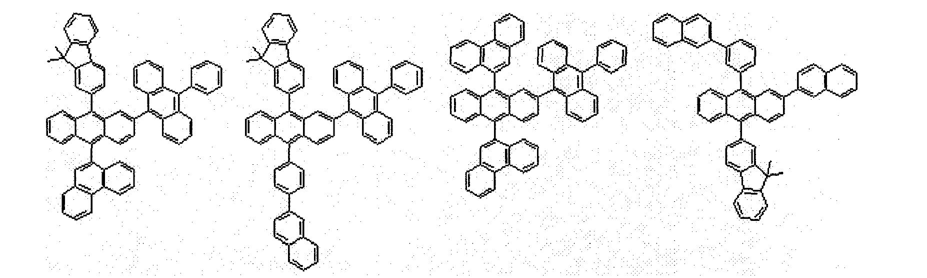

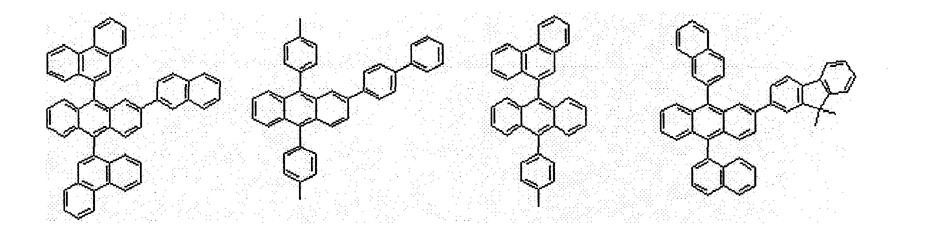

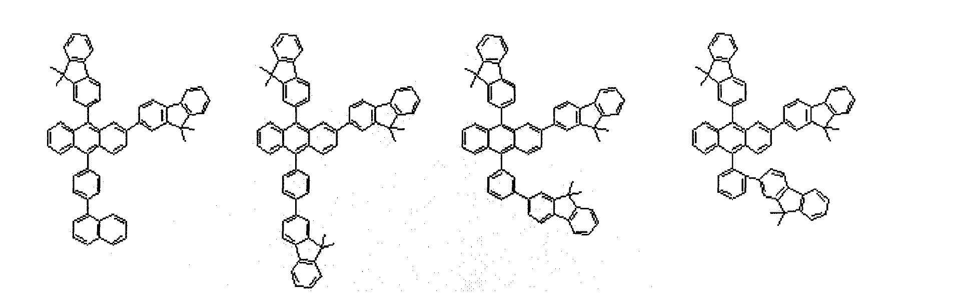

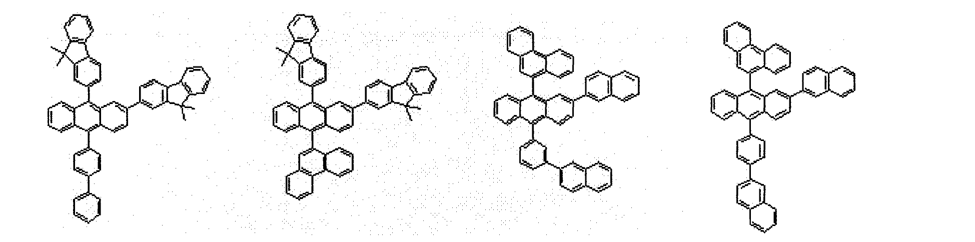

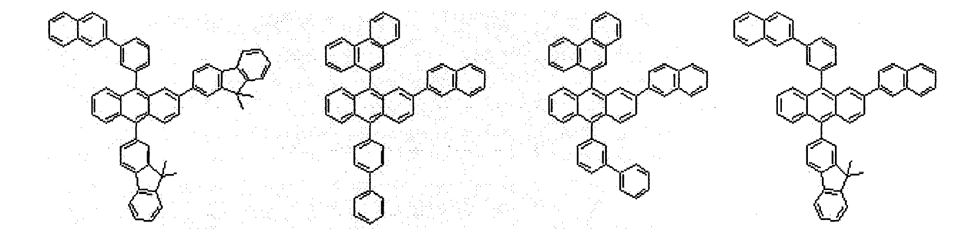

In the electroluminescent device according to the present invention, a layer (hereinafter referred to as "surface layer") selected from a chalcogenide layer, a metal halide layer and a metal oxide layermaybe placed on the inner surface of one or both electrode (s) among the pair of electrodes. More specifically, a chalcogenide (including oxide) layer of silicon or aluminum may be placed on the anode surface of the electroluminescent layer, and a metal halide layer or metal oxide layer may be placed on the cathode surface of the electroluminescent layer. A driving stability may be attained therefrom.

The chalcogenide may be, for example, SiOₓ (1 ≤ x ≤ 2), AlOₓ (1 ≤ x ≤ 1.5), SiON, SiAlON, etc. The metal halide may be, for example, LiF, MgF₂, CaF₂, a rare earth metal fluoride, etc. The metal oxide may be, for example, Cs₂O, Li₂O, MgO, SrO, BaO, CaO, etc.

Further, in the electroluminescent device according to the present invention, a mixed region of an electron transport compound and a reductive dopant or a mixed region of a hole transport compound and an oxidative dopant may be placed on the inner surface of one or both electrode(s) among the pair of electrodes. In that case, transport of electrons from the mixed region to the electroluminescent layer becomes easier, because the electron transport compound is reduced to an anion. Further, transport of holes from the mixed region to the electroluminescent layer becomes easier, because the hole transport compound is oxidized to a cation. Preferred examples of the oxidative dopant include various Lewis acids and acceptor compounds. Preferred examples of the reductive dopant include alkali metals, alkali metal compounds, alkaline earth metals, rare earth metals and mixtures thereof.

When used for a host material of an electroluminescent material in an OLED device, the electroluminescent compound according to the present invention provides better red luminous efficiency as compared to existing host materials and excellent life property of the material. Therefore, it can be used to manufacture OLEDs having very good operation life.

### EXAMPLES

Hereinafter, the novel electroluminescent compounds according to the present invention, preparation method thereof, and electroluminescence characteristics of the devices will be described for the understanding of the present invention. However, the following examples are for illustrative purposes only and not intended to limit the scope of the present invention.

### [Preparation Example 1] Preparation of Compound 191

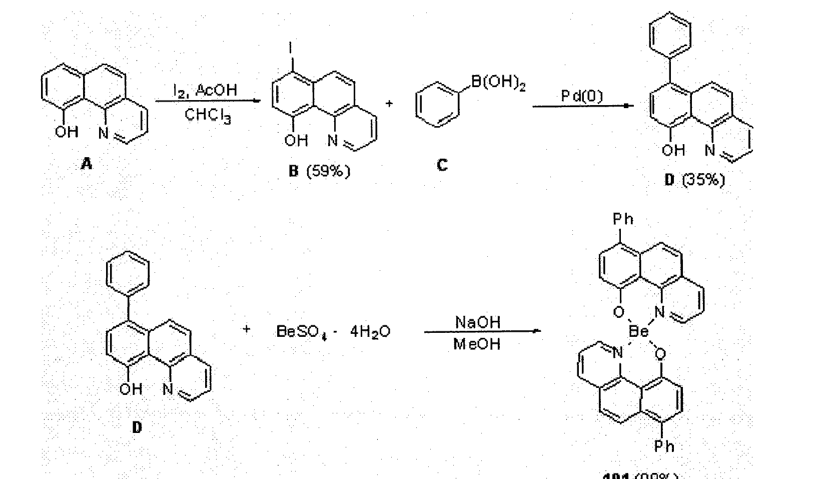

### Preparation of Compound B

Chloroform (1 L) and acetic acid (205 mL) were added to a 5 L round-bottom flask containing Compound **A** (10 g, 51.2 mmol). After slowly adding iodine (11.7 g, 46.1 mmol) dissolved in methanol (1 L), the mixture was stirred for 6 hours at 70 °C. The reaction mixture was cooled to room temperature and then neutralized. After washing with water and sodium thiosulfate, followed by extraction with methylene chloride, Compound **B** (9.7 g, 59%) was obtained by purification through silica column and drying.

### Preparation of Compound C

Compound **C** (4.2 g, 34.4 mmol) and tetrakis(triphenylphosphine)palladium (994 mg, 0.86 mmol) were added to a 500 mL flask containing Compound **B** (9.2 g, 28.7 mmol), and argon gas was filled therein. After adding toluene (100 mL), ethanol (50 mL) and 2 M potassium carbonate (50 mL), reflux was performed for 2 hours. After cooling to room temperature and washing with water, followed by extraction with ethyl acetate, Compound **D** (2.7 g, 35%) was obtained by purification through silica column and drying.

### Preparation of Compound 191

Sodium hydroxide (398 mg, 9.95 mmol) and methanol (180 mL) were added to a 500 mL flask containing Compound **D** (2.7 g, 9.95 mmol) and the mixture was stirred. After adding beryllium sulfate tetrahydrate (980 mg, 5.53 mmol), mixture was stirred for 12 hours. The produced solid was filtered, washed with water and ethanol and then dried. Compound **191** (2.7 g, 99%) was obtained.

### [Preparation Example 2] Preparation of Compound 191

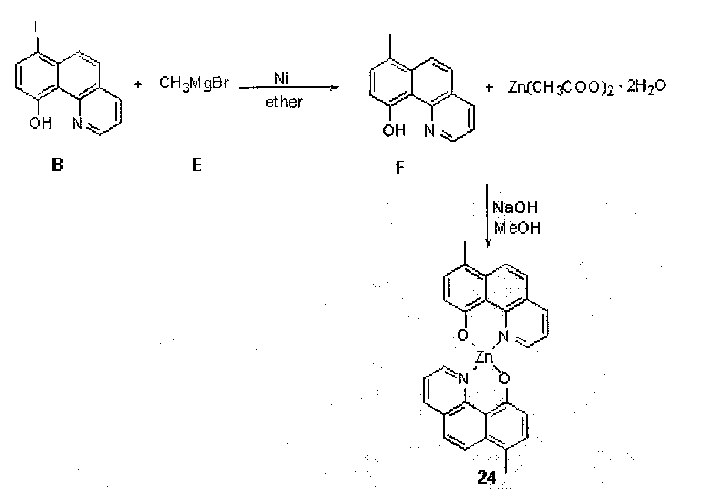

### Preparation of Compound F

Nickel (1.84 g, 2.8 mmol) was added to a 500 mL flask and argon gas was filled therein. After adding ether (50 mL) and Compound **E** (14.1 mL, 42.15 mmol), the mixture was stirred for 30 minutes at room temperature. After adding Compound **B** (5.0 g, 14.0 mmol) dissolved in ether, the mixture was stirred for 48 hours under reflux. After cooling to room temperature, the mixture was poured to saturated ammonium chloride. After washing with water, followed by extraction with ethyl acetate, Compound **F** (2.0 g, 69%) was obtained by purification through silica column and drying.

### Preparation of Compound 24

Sodium hydroxide (384 mg, 9.6 mmol) and methanol (180 mL) were added to a 500 mL flask containing Compound **F** (2.0 g, 9.6 mmol) and the mixture was stirred. Zinc acetate (1.2 g, 5.33 mmol) was added thereto and stirring was carried out for 12 hours. The produced solid was filtered, washed with water and ethanol and then dried. Compound **24** (2.0 g, 86%) was obtained.

Electroluminescent compounds (Compounds **1** to **356**) were prepared according to the method of Preparation Examples 1 and 2. Table 1 shows ¹H NMR and MS/FAB data of the prepared electroluminescent compounds.

**[Table 1]**

| compound | ¹H NMR (CDCl₃, 200 MHz) | MS/FAB |
|---|---|---|
| 1 | δ = 2.26 (6H, s), 6.93(2H, m), 7.58(2H, m), 7.66(2H, m), 7.81(2H, m), 8.06(2H, m), 8.38(2H, m), 8.82(2H, m) | 481.88 |
| 5 | δ = 7.41(2H, m), 7.51∼7.52(8H, m), 7.58(2H, m), 7.78∼7.81(6H, m), 8.06 (2H, m), 8.38 (2H, m), 8.82 (2H, m) | 606.02 |
| 9 | δ = 2.34(6H, s), 7.29∼7.33(8H, m), 7.58(2H, m), 7.78∼7.81(6H, m), 8.06(2H, m), 8.38(2H, m), 8.82(2H, m) | 634.07 |
| 14 | δ = 7.42(2H, m), 7.58(2H, m), 7.81(2H, m), 7.9(2H, m), 8.06(2H, m), 8.38(2H, m), 8.82(2H, m) | 503.85 |
| 25 | δ = 6.63(2H, m), 6.88 (2H, m), 7.58(2H, m), 7.81 (2H, m), 8.06(2H, m), 8.38(2H, m), 8.82(2H, m) | 489.81 |
| 32 | δ = 6.71(2H, m), 7.58∼7.59(8H, m), 7.73(2H, m), 7.8∼7.81(4H, m), 7.92 (2H, m), 8(4H, m), 8.06(2H, m), 8.38(2H, m), 8.82(2H, m) | 706.14 |
| 38 | δ = 6.5(4H, m), 7.14∼7.17(6H, m), 7.41(4H, m), 7.58(2H, m), 7.81 (2H, m), 8.06(2H, m), 8.38(2H, m), 8.82(2H, m) | 638.02 |
| 43 | δ = 2.34(36H, s), 6.65(2H, m), 6.91(6H, m), 6.98(1H, s), 7(1H, s), 7.45(2H, m), 7.58(2H, m), 7.81(2H, m), 8.06(2H,m), 8.38(2H, m), 8.82(2H, m) | 950.17 |
| 46 | δ = 1.72(12H, s), 6.71(2H, m), 7.28(2H, m), 7.38(2H, m), 7.53∼7.63(7H, m), 7.77∼7.81(5H, m), 7.87∼7.93(3H, m), 8.06(3H, m), 8.38(2H,m), 8.82(2H, m) | 838.34 |
| 52 | δ = 1.48(24H, m), 1.73∼1.75(16H, m), 2.72(4H, m), 6.81(2H, s), 7.58(2H, m), 7.81(2H, m), 8.06(2H, m), 8.38(2H, m), 8.82(2H, m) | 782.40 |
| 58 | δ = 3.83(12H, s), 6.21(2H, s), 7.58(2H, m), 7.81(2H, m), 8.06(2H, m), 8.38(2H, m), 8.82(2H, m) | 573.93 |
| 63 | δ = 7.55∼7.58(10H, m), 7.61(6H, s), 7.61(0H, m), 7.81(2H, m), 8.04∼8.08(10H, m), 8.38∼8.42(6H, m), 8.55(4H, m), 8.82(2H, m) | 958.45 |
| 71 | δ = 5.45(2H, m), 6.22(2H, m), 6.65(2H, m), 6.84 (2H, m), 7.4(2H, m), 7.57(2H, m), 7.72(2H, m), 7.81(2H, m), 8.06(2H, m), 8.24(2H, m) | 505.90 |
| 78 | δ = 6.65(2H, m), 7.35∼7.4(4H, m), 7.55(4H, m), 7.64(2H, m), 7.72(2H, m), 7. 81 (2H, m), 8.06∼8.1(8H, m), 8.46(2H, m), 8.55(2H, m) | 706.14 |
| 84 | δ = 6.65(2H, m), 6.95∼7.01(6H, m), 7.11(2H, m), 7.28(4H, m), 7.4(2H, m), 7.7∼7.72(4H, m), 8.02(2H, m), 8.33(2H, m) | 638.02 |
| 96 | δ = 6.65(2H, m), 7.32∼7.4(6H, m), 7.72(2H, m), 7.81∼7.85(4H, m), 8.06(2H, m), 8.19(2H, m), 8.59(2H, m), 8.91(2H, m) | 608.00 |
| 102 | δ = 6.65(2H, m), 7.4(2H, m), 7.72(2H, m), 8.1(2H, m), 8.26(2H, m), 8.58(2H, m), 8.97(2H, m) | 589.82 |
| 109 | δ = 6.65(2H, m), 7.37∼7.46(26H, m), 7.55(6H, m), 7.72∼7.74(4H, m), 7.81(2H, m), 8.06(2H, m), 8.92(2H, m) | 970.61 |
| 117 | δ = 2.34(12H, s), 6.65(2H, m), 7.29∼7.33(12H, m), 7.4(2H, m), 7.72(2H, m), 7.81(2H, m), 7.84(2H, s), 8.06(2H, m), 8.18(4H, m) | 814.32 |
| 128 | δ = 6.65(2H, m), 7.23(4H, m), 7.24(2H, s), 7.29(8H, m), 7.39∼7.4(10H, m), 7.72(2H, m), 7.81(2H, m), 8.06(2H, m) | 886.47 |
| 136 | δ = 6.74(2H, m), 7(2H, m), 7.26 (2H, m), 7.32∼7.36(4H, m), 7.51(2H, m), 7.81∼7.85(4H, m), 8.06(2H, m), 8.19(2H, m), 8.31(2H, m), 8.5(2H, m), 8.59(2H, m), 8.91(2H, m) | 762.16 |
| 149 | δ = 5.35(4H, s), 6.5(4H, m), 7.52(2H, m), 7.87(2H, m), 8.47(2H, m), 8.67(2H, m) | 517.83 |
| 158 | δ = 7.55(8H, m), 7.61(4H, m), 7.68(2H, m), 7.78∼7.81(6H, m), 8.04∼8.08(10H, m), 8.42(4H, m), 8.55(4H, m), 8.88(2H, m) | 958.45 |
| 166 | δ = 2.5(12H, s), 7.42 (2H, m), 7.52 (2H, m), 7.94(2H, m), 8.19(2H, m), 8.75(2H, m), 9.14(2H, m) | 621.97 |
| 178 | δ = 1.35(18H, s), 1.48(18H, s), 6.52(2H,m), 6.85(2H, m), 7.37(2H, m), 7.8(2H, m), 8.16(2H, m), 8.69(2H, m) | 742.55 |
| 186 | δ = 6.94(2H, m), 7.81∼7.84(4H, m), 8.01∼8.06(4H, m), 9.11(2H, m) | 569.82 |
| 196 | δ = 6.77(2H, m), 7.58(2H, m), 7.65(2H, m), 7.81(2H, m), 8.06(2H, m), 8.38(2H, m), 8.82(2H, m) | 447.45 |
| 203 | δ = 1.72(12H, s), 6.71 (2H, m), 7.28(2H, m), 7.38(2H, m), 7.53∼7.63(7H, m), 7.77∼7.81(5H, m), 7.87∼7.93(3H, m), 8.06 (3H, m), 8.38 (2H, m), 8.82(2H, m) | 781.94 |
| 217 | δ = 6.44(2H, m), 6.63∼6.65(10H, m), 6.81(4H, m), 7.2(8H, m), 7.4(2H, m), 7.72(2H, m), 7.8(2H, m), 8.05(2H, m), 8.65(2H, m) | 731.84 |
| 222 | δ = 6.35(2H, m), 7.02(2H, m), 7.58(2H, m), 7.81(2H, m), 8.06(2H, m), 8.38(2H, m), 8.82(2H, m) | 489.81 |
| 234 | δ = 6.89(2H, m), 7.56∼7.58(4H, m), 7.81(2H, m), 8.06(2H, m), 8.38(2H, m), 8.82(2H, m) | 503.85 |
| 242 | δ = 2.34(36H, s), 6.91∼6.92(10H, m), 7.58∼7.59(4H, m), 7.81(2H, m), 8.06(2H, m), 8.38(2H, m), 8.82(2H, m) | 950.17 |
| 255 | δ = 5.18(2H, m), 5.61(2H, m), 6.63∼6.65(4H, m), 7.4(2H, m), 7.72(2H, m), 7.81(2H, m), 8.06(2H, m), 8.38(2H, m), 8.86(2H, m) | 505.90 |
| 261 | δ = 6.65(2H, m), 7.23∼7.29(6H, m), 7.39∼7.4(6H, m), 7.72(2H, m), 7.81(2H, m), 8.06(2H, m), 8.14(2H, m), 8.76(2H, m) | 670.15 |
| 271 | δ = 7.55∼7.61(4H, m), 7.78∼7.81(3H, m), 8.04∼8.08(3H, m), 8.38∼8.42(2H, m), 8.55(1H, m), 8.82(1H, m) | 347.34 |
| 284 | δ = 6.89(1H, m), 7.56∼7.58(2H, m), 7.81(1H, m), 8.06(1H, m), 8.38(1H, m), 8.82(1H, m) | 246.20 |
| 292 | δ = 7.58(1H, m), 7.81∼7.84 (2H, m), 8.01∼8.06(2H, m), 8.38(1H, m), 8.82(1H, m) | 250.19 |
| 305 | δ = 1.72(6H, s), 7.28(1H, m), 7.38(1H, m), 7.55∼7.63(3H, m), 7.77∼7.81(4H, m), 7.87∼7.93(2H, m), 8.06(1H, m), 8.38(1H, m), 8.82(1H, m) | 413.45 |
| 314 | δ = 6.65(1H, m), 7.4(1H, m), 7.72(1H, m), 7.81(1H, m), 7.91(1H, m), 8.06(1H, m), 8.53(1H, m) | 239.18 |
| 326 | δ = 1.35(9H, s), 6.65(1H, m), 7.4(1H, m), 7.72(1H, m), 7.8(1H, m), 7.98∼8.03(2H, m), 8.68(1H, m) | 277.30 |
| 332 | δ = 6.65(1H, m), 7.4(1H, m), 7.72(1H, m), 7.81(1H, m), 8.06(1H, m), 9.11(1H, m), 9.45(1H, m) | 246.20 |
| 346 | δ = 6.65(1H, m), 7.37∼7.46(13H, m), 7.55(3H, m), 7.72(1H, m), 7.81(1H, m), 8.06(1H, m), 8.48(1H, m), 9.1(1H, m) | 479.58 |
| 354 | δ = 6.65(1H, m), 7.11(1H, m), 7.4(1H, m), 7.7∼7.72(2H, m), 8.02(1H, m), 8.33(1H, m), 11.53(1H, s) | 237.19 |

### [Examples 1-9] Manufacture of OLED device using the electroluminescent compound according to the present invention

An OLED device was manufactured using the electroluminescent compound according to the present invention as a host material. FIG. 1 shows the cross-sectional view of the OLED device.

First, a transparent electrode ITO film (15 Ω/□, Samsung Corning) 2 prepared from a glass substrate for an OLED 1 was subjected to ultrasonic washing sequentially using trichloroethylene, acetone, ethanol and distilled water, and stored in isopropanol for later use.

Next, the ITO substrate was mounted on a substrate holder of a vacuum deposition apparatus. After filling 4,4',4"-tris(N,N-(2-naphthyl)-phenylamino)triphenylamine (2-TNATA) in a cell of the vacuum deposition apparatus, the pressure inside the chamber was reduced to 10⁻⁶ torr. Then, 2-TNATA was evaporated by applying electrical current to the cell. A hole injection layer 3 having a thickness of 60 nm was formed on the ITO substrate.

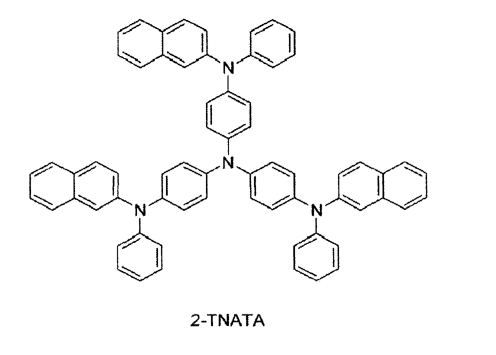

Subsequently, after filling *N,N*'-bis(α-naphthyl)-*N,N*'-diphenyl-4,4'-diamine (NPB) in another cell of the vacuum deposition apparatus, NPB was evaporated by applying electrical current. A hole transport layer 4 having a thickness of 20 nm was formed on the hole injection layer.

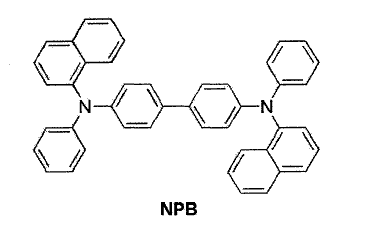

An electroluminescent layer 5 was formed on the hole transport layer as follows. The compound according to the present invention (e.g., Compound 1) vacuum sublimed at 10⁻⁶ torr was filled in a cell of a vacuum deposition apparatus as a host material, and an electroluminescent dopant (e.g., (piq)₂Ir(acac)) was filled in another cell. The two materials were evaporated at different speed, so that an electroluminescent layer 5 having a thickness of 30 nm was formed on the hole transport layer at 4 to 10 mol%.

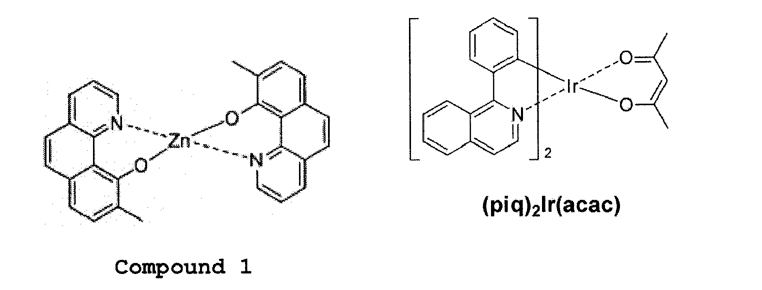

Thereafter, tris(8-hydroxyquinoline)-aluminum(III) (Alq) was deposited with a thickness of 20 nm as an electron transport layer 6. Then, lithium quinolate (Liq) was deposited with a thickness of 1 to 2 nm as an electron injection layer 7. Then, an Al cathode 8 having a thickness of 150 nm was formed using another vacuum deposition apparatus to manufacture an OLED.

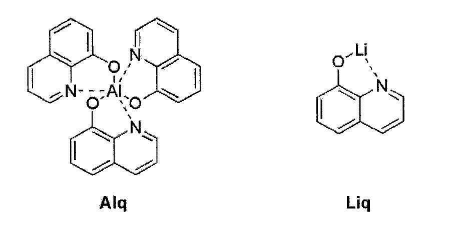

Each OLED electroluminescent material was purified by vacuum sublimation at 10⁻⁶ torr.

### [Comparative Example 1] Manufacture of OLED device using existing electroluminescent material

An OLED device was manufactured as in Example 1, except that bis(2-methyl-8-quinolinato)(p-phenylphenolato)aluminum(III) (BAlq) was used as an electroluminescent host material instead of the electroluminescent compound according to the present invention.

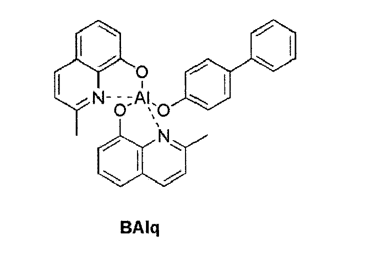

### [Test Example 1] Confirmation of characteristics of manufactured OLED device

Driving voltage and power efficiency of the OLED devices manufactured in Examples 1-9 and Comparative Example 1 were measured at 1,000 cd/m². The result is given in Table 2.

As seen in Table 2, the electroluminescent compounds according to the present invention exhibited superior electroluminescence characteristics as compared to the existing material.

**[Table 2]**

| | Host | Dopant | Driving voltage (V) @ 1,000 cd/m² | Power efficiency (lm/W) @ 1,000 cd/m² | Color |
|---|---|---|---|---|---|
| Example 1 | 1 | (piq)₂Ir(acac) | 4.6 | 4.9 | red |
| Example 2 | 5 | (piq)₂Ir(acac) | 4.5 | 5.3 | red |
| Example 3 | 31 | (piq)₂Ir(acac) | 4.4 | 6.5 | red |
| Example 4 | 48 | (piq)₂Ir(acac) | 4.8 | 4.7 | red |
| Example 5 | 77 | (piq)₂Ir(acac) | 5.0 | 4.5 | red |
| Example 6 | 189 | (piq)₂Ir(acac) | 5.1 | 5.8 | red |
| Example 7 | 193 | (piq)₂Ir(acac) | 4.7 | 6.2 | red |
| Example 8 | 201 | (piq)₂Ir(acac) | 4.5 | 6.3 | red |
| Example 9 | 203 | (piq)₂Ir(acac) | 4.9 | 4.8 | red |
| Comp. Ex. 1 | BAlq | (piq)₂Ir(acac) | 7.5 | 2.6 | red |

As seen in Table 2, the complexes according to the present invention exhibited superior electroluminescence characteristics as compared to the existing material. Driving voltage of the device was decreased by 2 V or more and power efficiency was improved by 2.6 times or more as compared to the existing material (Comparative Example 1). Especially, the device of Example 3 was operated at a voltage 3.1 V lower than that of Comparative Example 1, and the device of Example 8 exhibited a driving voltage of 4.5 V and a power efficiency of 6.3 lm/W at 1000 cd/m².

Accordingly, the device using the electroluminescent compound according to the present invention as host material has excellent electroluminescence characteristics and power consumption can be improved by increasing power efficiency by 1.9-3.9 lm/W through decreased driving voltage.

While the exemplary embodiments have been shown and described, it will be understood by those skilled in the art that various changes in form and details may be made thereto without departing from the spirit and scope of this disclosure as defined by the appended claims.

In addition, many modifications can be made to adapt a particular situation or material to the teachings of this disclosure without departing from the essential scope thereof. Therefore, it is intended that this disclosure not be limited to the particular exemplary embodiments disclosed as the best mode contemplated for carrying out this disclosure, but that this disclosure will include all embodiments falling within the scope of the appended claims.

## Claims

1. An electroluminescent compound represented by Chemical Formula 1: 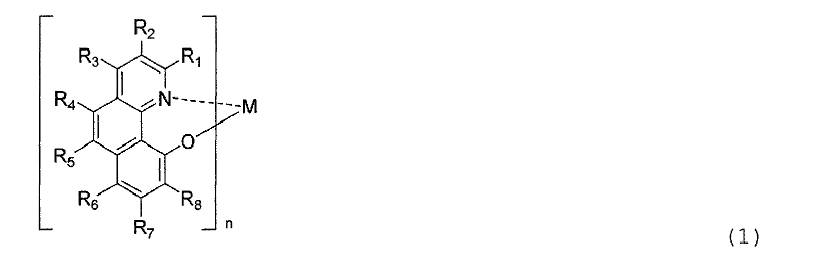 wherein
M represents a divalent or trivalent metal;
n represents the cationic valence of M;
R₁ through R₈ independently represent hydrogen, (C1-C60)alkyl, halogen, cyano, (C3-C60)cycloalkyl, 5- or 6-membered heterocycloalkyl containing one or more heteroatom(s) selected from N, O and S, (C7-C60)bicycloalkyl, adamantyl, (C2-C60)alkenyl, (C2-C60)alkynyl, (C6-C60)aryl, (C1-C60)alkoxy, (C1-C60)alkylthio, (C6-C60)aryloxy, (C3-C60)heteroaryl containing one or more heteroatom (s) selected from N, O and S, (C6-C60)arylthio, (C1-C60)alkoxycarbonyl, (C1-C60)alkylcarbonyl, (C6-C60)arylcarbonyl, mono- or di(C1-C30)alkylamino, mono- or di(C6-C30)arylamino, tri(C1-C30)alkylsilyl, di(C1-C30)alkyl(C6-C30)arylsilyl, tri(C6-C30)arylsilyl, di(C6-C60)arylboranyl, di(C1-C60)alkylboranyl, carboxyl, nitro or hydroxyl; and
the alkyl, cycloalkyl, heterocycloalkyl, bicycloalkyl, adamantyl, alkenyl, alkynyl, aryl, alkoxy, alkylthio, aryloxy, heteroaryl, arylthio, alkoxycarbonyl, alkylcarbonyl, arylcarbonyl, alkylamino, arylamino, trialkylsilyl, dialkylarylsilyl, triarylsilyl, diarylboranyl or dialkylboranyl of R₁ through R₈ may be further substituted by one or more substituent(s) selected from a group consisting of (C1-C60)alkyl, halogen, cyano, (C3-C60)cycloalkyl, 5- or 6-membered heterocycloalkyl containing one or more heteroatom(s) selected from N, O and S, (C7-C60)bicycloalkyl, adamantyl, (C2-C60)alkenyl, (C2-C60)alkynyl, (C6-C60)aryl, (C1-C60)alkoxy, (C1-C60)alkylthio, (C6-C60)aryloxy, (C3-C60)heteroaryl containing one or more heteroatom (s) selected from N, O and S, (C6-C60)arylthio, (C1-C60)alkoxycarbonyl, (C1-C60)alkylcarbonyl, (C6-C60)arylcarbonyl, mono- or di(C1-C30)alkylamino, mono- or di(C6-C30)arylamino, tri(C1-C30)alkylsilyl, di(C1-C30)alkyl(C6-C30)arylsilyl, tri(C6-C30)arylsilyl, carboxyl, nitro and hydroxyl,
except for the case where all of R₁ through R₈ are hydrogens.

2. The electroluminescent compound according to claim 1, wherein M represents a divalent metal selected from a group consisting of Be, Zn, Mg, Ca, Cu and Ni.

3. The electroluminescent compound according to claim 1, wherein M represents a trivalent metal selected from a group consisting of Al, In, Co, Ga and B.

4. The electroluminescent device which comprises a first electrode; a second electrode; and one or more organic layer(s) interposed between the first electrode and the second electrode, wherein the organic layer comprises an electroluminescent compound represented by Chemical Formula 1: 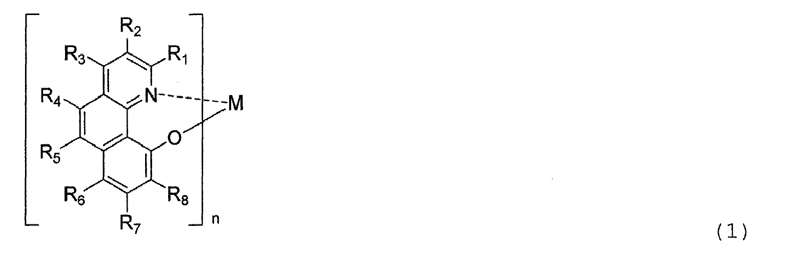 wherein
M represents a divalent or trivalent metal;
n represents the cationic valence of M;
R₁ through R₈ independently represent hydrogen, (C1-C60)alkyl, halogen, cyano, (C3-C60)cycloalkyl, 5- or 6-membered heterocycloalkyl containing one or more heteroatom(s) selected from N, O and S, (C7-C60)bicycloalkyl, adamantyl, (C2-C60)alkenyl, (C2-C60)alkynyl, (C6-C60)aryl, (C1-C60)alkoxy, (C1-C60)alkylthio, (C6-C60)aryloxy, (C3-C60) heteroaryl containing one or more heteroatom (s) selected from N, O and S, (C6-C60)arylthio, (C1-C60)alkoxycarbonyl, (C1-C60)alkylcarbonyl, (C6-C60)arylcarbonyl, mono- or di(C1-C30)alkylamino, mono- or di(C6-C30)arylamino, tri(C1-C30)alkylsilyl, di(C1-C30)alkyl(C6-C30)arylsilyl, tri(C6-C30)arylsilyl, di(C6-C60)arylboranyl, di(C1-C60)alkylboranyl, carboxyl, nitro or hydroxyl; and
the alkyl, cycloalkyl, heterocycloalkyl, bicycloalkyl, adamantyl, alkenyl, alkynyl, aryl, alkoxy, alkylthio, aryloxy, heteroaryl, arylthio, alkoxycarbonyl, alkylcarbonyl, arylcarbonyl, alkylamino, arylamino, trialkylsilyl, dialkylarylsilyl, triarylsilyl, diarylboranyl or dialkylboranyl of R₁ through R₈ may be further substituted by one or more substituent(s) selected from a group consisting of (C1-C60)alkyl, halogen, cyano, (C3-C60) cycloalkyl, 5- or 6-membered heterocycloalkyl containing one or more heteroatom(s) selected from N, O and S, (C7-C60)bicycloalkyl, adamantyl, (C2-C60)alkenyl, (C2-C60)alkynyl, (C6-C60)aryl, (C1-C60)alkoxy, (C1-C60)alkylthio, (C6-C60)aryloxy, (C3-C60)heteroaryl containing one or more heteroatom (s) selected from N, O and S, (C6-C60)arylthio, (C1-C60)alkoxycarbonyl, (C1-C60)alkylcarbonyl, (C6-C60)arylcarbonyl, mono- or di(C1-C30)alkylamino, mono- or di(C6-C30)arylamino, tri(C1-C30)alkylsilyl, di(C1-C30)alkyl(C6-C30)arylsilyl, tri(C6-C30)arylsilyl, carboxyl, nitro and hydroxyl,
except for the case where all of R₁ through R₈ are hydrogens and one or more dopant(s) represented by Chemical Formula 2:
**M¹L¹⁰¹L¹⁰²L¹⁰³** (2)
wherein
M¹ is a metal selected from a group consisting of Group 7, Group 8, Group 9, Group 10, Group 11, Group 13, Group 14, Group 15 and Group 16 metals, and ligands L¹⁰¹, L¹⁰² and L¹⁰³ are independently selected from the following structures: 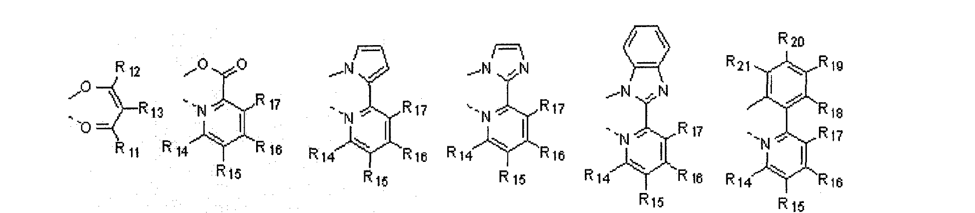 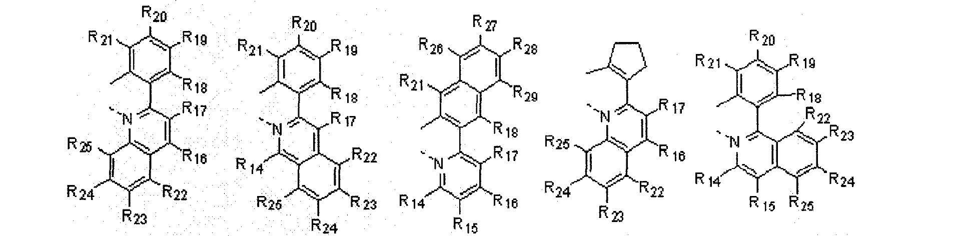 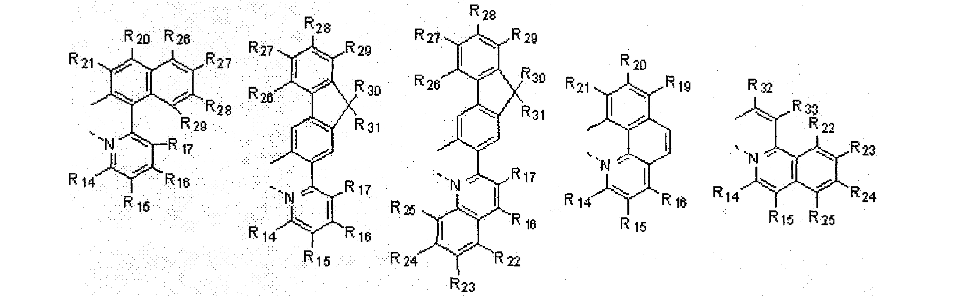 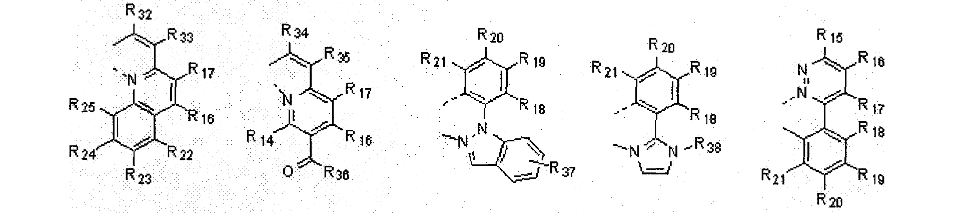 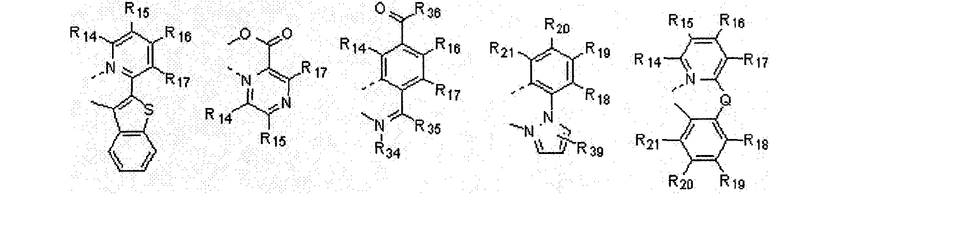 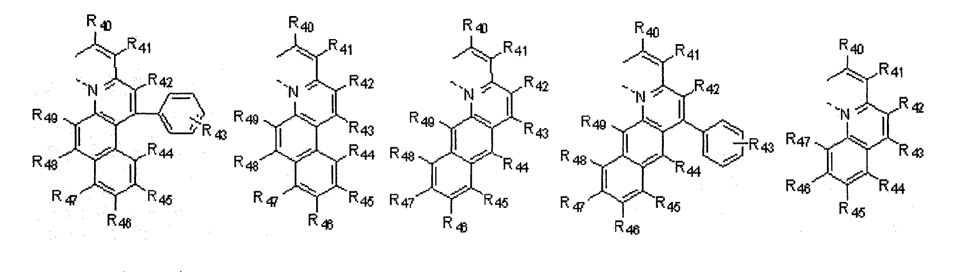 wherein
R₁₁ through R₁₃ independently represent hydrogen, (C1-C60)alkyl with or without halogen substituent, (C6-C60)aryl with or without (C1-C60)alkyl substituent or halogen;
R₁₄ through R₂₉ independently represent hydrogen, (C1-C60)alkyl, (C1-C30)alkoxy, (C3-C60)cycloalkyl, (C2-C30)alkenyl, (C6-C60)aryl, mono- or di(C1-C30)alkylamino, mono- or di(C6-C30)arylamino, SF₅, tri(C1-C30)alkylsilyl, di(C1-C30)alkyl(C6-C30)arylsilyl, tri(C6-C30)arylsilyl, cyano or halogen, and the alkyl, cycloalkyl, alkenyl or aryl of R₁₄ through R₂₉ may be further substituted by one or more substituent (s) selected from (C1-C60)alkyl, (C6-C60) aryl and halogen;
R₃₀ through R₃₃ independently represent hydrogen, (C1-C60)alkyl with or without halogen substituent or (C6-C60)aryl with or without (C1-C60)alkyl substituent;
R₃₄ and R₃₅ independently represent hydrogen, (C1-C60)alkyl, (C6-C60) aryl or halogen, or R₃₄ and R₃₅ may be linked via (C3-C12)alkylene or (C3-C12)alkenylene with or without a fused ring to form an alicyclic ring or a monocyclic or polycyclic aromatic ring, and the alkyl or aryl of R₃₄ and R₃₅, or the alicyclic ring or the monocyclic or polycyclic aromatic ring formed as they are linked via (C3-C12)alkylene or (C3-C12)alkenylene with or without a fused ring may be further substituted by one or more substituent (s) selected from (C1-C60)alkyl with or without halogen substituent, (C1-C30)alkoxy, halogen, tri(C1-C30)alkylsilyl, tri(C6-C30)arylsilyl and (C6-C60)aryl;
R₃₆ represents (C1-C60)alkyl, (C6-C60)aryl, (C5-C60)heteroaryl containing one or more heteroatom(s) selected from N, O and S or halogen;
R₃₇ through R₃₉ independently represent hydrogen, (C1-C60)alkyl, (C6-C60)aryl or halogen, and the alkyl or aryl of R₃₆ through R₃₉ may be further substituted halogen or (C1-C60)alkyl;
R₄₀ and R₄₁ independently represent hydrogen, (C1-C20) alkyl with or without halogen substituent, (C6-C20)aryl, halogen, cyano, tri(C1-C20)alkylsilyl, di(C1-C20)alkyl(C6-C20)arylsilyl, tri(C6-C20)arylsilyl, (C1-C20)alkoxy, (C1-C20)alkylcarbonyl, (C6-C20)arylcarbonyl, di(C1-C20)alkylamino or di(C6-C20)arylamino, or R₄₀ and R₄₁ may be linked via (C3-C12)alkylene or (C3-C12)alkenylene with or without a fused ring to form an alicyclic ring or a monocyclic or polycyclic aromatic ring;
the alkyl or aryl of R₄₀ and R₄₁, or the alicyclic ring or the monocyclic or polycyclic aromatic ring formed as they are linked via (C3-C12)alkylene or (C3-C12)alkenylene with or without a fused ring may be further substituted by one or more substituent(s) selected from (C1-C20)alkyl with or without halogen substituent, halogen, cyano, tri(C1-C20)alkylsilyl, di(C1-C20)alkyl(C6-C20)arylsilyl, tri(C6-C20)arylsilyl, (C1-C20)alkoxy, (C1-C20)alkylcarbonyl, (C6-C20)arylcarbonyl, di(C1-C20)alkylamino, di(C6-C20)arylamino, phenyl, naphthyl, anthryl, fluorenyl and spirobifluorenyl, or may be further substituted by phenyl or fluorenyl with one or more substituent(s) selected from a group consisting of (C1-C20)alkyl with or without halogen substituent, halogen, cyano, tri(C1-C20)alkylsilyl, di(C1-C20)alkyl(C6-C20)arylsilyl, tri(C6-C20)arylsilyl, (C1-C20)alkoxy, (C1-C20)alkylcarbonyl, (C6-C20)arylcarbonyl, di(C1-C20)alkylamino, di(C6-C20)arylamino, phenyl, naphthyl, anthryl, fluorenyl, and spirobifluorenyl;
R₄₂ through R₄₉ independently represent hydrogen, (C1-C20)alkyl with or without halogen substituent, (C1-C20)alkoxy, (C3-C12)cycloalkyl, halogen, cyano, (C6-C20)aryl, (C4-C20)heteroaryl, tri(C1-C20)alkylsilyl, di(C1-C20)alkyl(C6-C20)arylsilyl or tri(C6-C20)arylsilyl; and
Q represents 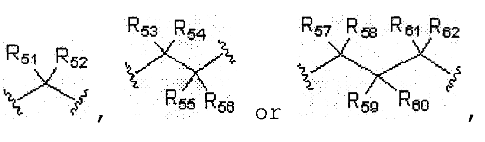 wherein R₅₁ through R₆₂ independently represent hydrogen, (C1-C60)alkyl with or without halogen substituent, (C1-C30)alkoxy, halogen, (C6-C60)aryl, cyano or (C5-C60)cycloalkyl, ore each of R₅₁ through R₆₂ may be linked to an adjacent substituent via alkylene or alkenylene to form a (C5-C7)spiro ring or a (C5-C9)fused ring, or may be linked to R₁₇ or R₁₈ via alkylene or alkenylene to form a (C5-C7)fused ring.

5. The electroluminescent device according to claim 4, wherein the organic layer comprises one or more compound(s) selected from a group consisting of arylamine compounds and styrylarylamine compounds.

6. The electroluminescent device according to claim 4, wherein the organic layer further comprises one or more metal(s) selected from a group consisting of organic metals of Group 1, Group 2, 4th period and 5th period transition metals, lanthanide metals and d-transition elements.

7. The electroluminescent device according to claim 4, wherein the organic comprises an electroluminescent layer and a charge generating layer.

8. A white-emitting electroluminescent device comprising the electroluminescent compound represented by Chemical Formula 1: 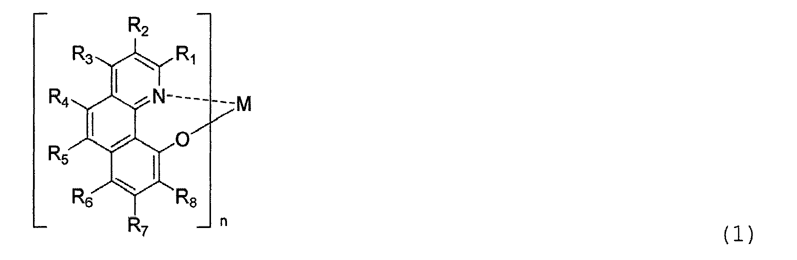 wherein
M represents a divalent or trivalent metal;
n represents the cationic valence of M;
R₁ through R₈ independently represent hydrogen, (C1-C60)alkyl, halogen, cyano, (C3-C60)cycloalkyl, 5- or 6-membered heterocycloalkyl containing one or more heteroatom(s) selected from N, O and S, (C7-C60)bicycloalkyl, adamantyl, (C2-C60)alkenyl, (C2-C60)alkynyl, (C6-C60)aryl, (C1-C60)alkoxy, (C1-C60)alkylthio, (C6-C60)aryloxy, (C3-C60)heteroaryl containing one or more heteroatom (s) selected from N, O and S, (C6-C60)arylthio, (C1-C60)alkoxycarbonyl, (C1-C60)alkylcarbonyl, (C6-C60)arylcarbonyl, mono- or di(C1-C30)alkylamino, mono- or di(C6-C30)arylamino, tri(C1-C30)alkylsilyl, di(C1-C30)alkyl(C6-C30)arylsilyl, tri(C6-C30)arylsilyl, di(C6-C60)arylboranyl, di(C1-C60)alkylboranyl, carboxyl, nitro or hydroxyl; and
the alkyl, cycloalkyl, heterocycloalkyl, bicycloalkyl, adamantyl, alkenyl, alkynyl, aryl, alkoxy, alkylthio, aryloxy, heteroaryl, arylthio, alkoxycarbonyl, alkylcarbonyl, arylcarbonyl, alkylamino, arylamino, trialkylsilyl, dialkylarylsilyl, triarylsilyl, diarylboranyl or dialkylboranyl of R₁ through R₈ may be further substituted by one or more substituent(s) selected from a group consisting of (C1-C60)alkyl, halogen, cyano, (C3-C60) cycloalkyl, 5- or 6-membered heterocycloalkyl containing one or more heteroatom(s) selected from N, O and S, (C7-C60)bicycloalkyl, adamantyl, (C2-C60)alkenyl, (C2-C60)alkynyl, (C6-C60)aryl, (C1-C60)alkoxy, (C1-C60)alkylthio, (C6-C60)aryloxy, (C3-C60) heteroaryl containing one or more heteroatom (s) selected from N, O and S, (C6-C60)arylthio, (C1-C60)alkoxycarbonyl, (C1-C60)alkylcarbonyl, (C6-C60)arylcarbonyl, mono- or di(C1-C30)alkylamino, mono- or di(C6-C30)arylamino, tri(C1-C30)alkylsilyl, di(C1-C30)alkyl(C6-C30)arylsilyl, tri(C6-C30)arylsilyl, carboxyl, nitro and hydroxyl,
except for the case where all of R₁ through R₈ are hydrogens.

9. An organic solar cell comprising the electroluminescent compound represented by Chemical Formula 1: 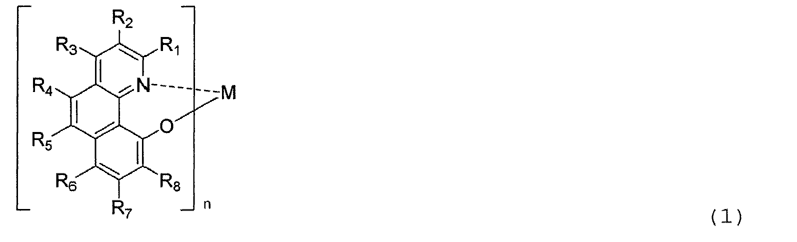 wherein
M represents a divalent or trivalent metal;
n represents the cationic valence of M;
R₁ through R₈ independently represent hydrogen, (C1-C60)alkyl, halogen, cyano, (C3-C60)cycloalkyl, 5- or 6-membered heterocycloalkyl containing one or more heteroatom(s) selected from N, O and S, (C7-C60)bicycloalkyl, adamantyl, (C2-C60)alkenyl, (C2-C60)alkynyl, (C6-C60)aryl, (C1-C60)alkoxy, (C1-C60)alkylthio, (C6-C60)aryloxy, (C3-C60)heteroaryl containing one or more heteroatom (s) selected from N, O and S, (C6-C60)arylthio, (C1-C60)alkoxycarbonyl, (C1-C60)alkylcarbonyl, (C6-C60)arylcarbonyl, mono- or di(C1-C30)alkylamino, mono- or di(C6-C30)arylamino, tri(C1-C30)alkylsilyl, di(C1-C30)alkyl(C6-C30)arylsilyl, tri(C6-C30)arylsilyl, di(C6-C60)arylboranyl, di(C1-C60)alkylboranyl, carboxyl, nitro or hydroxyl; and
the alkyl, cycloalkyl, heterocycloalkyl, bicycloalkyl, adamantyl, alkenyl, alkynyl, aryl, alkoxy, alkylthio, aryloxy, heteroaryl, arylthio, alkoxycarbonyl, alkylcarbonyl, arylcarbonyl, alkylamino, arylamino, trialkylsilyl, dialkylarylsilyl, triarylsilyl, diarylboranyl or dialkylboranyl of R₁ through R₈ may be further substituted by one or more substituent(s) selected from a group consisting of (C1-C60)alkyl, halogen, cyano, (C3-C60) cycloalkyl, 5- or 6-membered heterocycloalkyl containing one or more heteroatom(s) selected from N, O and S, (C7-C60)bicycloalkyl, adamantyl, (C2-C60)alkenyl, (C2-C60)alkynyl, (C6-C60)aryl, (C1-C60)alkoxy, (C1-C60)alkylthio, (C6-C60)aryloxy, (C3-C60) heteroaryl containing one or more heteroatom (s) selected from N, O and S, (C6-C60)arylthio, (C1-C60)alkoxycarbonyl, (C1-C60)alkylcarbonyl, (C6-C60)arylcarbonyl, mono- or di(C1-C30)alkylamino, mono- or di(C6-C30)arylamino, tri(C1-C30)alkylsilyl, di(C1-C30)alkyl(C6-C30)arylsilyl, tri(C6-C30)arylsilyl, carboxyl, nitro and hydroxyl,
except for the case where all of R₁ through R₈ are hydrogens.
